# EUROPEAN PATENT APPLICATION

(11) **EP 3 842 527 A1**
(43) Date of publication of application: **30.06.2021**
(21) Application number: 19852199.9
(22) Date of filing: 24.06.2019
(51) Int. Cl.: C12N 5/10, A61K 35/12, A61P 25/02, A61P 27/00, C12N 5/079

(54) **CELL AGGREGATION INCLUDING OLFACTORY NEURON OR PRECURSOR CELL THEREOF, AND METHOD FOR PRODUCING SAME**

(30) Priority: 24.08.2018 JP 2018157653
(71) Applicant: SUMITOMO CHEMICAL COMPANY LIMITED, Chuo-ku Tokyo 104-8260 (JP)
(72) Inventor: NAKANO, Tokushige, Osaka-shi, Osaka 554-8558 (JP)
(74) Representative: J A Kemp LLP
(86) International application number: PCT/JP2019/024964
(87) International publication number: WO 2020/039732

(57) **Abstract**

A method for producing a cell cluster including an olfactory receptor neuron or a precursor cell thereof, comprising the following steps (1) to (3): step (1) of suspension-culturing a pluripotent stem cell in the presence of a Wnt signaling pathway inhibitory substance to form a cell aggregate; step (2) of suspension-culturing the cell aggregate obtained in the step (1) in the presence of a BMP signaling pathway-activating substance; and step (3) of suspension-culturing the cell aggregate obtained in the step (2) to obtain the cell cluster, wherein step (3) comprises at least one step selected from the group consisting of: step (3a) of suspension-culturing in the presence of an FGF signaling pathway-activating substance; step (3b) of suspension-culturing in the presence of a BMP signaling pathway inhibitory substance; and step (3c) of culturing in the presence of an FGF signaling pathway-activating substance and a BMP signaling pathway inhibitory substance.

## Description

### TECHNICAL FIELD

The present invention relates to cell clusters including olfactory receptor neurons or precursor cells thereof *in vitro,* and a production method for the same. Further, the present invention also relates to cell clusters including olfactory receptor neurons or precursor cells thereof in which the cell clusters include both neural cells and non-neural epithelial tissues.

### BACKGROUND ART

Int J Clin Exp Pathol 2017; 10(7):8072-8081 (NPL 1) documents that when an embryoid body formed from mouse iPS cells is co-cultured with primary cultured cells of the olfactory epithelium or olfactory bulb collected from a mouse, neurons that express some olfactory receptor neuronal markers are induced to differentiate.

### CITATION LIST

### NON PATENT LITERATURE

NPL 1: Int J Clin Exp Pathol 2017; 10(7):8072-8081

### SUMMARY OF INVENTION

### TECHNICAL PROBLEM

An object of the present invention is to provide a technique for efficiently producing cell clusters including olfactory receptor neurons or precursor cells thereof from pluripotent stem cells. In particular, an object of the present invention is to provide a technique for efficiently producing cell clusters including olfactory receptor neurons or precursor cells thereof using feeder-free cultured pluripotent stem cells as a starting material without using primary cultured cells of the olfactory epithelium or olfactory bulb separated from a living organism.

### SOLUTION TO PROBLEM

The present inventors conducted studies to solve the above problems and found that cell clusters including olfactory receptor neurons or precursor cells thereof can be efficiently produced by suspension culturing pluripotent stem cells in the presence of a first Wnt signaling pathway inhibitory substance, adding a BMP signaling pathway-activating substance once and culturing for a certain period of time, and then further adding at least one of an FGF signaling pathway-activating substance and a BMP signaling pathway inhibitory substance. Additionally, the present inventors optimized addition conditions for the BMP signaling pathway-activating substance, the FGF signaling pathway-activating substance, and the BMP signaling pathway inhibitory substance and identified optimum time to add a BMP signaling pathway-activating substance 72 hours or less after the start of suspension culture of pluripotent stem cells, and continuously adding an FGF signaling pathway-activating substance or a BMP signaling pathway inhibitory substance 96 hours or less after addition of the BMP signaling pathway-activating substance, and thereby succeeded in more efficient production of the cell clusters including olfactory receptor neurons or precursor cells thereof. Additionally, the present inventors found that the efficiency in production of the cell clusters including olfactory receptor neurons or precursor cells thereof can be improved by preculturing pluripotent stem cells in the absence of feeder cells using at least one selected from the group consisting of TGFβ family signaling pathway inhibitory substances and sonic hedgehog signaling pathway-activating substances. That is, the present invention relates to those illustrated below.

[1] A method for producing a cell cluster including an olfactory receptor neuron or a precursor cell thereof, comprising the following steps (1) to (3):
   step (1) of suspension culturing a pluripotent stem cell in the presence of a first Wnt signaling pathway inhibitory substance to form a cell aggregate;
   step (2) of suspension culturing the cell aggregate obtained in the step (1) in the presence of a BMP signaling pathway-activating substance; and
   step (3) of suspension culturing the cell aggregate obtained in the step (2) to obtain the cell cluster,
   wherein step (3) comprises at least one step selected from the group consisting of:
      step (3a) of suspension culturing in the presence of an FGF signaling pathway-activating substance;
      step (3b) of suspension culturing in the presence of a BMP signaling pathway inhibitory substance; and
      step (3c) of suspension culturing in the presence of an FGF signaling pathway-activating substance and a BMP signaling pathway inhibitory substance.
[2] The production method according to [1], wherein the step (3) comprises the step (3a) and further comprises the step (3c) after the step (3a).
[3] The production method according to [1] or [2], wherein, in the step (1), the pluripotent stem cell is dispersed into single cells.
[4] The production method according to any of [1] to [3], wherein the step (3) comprises the step (3b) or the step (3c) and further comprises step (3d) of suspension culturing in the absence of a BMP signaling pathway inhibitory substance after the step (3b) or the step (3c).
[5] The production method according to any of [1] to [4], wherein, in the step (3), an EGF signaling pathway-activating substance is further present.
[6] The production method according to any of [1] to [5] further comprising step (a), before the step (1), of culturing the pluripotent stem cell in the absence of a feeder cell in medium containing 1) at least one selected from the group consisting of TGFβ family signaling pathway inhibitory substances and sonic hedgehog signaling pathway-activating substances and 2) an undifferentiated state maintenance factor.
[7] The production method according to any of [1] to [6], wherein a start time of the step (2) is 0.5 hours or more and 72 hours or less after start of suspension culture of pluripotent stem cell in the step (1).
[8] The production method according to any of [1] to [7], wherein a start time of the step (2) is a time at which 10% or more of cells on the surface layer of the cell aggregate formed in the step (1) is forming a tight junction with each other.
[9] The production method according to any of [1] to [8], wherein the BMP signaling pathway-activating substance includes at least one protein selected from the group consisting of BMP2, BMP4, BMP7, BMP13, and GDF7.
[10] The production method according to any of [1] to [9], wherein the BMP signaling pathway-activating substance includes BMP4, and culture of the step (2) is started in medium having a concentration of the BMP4 of 25 pM to 5 nM.
[11] The production method according to any of [1] to [10], wherein the step (3) comprises at least one step selected from the group consisting of the step (3a) and the step (3c), and the FGF signaling pathway-activating substance includes at least one selected from the group consisting of FGF2 and FGF8, and variants thereof.
[12] The production method according to any of [1] to [11], wherein a start time of the step (3) is 12 hours or more and 72 hours or less after addition of the BMP signaling pathway-activating substance in the step (2).
[13] The production method according to any of [1] to [12], wherein, in at least one step selected from the group consisting of the step (2) and the step (3), the first Wnt signaling pathway inhibitory substance is present.
[14] The production method according to any of [1] to [13], wherein the first Wnt signaling pathway inhibitory substance includes a substance having an inhibitory activity on a non-canonical Wnt pathway.
[15] The production method according to any of [1] to [14], wherein the first Wnt signaling pathway inhibitory substance includes a PORCN inhibitor.
[16] The production method according to [15], wherein the PORCN inhibitor includes at least one selected from the group consisting of IWP-2, IWP-3, IWP-4, IWP-L6, IWP-12, LGK-974, Wnt-C59, ETC-159, and GNF-6231.
[17] The production method according to [15] or [16], wherein the PORCN inhibitor includes IWP-2, and culture of the step (1) is started in medium having a concentration of the IWP-2 of 10 nM to 50 µM.
[18] The production method according to any of [1] to [17], wherein the first Wnt signaling pathway inhibitory substance includes at least one selected from the group consisting of KY02111 and KY03-I.
[19] The production method according to any of [1] to [18] wherein the first Wnt signaling pathway inhibitory substance includes KY02111, and culture of the step (1) is started in medium having a concentration of the KY02111 of 10 nM to 50 µM.
[20] The production method according to any of [1] to [19], wherein the step (3) comprises at least one step selected from the group consisting of the step (3b) and the step (3c), and the BMP signaling pathway inhibitory substance includes a type 1 BMP receptor inhibitor.
[21] The production method according to [20], wherein the type 1 BMP receptor inhibitor includes at least one selected from the group consisting of K02288, Dorsomorphin, LDN-193189, LDN-212854, LDN-214117, ML347, DMH1, and DMH2.
[22] The production method according to [20] or [21], wherein the type 1 BMP receptor inhibitor includes K02288, and the step (3) is started in medium having a concentration of the K02288 of 10 nM to 50 µM.
[23] The production method according to any of [1] to [22], wherein, in at least one step selected from the group consisting of the step (1), the step (2), and the step (3), a TGFβ signaling pathway inhibitory substance is further present.
[24] The production method according to [23], wherein the TGFβ signaling pathway inhibitory substance includes an Alk5/TGFβR1 inhibitor.
[25] The production method according to [24], wherein the Alk5/TGFβR1 inhibitor includes at least one selected from the group consisting of SB431542, SB505124, SB525334, LY2157299, GW788388, LY364947, SD-208, EW-7197, A83-01, and RepSox.
[26] The production method according to [24] or [25], wherein the Alk5/TGFβR1 inhibitor includes SB431542, and a concentration of the SB431542 in medium is 10 nM to 100 µM.
[27] The production method according to [6], wherein the sonic hedgehog signaling pathway-activating substance includes at least one selected from the group consisting of SAG, Purmorphamine, and GSA-10.
[28] The production method according to [6] or [27], wherein the sonic hedgehog signaling pathway-activating substance is in a concentration having a sonic hedgehog signaling acceleration activity equivalent to SAG at 10 nM to 700 nM.
[29] The production method according to any of [1] to [28], wherein, in at least one step selected from the group consisting of the step (1), the step (2), and the step (3), a Wnt signaling pathway-activating substance is further present.
[30] The production method according to [29], wherein the Wnt signaling pathway-activating substance acts on a signaling factor downstream of an active site of the first Wnt signaling pathway inhibitory substance.
[31] The production method according to [29] or [30], wherein the Wnt signaling pathway-activating substance includes a substance that activates a Wnt-Canonical pathway.
[32] The production method according to [31], wherein the substance that activates a Wnt-Canonical pathway inhibits decomposition of β-catenin or accelerates stabilization of β-catenin.
[33] The production method according to any of [29] to [32], wherein the Wnt signaling pathway-activating substance includes a GSK3 inhibitor.
[34] The production method according to any of [29] to [33], wherein the first Wnt signaling pathway inhibitory substance includes a PORCN inhibitor, and the Wnt signaling pathway-activating substance includes a GSK3 inhibitor.
[35] The production method according to [33] or [34], wherein the GSK3 inhibitor includes at least one selected from the group consisting of CHIR99021, CHIR98014, TWS119, SB216763, SB415286, BIO, AZD2858, AZD1080, AR-A014418, TDZD-8, LY2090314, IM-12, Indirubin, Bikinin, A 1070722, 3F8, Kenpaullone, 10Z-Hymenialdisine, Indirubin-3'-oxime, NSC 693868, TC-G 24, TCS 2002, TCS 21311, CP21R7, and derivatives of these compounds.
[36] The production method according to any of [33] to [35], wherein the GSK3 inhibitor includes CHIR99021, and a concentration of the CHIR99021 in medium is 10 nM to 50 µM.
[37] The production method according to any of [29] to [36], wherein the Wnt signaling pathway-activating substance includes at least one selected from the group consisting of BML-284 and SKL2001.
[38] The production method according to any of [1] to [37], wherein, in the step (3), a TAK1 inhibitory substance is further present.
[39] The production method according to [38], wherein the TAK1 inhibitory substance includes at least one selected from the group consisting of (5Z)-7-Oxozeaenol, N-Des(aminocarbonyl)AZ-TAK1 inhibitor, Takinib, NG25, and derivatives of these compounds.
[40] The production method according to [38] or [39], wherein the TAK1 inhibitory substance includes (5Z)-7-Oxozeaenol, and a concentration of the (5Z)-7-Oxozeaenol in medium is 10 nM to 50 µM.
[41] The production method according to any of [1] to [40], wherein the step (3) further comprises, after the step (3a), the step (3b), or the step (3c), step (3e) of culturing.
[42] The production method according to [41], wherein, in the step (3e), at least one selected from the group consisting of BMP signaling pathway inhibitory substances, TGFβ signaling pathway inhibitory substances, Wnt signaling pathway-activating substances, FGF signaling pathway-activating substances, and EGF signaling pathway-activating substances is present.
[43] The production method according to [41] or [42], wherein, in the step (3e), a retinoic acid signaling pathway-activating substance is further present.
[44] The production method according to [43], wherein the retinoic acid signaling pathway-activating substance includes at least one selected from the group consisting of all-trans-retinoic acid, isotretinoin, 9-cis retinoic acid, TTNPB, Ch55, EC19, EC23, Fenretinide, Acitretin, Trifarotene, and Adapalene.
[45] The production method according to [43] or [44], wherein the retinoic acid signaling pathway-activating substance includes EC23, and a concentration of the EC23 in medium is 10 pM to 10 µM.
[46] The production method according to any of [41] to [45], wherein, in the step (3e), serum is further present.
[47] The production method according to [46], wherein a concentration of the serum in medium is 1% to 20%.
[48] The production method according to any of [41] to [47], wherein, in the step (3e), an insulin-like growth factor receptor-activating substance is further present.
[49] The production method according to any of [41] to [48], wherein, in the step (3e), a neurotrophic factor receptor-activating substance is further present.
[50] The production method according to any of [41] to [49], wherein the step (3e) is carried out in medium containing a thickener.
[51] The production method according to [50], wherein the medium containing a thickener has a viscosity of 100 mPa·s or more.
[52] The production method according to [50] or [51], wherein the thickener includes at least one selected from the group consisting of methylcellulose, pectin, guar gum, xanthan gum, tamarind gum, carrageenan, locust bean gum, gellan gum, dextrin, diutan gum, starch, tara gum, alginic acid, curdlan, casein sodium, carob bean gum, chitin, chitosan, glucosamine, pullulan, agarose, dietary fibers and chemically modified substances or derivatives thereof.
[53] The production method according to any of [50] to [52], wherein the thickener includes methylcellulose, and a concentration of the methylcellulose in medium is 1% or more.
[54] The production method according to any of [41] to [53], wherein, in the step (3e), adherent culture is carried out.
[55] The production method according to [54], wherein the adherent culture is carried out on a culture vessel coated with at least one selected from the group consisting of extracellular matrices, basement membrane preparations, and synthesized cell adhesion molecules.
[56] The production method according to any of [41] to [55], wherein, in the step (3e), culture is carried out in a cell culture insert or a porous membrane.
[57] The production method according to any of [41] to [56], wherein, in the step (3e), culture is carried out by air liquid interface culture.
[58] The production method according to any of [1] to [57], wherein the step (3) comprises a step of culturing a cell aggregate that is embedded in a gel.
[59] The production method according to [58], wherein the gel is Matrigel.
[60] The production method according to any of [1] to [59], wherein the step (3) comprises a step of suspension culturing in medium containing a basement membrane preparation.
[61] The production method according to [60], wherein the basement membrane preparation is Matrigel, and a concentration of the Matrigel in medium is 0.5% to 4%.
[62] The production method according to any of [1] to [61], wherein, in the step (3), a second Wnt signaling pathway inhibitory substance different from the first Wnt signaling pathway inhibitory substance is further present.
[63] The production method according to [62], wherein the second Wnt signaling pathway inhibitory substance includes a substance having an inhibitory activity on a canonical Wnt pathway.
[64] The production method according to [62] or [63], wherein the second Wnt signaling pathway inhibitory substance includes a Tankyrase inhibitor.
[65] The production method according to [64], wherein the Tankyrase inhibitor includes at least one selected from the group consisting of XAV939, IWR1-endo, MN-64, WIKI4, TC-E 5001, JW 55, and AZ6102.
[66] The production method according to [64] or [65], wherein the Tankyrase inhibitor includes XAV939, and a concentration of the XAV939 in medium is 10 nM to 50 µM.
[67] The production method according to any of [62] to [66], wherein the second Wnt signaling pathway inhibitory substance is added to medium 28 days or less after start of suspension culture in the step (1).
[68] The production method according to any of [1] to [58], wherein at least one step selected from the group consisting of the step (1), the step (2), and step (3) is carried out in the absence of Matrigel.
[69] A cell cluster including an olfactory receptor neuron or a precursor cell thereof obtained by the production method according to any of [1] to [68].
[70] A cell cluster including
   1) a non-neural epithelial tissue part including an olfactory receptor neuron or a precursor cell thereof; and
   2) a nervous tissue part including a neural cell or a precursor cell thereof,
   wherein the neural cell or a precursor cell thereof includes a neural cell or a precursor cell thereof constituting a central nervous system, and at least a part of the surface of the nervous tissue part is covered with the non-neural epithelial tissue part.
[71] The cell cluster according to [70], wherein the olfactory receptor neuron or a precursor cell thereof is expressing Tuj 1, EpCAM, and Lhx2.
[72] The cell cluster according to [70] or [71], wherein the non-neural epithelial tissue part further includes a basement membrane-like structure, and the basement membrane-like structure is formed between the non-neural epithelial tissue part and the nervous tissue part.
[73] The cell cluster according to [70], wherein the non-neural epithelial tissue part forms pseudostratified epithelium or stratified epithelium.
[74] The cell cluster according to any of [70] to [73], wherein the non-neural epithelial tissue part includes an olfactory epithelial-like tissue, and the olfactory receptor neuron or a precursor cell thereof is included in the olfactory epithelial-like tissue.
[75] The cell cluster according to [74], wherein the olfactory epithelial-like tissue further includes at least two kinds of cells selected from the group consisting of supporting cells, basal cells and Bowman's gland cells, and precursor cells thereof.
[76] The cell cluster according to [74] or [75], wherein the olfactory epithelial-like tissue includes a basal cell or a precursor cell thereof.
[77] The cell cluster according to any of [74] to [76],
   wherein the olfactory epithelial-like tissue has a basal surface opposing to the nervous tissue part and a top end surface positioned on the opposite side of the basal surface;
   the basal surface faces a basement membrane; and
   the top end surface is PKCζ-positive.
[78] The cell cluster according to any of [74] to [77],
   wherein the olfactory epithelial-like tissue includes a medial olfactory epithelium and a lateral olfactory epithelium provided around the medial olfactory epithelium;
   the medial olfactory epithelium includes Sox2-, Tuj1-, and Ascl1-positive cells; and
   the lateral olfactory epithelium includes Pax6- and Pbx-positive cells.
[79] The cell cluster according to any of [74] to [78], wherein the olfactory epithelial-like tissue further includes an olfactory ensheathing glia or a precursor cell thereof.
[80] The cell cluster according to any of [74] to [79], wherein the non-neural epithelial tissue part further includes a non-neural epithelial tissue other than the olfactory epithelial-like tissue.
[81] The cell cluster according to [80], wherein the non-neural epithelial tissue includes a respiratory epithelial cell.
[82] The cell cluster according to any of [70] to [81], wherein the nervous tissue part forms an epithelial structure.
[83] The cell cluster according to any of [70] to [82], wherein the neural cell or a precursor cell thereof further includes a cell or a precursor cell thereof constituting the retina.
[84] The cell cluster according to any of [70] to [83], wherein the neural cell or a precursor cell thereof includes the cerebrum.
[85] The cell cluster according to [84], wherein the cerebrum includes an olfactory bulb.
[86] The cell cluster according to any of [70] to [85] which does not include a bone tissue.
[87] A therapeutic drug for diseases due to an olfactory system disorder comprising a cell or a tissue included in the cell cluster according to any of [69] to [86].
[88] A therapeutic drug for diseases due to a nervous tissue disorder comprising a cell or a tissue included in the cell cluster according to any of [69] to [86].
[89] A neurotoxicity or drug efficacy evaluation kit comprising a neuron or a nervous tissue included in the cell cluster according to any of [69] to [86].
[90] A screening kit for olfactory receptors comprising an olfactory receptor neuron or an olfactory epithelial-like tissue included in the cell cluster according to any of [69] to [86].

### ADVANTAGEOUS EFFECTS OF INVENTION

According to the present invention, efficient production of cell clusters including olfactory receptor neurons or precursor cells thereof from pluripotent stem cells is enabled.

### BRIEF DESCRIPTION OF DRAWINGS

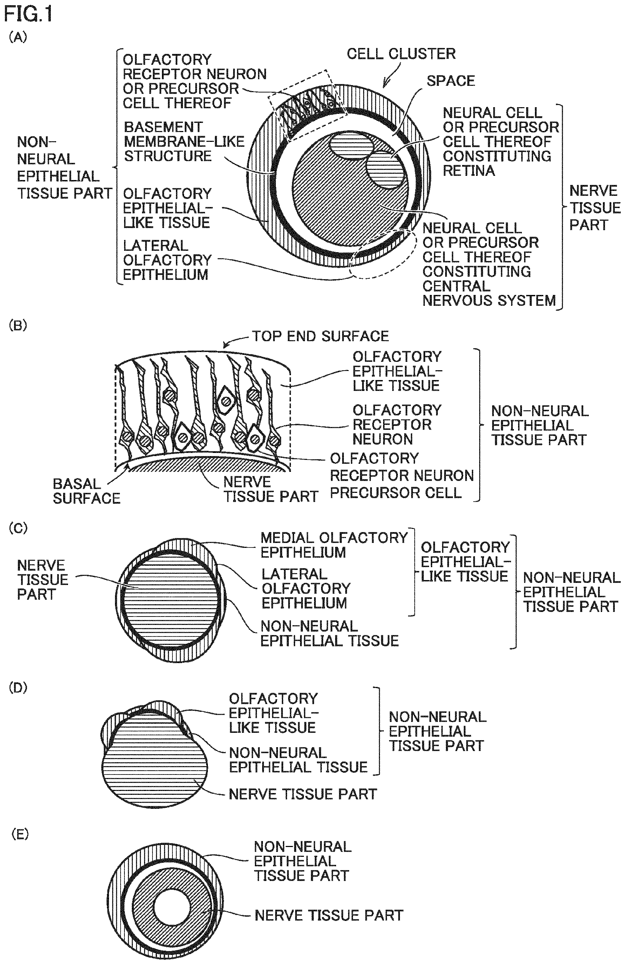

FIG. 1A is a diagram schematically depicting the structure of a cell cluster including an olfactory receptor neuron or a precursor cell thereof. FIG. 1B is a schematic diagram enlarging the portion surrounded by a dotted line in FIG. 1A. FIGS. 1C to 1E are diagrams schematically depicting other embodiments of the cell cluster of the present invention.

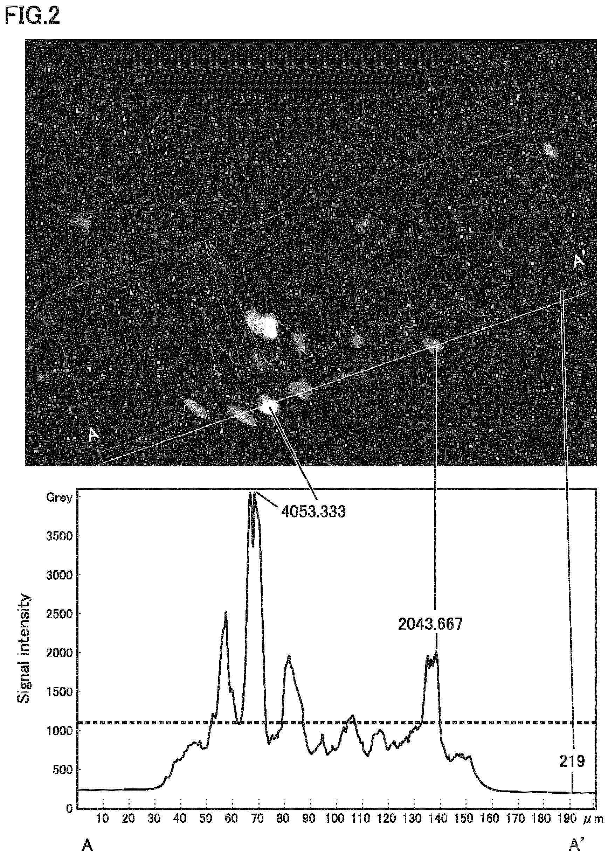

FIG. 2, in the upper section, shows the immunofluorescence staining result with an anti-Lhx2 antibody of a frozen section of a cell cluster on day 28 of culturing in Preliminary Experiment 1. FIG. 2, in the lower section, is a graph of an output of a linear fluorescence intensity profile of the region of interest shown in line A-A' in the upper section.

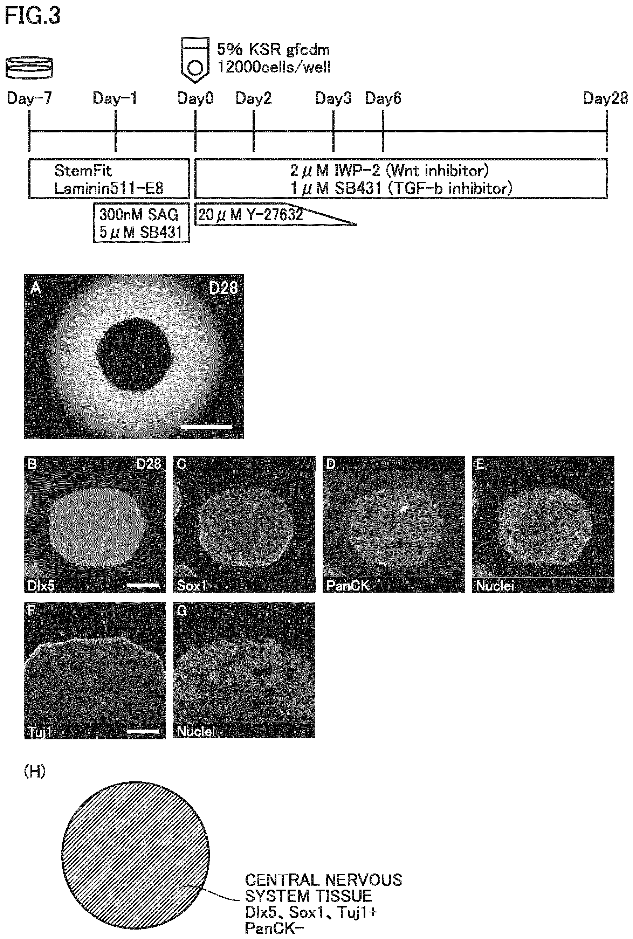

FIG. 3, in the upper section, is a diagram schematically showing the procedures for producing a cell aggregate including neuron from human ES cells in Comparative Experiment 1. FIG. 3A in the lower section is a diagram showing a bright-field observation image by inverted microscopy of the cell aggregate after 28 days from the start of suspension culturing in Comparative Experiment 1. FIGS. 3B to 3G in the lower section are diagrams showing the results of examining the expression status of each cell marker in the aggregate after 28 days from the start of suspension culturing by immunofluorescence staining. FIGS. 3B to 3E show staining images with Dlx5, Sox1, and PanCK, and the nuclei staining image, respectively. FIGS. 3F and 3G show a staining image with Tuj 1, and the nuclei staining image, respectively. The scale bar in FIG. 3A represents 500 µm, the scale bar in FIG. 3B represents 200 µm, and the scale bar in FIG. 3F represents 100 µm. FIG. 3H is a diagram schematically depicting the structure of the cell aggregate on day 28 of culturing.

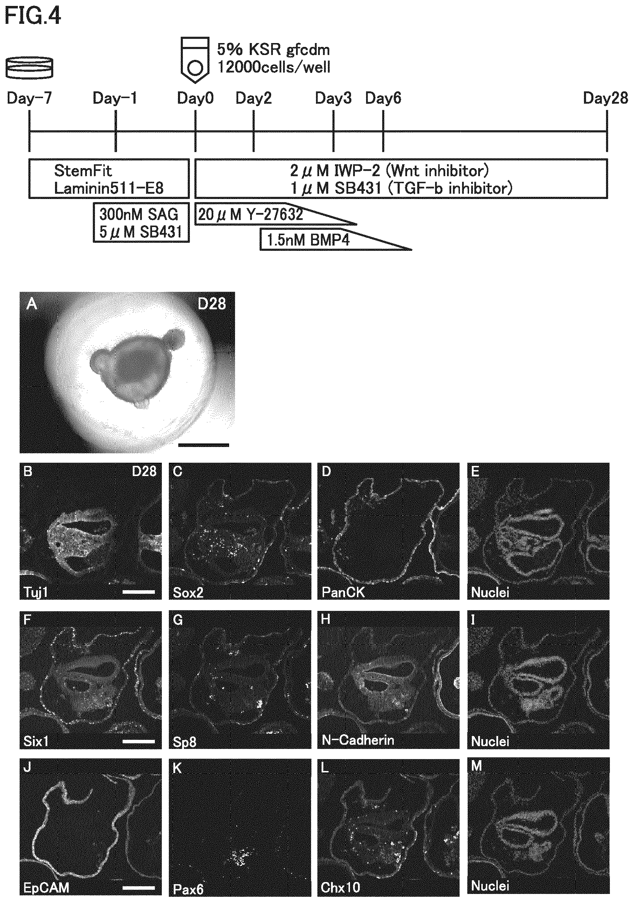

FIG. 4, in the upper section, is a diagram schematically showing the procedures for producing a cell aggregate including a nervous tissue and a non-neural epithelial tissue from human ES cells in Comparative Experiment 2. FIG. 4A in the lower section is a diagram showing a bright-field observation image by inverted microscopy of the cell cluster after 28 days from the start of suspension culturing in Comparative Experiment 2. FIGS. 4B to 4M in the lower section are diagrams showing the results of examining the expression status of each cell marker in the cell cluster after 28 days from the start of suspension culturing by immunofluorescence staining. FIGS. 4B to 4E show staining images with Tuj 1, Sox2, and PanCK, and the nuclei staining image, respectively. FIGS. 4F to 4I show staining images with Six1, Sp8, and N-Cadherin, and the nuclei staining image, respectively. FIGS. 4J to 4M show staining images with EpCAM, Pax6, and Chx10, and the nuclei staining image, respectively. The scale bars in FIGS. 4A, 4B, 4F, and 4J represent 200 µm.

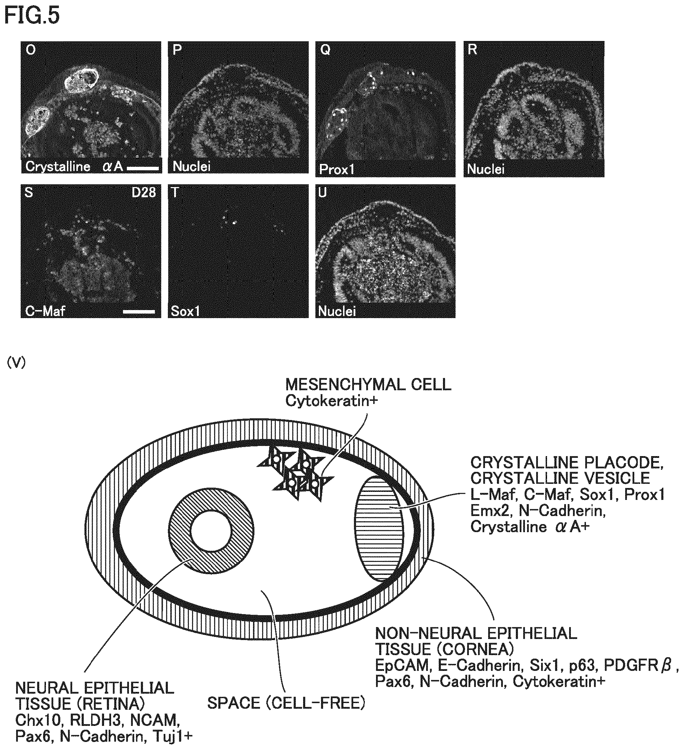

FIGS. 5O to 5U are diagrams showing the results of examining the expression status of each cell marker in the cell aggregate after 28 days from the start of suspension culturing in Comparative Experiment 2 by immunofluorescence staining. FIGS. 5O and 5P show a staining image with Crystalline aA, and the nuclei staining image, respectively. FIGS. 5Q and 5R show a staining image with Prox1, and the nuclei staining image, respectively. FIGS. 5S to 5U show staining images with C-Maf and Sox1, and the nuclei staining image, respectively. The scale bars in FIGS. 5O and 5S represent 100 µm. FIG. 5V in the lower section is a diagram schematically depicting the structure of the cell aggregate on day 28 of culturing.

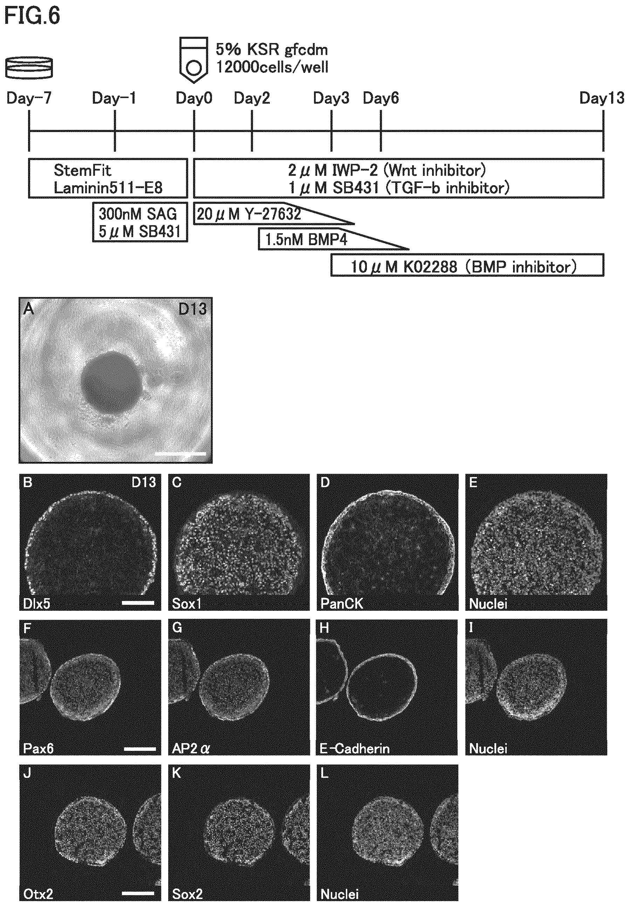

FIG. 6, in the upper section, is a diagram schematically showing the procedures for producing a cell cluster including an olfactory receptor neuron or a precursor cell thereof from human ES cells in Experiment 1. FIG. 6A in the lower section is a diagram showing a bright-field observation image by inverted microscopy of the cell cluster after 13 days from the start of suspension culturing in Experiment 1. FIGS. 6B to 6L are diagrams showing the results of examining the expression status of each cell marker in the cell cluster after 13 days from the start of suspension culturing in Experiment 1 by immunofluorescence staining. FIGS. 6B to 6E show staining images with Dlx5, Sox1, and PanCK, and the nuclei staining image, respectively. FIGS. 6F to 6I show staining images with Pax6, AP2α, and E-Cadherin, and the nuclei staining image, respectively. FIGS. 6J to 6L show staining images with Otx2 and Sox2, and the nuclei staining image, respectively. The scale bar in FIG. 6A represents 500 µm. The scale bar in FIG. 6B represents 100 µm, and the scale bars in FIGS. 6F and 6J represent 200 µm.

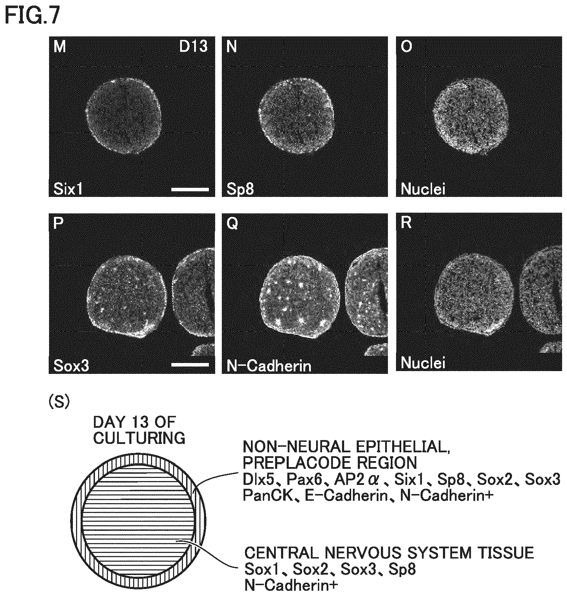

FIGS. 7M to 7R are diagrams showing the results of examining the expression status of each cell marker in the cell aggregate after 13 days from the start of suspension culturing in Experiment 1 by immunofluorescence staining. FIGS. 7M to 7O show staining images with Six1 and Sp8, and the nuclei staining image, respectively. FIGS. 7P to 7R show staining images with Sox3 and N-Cadherin, and the nuclei staining image, respectively. The scale bars in FIGS. 7M and 7P represent 200 µm. FIG. 7S in the lower section is a diagram schematically depicting the structure of a cell aggregate including a placode-derived tissue on day 13 of culturing.

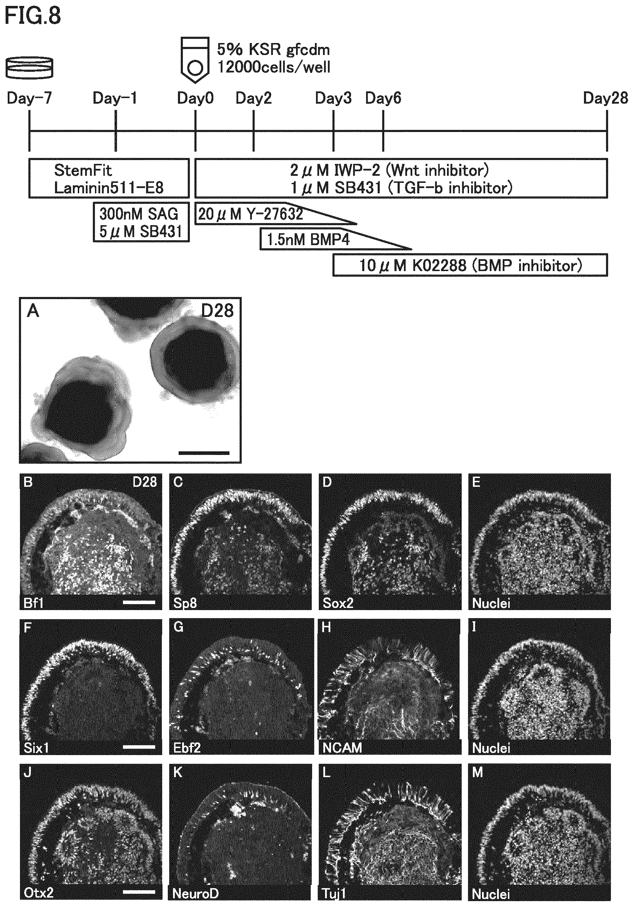

FIG. 8, in the upper section, shows a diagram schematically showing the procedures for producing a cell cluster including an olfactory receptor neuron or a precursor cell thereof from human ES cells in Experiment 2. FIG. 8A in the lower section is a diagram showing a bright-field observation image by inverted microscopy of the cell cluster after 28 days from the start of suspension culturing in Experiment 2. FIGS. 8B to 8M are diagrams showing the results of examining the expression status of each cell marker in the cell cluster after 28 days from the start of suspension culturing in Experiment 2 by immunofluorescence staining. FIGS. 8B to 8E show staining images with Bf1, Sp8, and Sox2, and the nuclei staining image, respectively. FIGS. 8F to 8I show staining images with Six1, Ebf2, and NCAM, and the nuclei staining image, respectively. FIGS. 8J to 8M show staining images with Otx2, NeuroD, and Tuj 1, and the nuclei staining image, respectively. The scale bar in FIG. 8A represents 500 µm, and the scale bars in FIGS. 8B, 8F and 8J represent 100 µm.

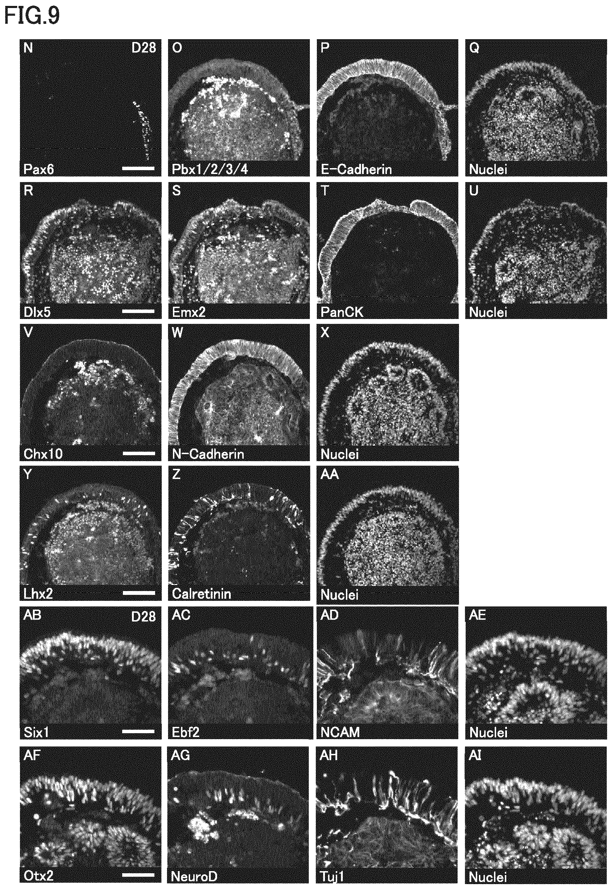

FIGS. 9N to 9AI are diagrams showing the results of examining the expression status of each cell marker in the cell cluster after 28 days from the start of suspension culturing in Experiment 2 by immunofluorescence staining. FIGS. 9N to 9Q show staining images with Pax6, Pbx1/2/3/4, and E-Cadherin, and the nuclei staining image, respectively. FIGS. 9R to 9U show staining images with Dlx5, Emx2, and PanCK, and the nuclei staining image, respectively. FIGS. 9V to 9X show staining images with Chx10 and N-Cadherin, and the nuclei staining image, respectively. FIGS. 9Y to 9AA show staining images with Lhx2 and Calretinin, and the nuclei staining image, respectively. FIGS. 9AB to 9AE show diagrams enlarging portions of FIGS. 8F to 8I, respectively. FIGS. 9AF to 9AL show diagrams enlarging portions of FIGS. 8J to 8M, respectively. The scale bars in FIGS. 9N, 9R, 9V and 9Y represent 100 µm, and the scale bars in FIGS. 9AB and 9AF represent 50 µm.

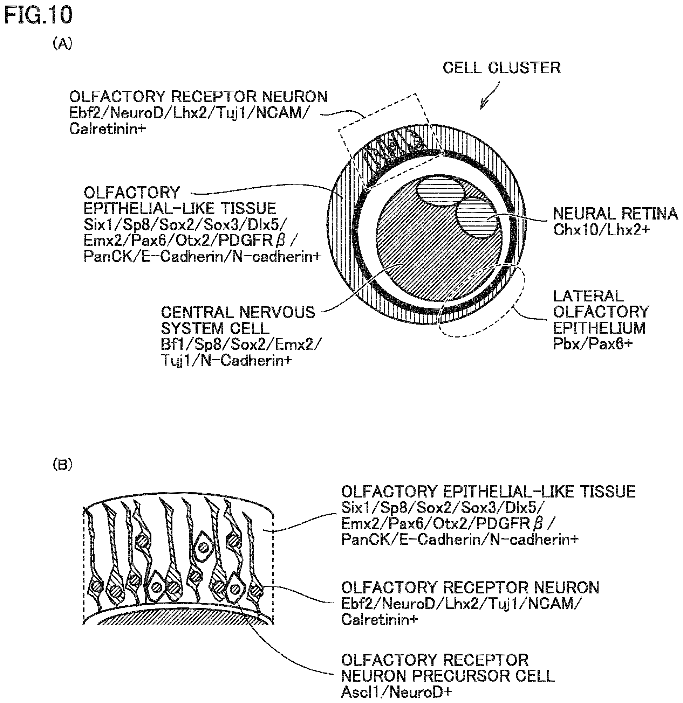

FIG. 10A is a diagram schematically depicting the structure of a cell cluster including an olfactory receptor neuron or a precursor cell thereof on day 28 of culturing. FIG. 10B is a schematic diagram enlarging the portion surrounded by a dotted line in FIG. 10A.

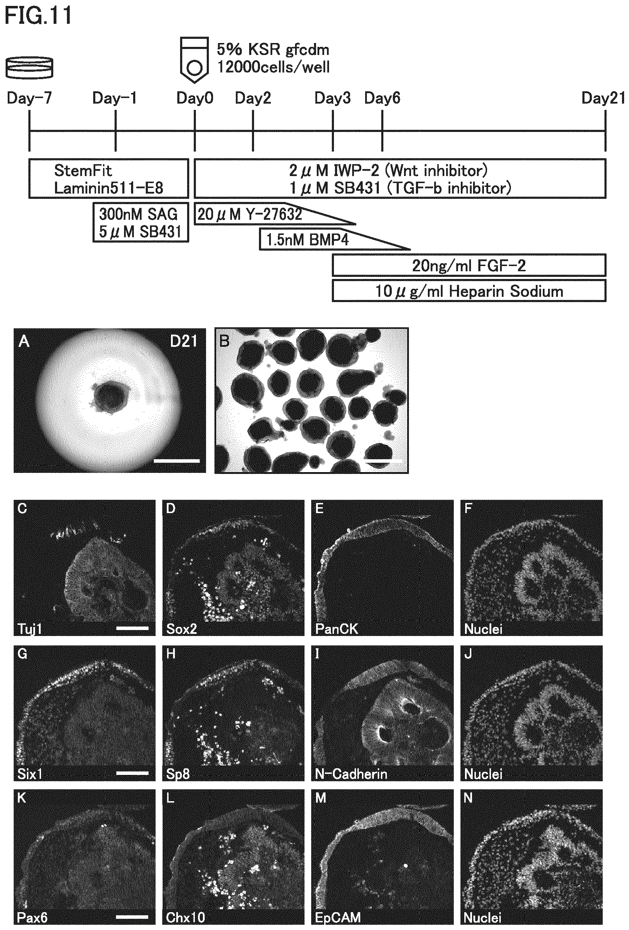

FIG. 11, in the upper section, is a diagram schematically showing the procedures for producing a cell cluster including an olfactory receptor neuron or a precursor cell thereof from human iPS cells in Experiment 3. FIGS. 11A and 11B in the lower section are diagrams showing bright-field observation images by inverted microscopy of the cell cluster after 21 days from the start of suspension culturing in Experiment 3. FIGS. 11C to 11N are diagrams showing the results of examining the expression status of each cell marker in the cell cluster after 28 days from the start of suspension culturing in Experiment 3 by immunofluorescence staining. FIGS. 11C to 11F show staining images with Tuj 1, Sox2, and PanCK, and the nuclei staining image, respectively. FIGS. 11G to 11J show staining images with Six1, Sp8, and N-Cadherin, and the nuclei staining image, respectively. FIGS. 11K to 11N show staining images with Pax6, Chx10, and EpCAM, and the nuclei staining image, respectively. The scale bars in FIGS. 11A and 11B represent 500 µm, and the scale bars in FIGS. 11C, 11G, and 11K represent 100 µm.

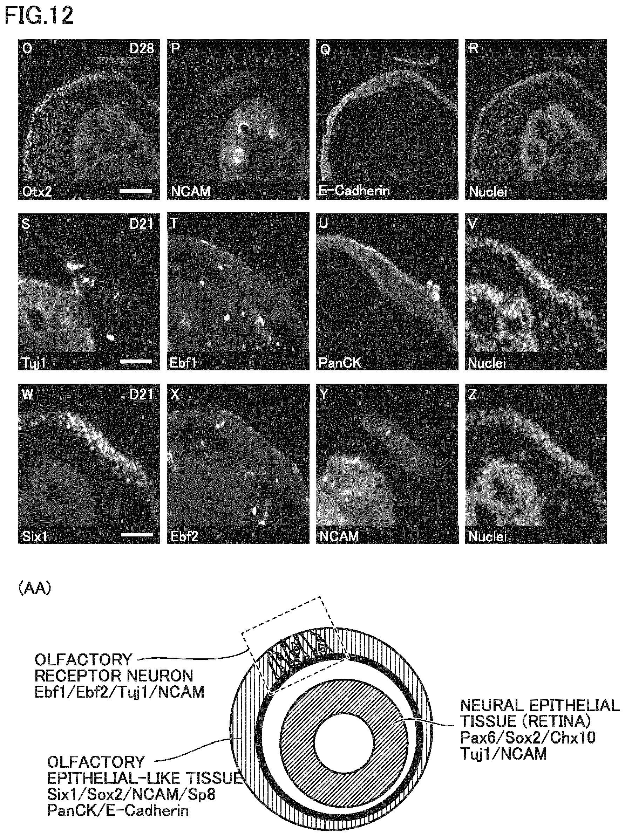

FIGS. 12O to 12Z are diagrams showing the results of examining the expression status of each cell marker in the cell cluster after 21 days from the start of suspension culturing in Experiment 3 by immunofluorescence staining. FIGS. 12O to 12R show staining images with Otx2, NCAM, and E-Cadherin, and the nuclei staining image, respectively. FIGS. 12S to 12V show staining images with Tuj 1, Ebf1, and PanCK, and the nuclei staining image, respectively. FIGS. 12W to 12Z show staining images with Six1, Ebf2, and NCAM, and the nuclei staining image, respectively. The scale bar in FIG. 12O represents 100 µm, and the scale bars in FIGS. 12S and 12W represent 50 µm. FIG. 12AA in the lower section is a diagram schematically depicting the structure of a cell cluster including an olfactory receptor neuron or a precursor cell thereof on day 21 of culturing.

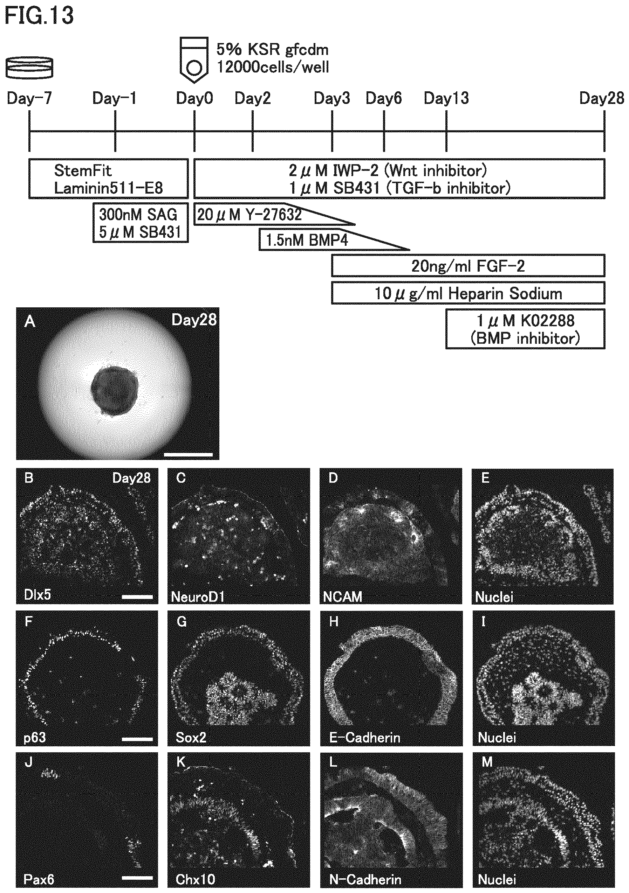

FIG. 13, in the upper section, is a diagram schematically showing the procedures for producing a cell cluster including an olfactory receptor neuron or a precursor cell thereof from human iPS cells in Experiment 4. FIG. 13A in the lower section is a diagram showing a bright-field observation image by inverted microscopy of the cell cluster after 28 days from the start of suspension culturing in Experiment 4. FIGS. 13B to 13M are diagrams showing the results of examining the expression status of each cell marker in the cell cluster after 28 days from the start of suspension culturing in Experiment 4 by immunofluorescence staining. FIGS. 13B to 13E show staining images with Dlx5, NeuroD1, and NCAM, and the nuclei staining image, respectively.
FIGS. 13F to 13I show staining images with p63, Sox2, and E-Cadherin, and the nuclei staining image, respectively. FIGS. 13J to 13M show staining images with Pax6, Chx10, and N-Cadherin, and the nuclei staining image, respectively. The scale bar in FIG. 13A represents 500 µm, the scale bars in FIGS. 13B and 13F represent 100 µm, and the scale bar in FIG. 13J represents 50 µm.

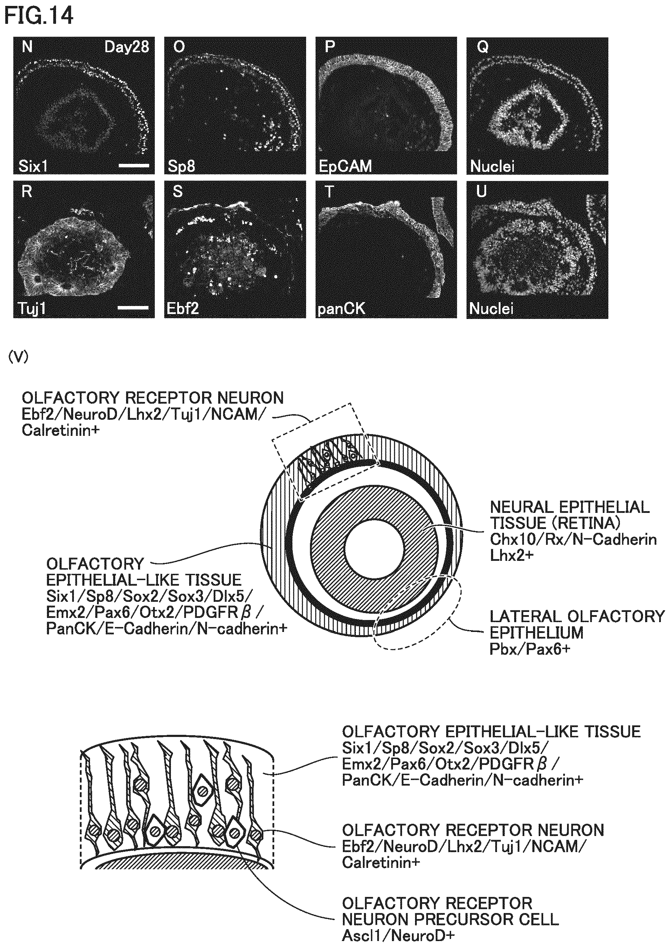

FIGS. 14N to 14U are diagrams showing the results of examining the expression status of each cell marker in the cell cluster after 28 days from the start of suspension culturing in Experiment 4 by immunofluorescence staining. FIGS. 14N to 14Q show staining images with Six1, Sp8, and EpCAM, and the nuclei staining image, respectively. FIGS. 14R to 14U show staining images with Tuj1, Ebf2, and PanCK, and the nuclei staining image, respectively. The scale bars in FIGS. 14N and 14R represent 100 µm. FIG. 14V is a diagram schematically depicting the structure of a cell cluster including an olfactory receptor neuron or a precursor cell thereof on day 28 of culturing produced by the method described in Experiment 4.

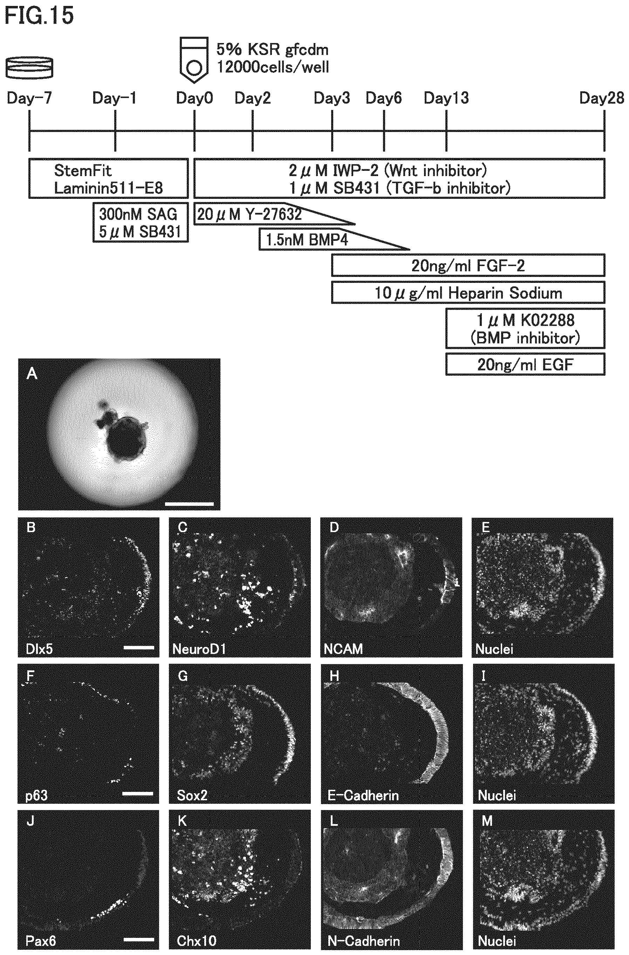

FIG. 15, in the upper section, is a diagram schematically showing the procedures for producing a cell cluster including an olfactory receptor neuron or a precursor cell thereof from human iPS cells in Experiment 5. FIG. 15A in the lower section is a diagram showing a bright-field observation image by inverted microscopy of the cell cluster after 28 days from the start of suspension culturing in Experiment 5. FIGS. 15B to 15M are diagrams showing the results of examining the expression status of each cell marker in the cell cluster after 28 days from the start of suspension culturing in Experiment 5 by immunofluorescence staining. FIGS. 15B to 15E show staining images with Dlx5, NeuroD1, and NCAM, and the nuclei staining image, respectively. FIGS. 15F to 15I show staining images with p63, Sox2, and E-Cadherin, and the nuclei staining image, respectively. FIGS. 15J to 15M show staining images with Pax6, Chx10, and N-Cadherin, and the nuclei staining image, respectively. The scale bar in FIG. 15A represents 500 µm, and the scale bars in FIGS. 15B, 15F, and 15J represent 100 µm.

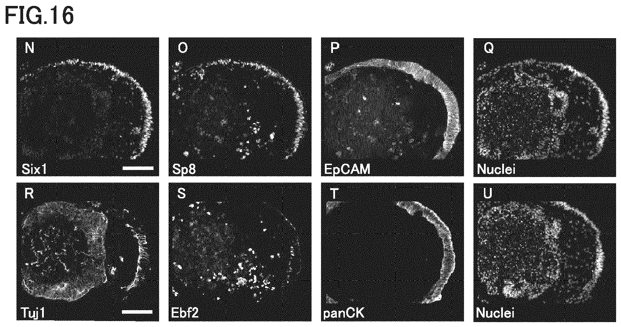

FIGS. 16N to 16U are diagrams showing the results of examining the expression status of each cell marker in the cell cluster after 28 days from the start of suspension culturing in Experiment 5 by immunofluorescence staining. FIGS. 16N to 16Q show staining images with Six1, Sp8, and EpCAM, and the nuclei staining image, respectively. FIGS. 16R to 16U show staining images with Tuj1, Ebf2, and PanCK, and the nuclei staining image, respectively. The scale bars in FIGS. 16N and 16R represent 100 µm.

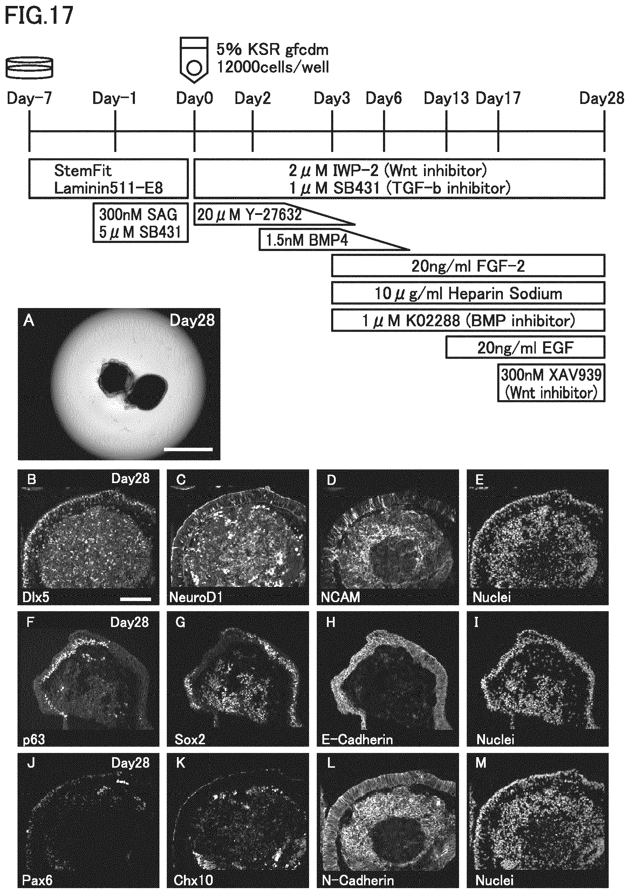

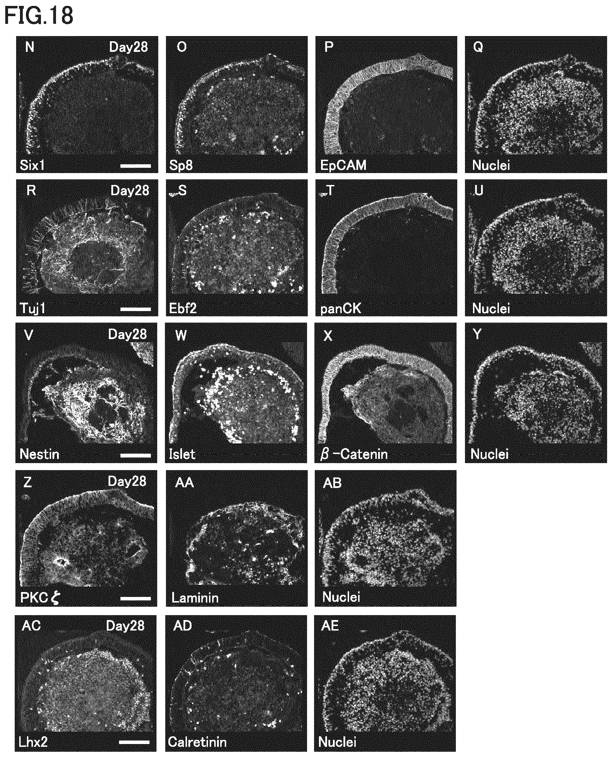

FIG. 17, in the upper section, is a diagram schematically showing the procedures for producing a cell cluster including an olfactory receptor neuron or a precursor cell thereof from human iPS cells in Experiment 6. FIG. 16A in the lower section is a diagram showing a bright-field observation image by inverted microscopy of the cell cluster after 28 days from the start of suspension culturing in Experiment 6. FIGS. 16B to 16M are diagrams showing the results of examining the expression status of each cell marker in the cell cluster after 28 days from the start of suspension culturing in Experiment 6 by immunofluorescence staining. FIGS. 16B to 16E show staining images with Dlx5, NeuroD1, and NCAM, and the nuclei staining image, respectively. FIGS. 16F to 16I show staining images with p63, Sox2, and E-Cadherin, and the nuclei staining image, respectively. FIGS. 16J to 16M show staining images with Pax6, Chx10, and N-Cadherin, and the nuclei staining image, respectively. The scale bar in FIG. 16A represents 500 µm, and the scale bar in FIG. 16B represents 100 µm. FIGS. 18N to 18AE are diagrams showing the results of examining the expression status of each cell marker in the cell cluster after 28 days from the start of suspension culturing in Experiment 6 by immunofluorescence staining. FIGS. 18N to 18Q show staining images with Six1, Sp8, and EpCAM, and the nuclei staining image, respectively. FIGS. 18R to 18U show staining images with Tuj1, Ebf2, and PanCK, and the nuclei staining image, respectively. FIGS. 18V to 18Y show staining images with Nestin, Islet, and β-Catenin, and the nuclei staining image, respectively. FIGS. 18Z to 18AB show staining images with PKCζ and Laminin, and the nuclei staining image, respectively. FIGS. 18AC to 18AE show staining images with Lhx2 and Calretinin, and the nuclei staining image, respectively. The scale bars in FIGS. 18N, 18R, 18V, 18Z, and 18AC represent 100 µm.

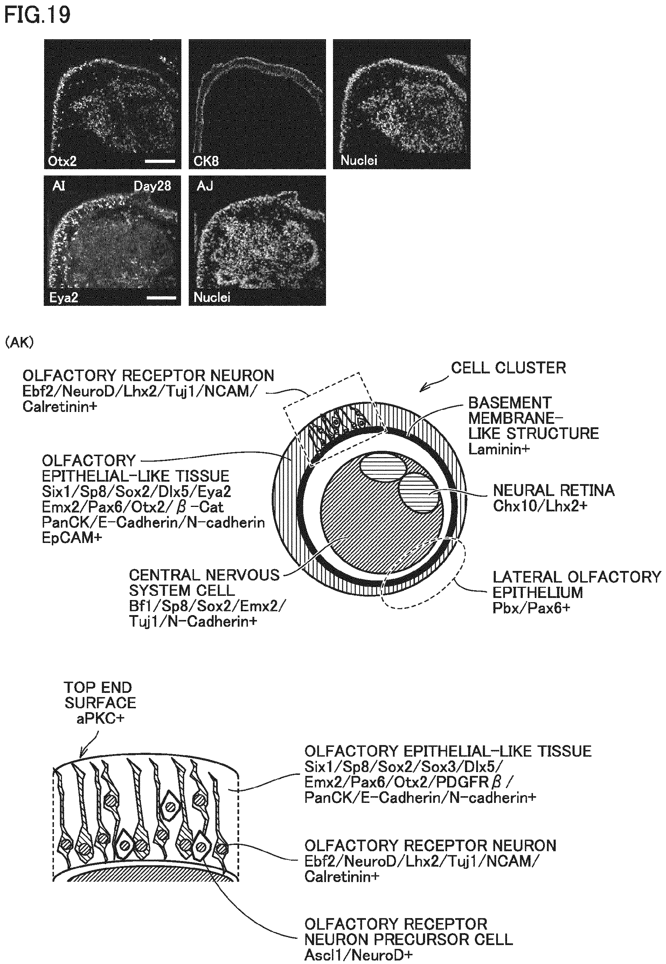

FIGS. 19AF to 19AJ are diagrams showing the results of examining the expression status of each cell marker in the cell cluster after 28 days from the start of suspension culturing in Experiment 6 by immunofluorescence staining. FIGS. 19AF to 19AH show staining images with Otx2 and CK8, and the nuclei staining image, respectively. FIGS. 19AI and 19AJ show staining image with Eya2, and the nuclei staining image, respectively. FIG. 19AK is a diagram schematically depicting the structure of a cell cluster including an olfactory receptor neuron or a precursor cell thereof on day 28 of culturing produced by the method described in Experiment 6. The scale bars in FIGS. 19AF and 19AI represent 100 µm.

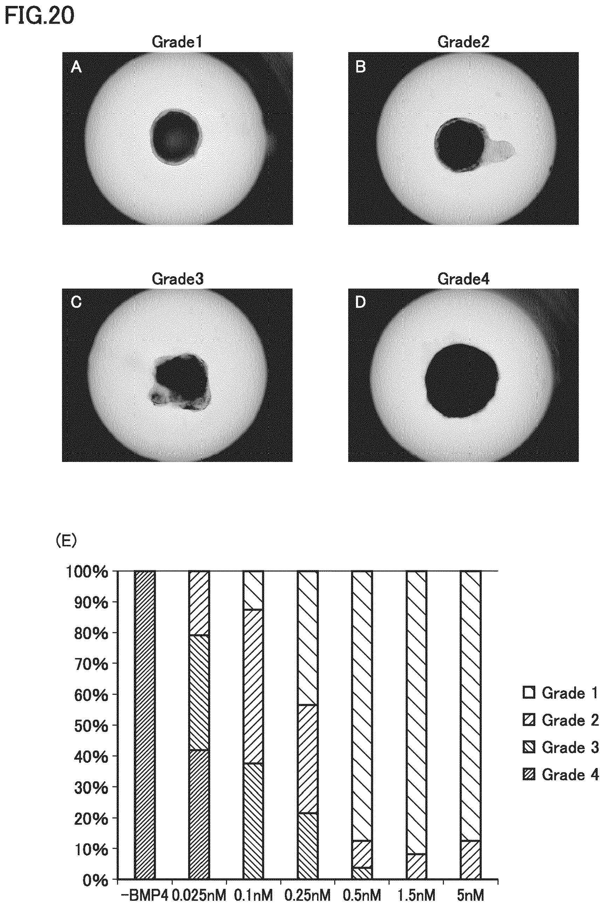

FIGS. 20A to 20D in the upper section are diagrams showing bright-field observation images by inverted microscopy of the cell clusters after 28 days from the start of suspension culturing when the addition concentration of BMP4 was changed when a cell cluster including an olfactory receptor neuron or a precursor cell thereof are produced from human ES cells in Experiment 7. FIG. 20A is an example of a cell cluster of Grade 1, FIG. 20B is an example of a cell cluster of Grade 2, FIG. 20C is an example of a cell cluster of Grade 3, FIG. 20D is a cell cluster of Grade 4. FIG. 20E is a graphical representation of the quality evaluation results of the cell clusters formed at each BMP4 concentration.

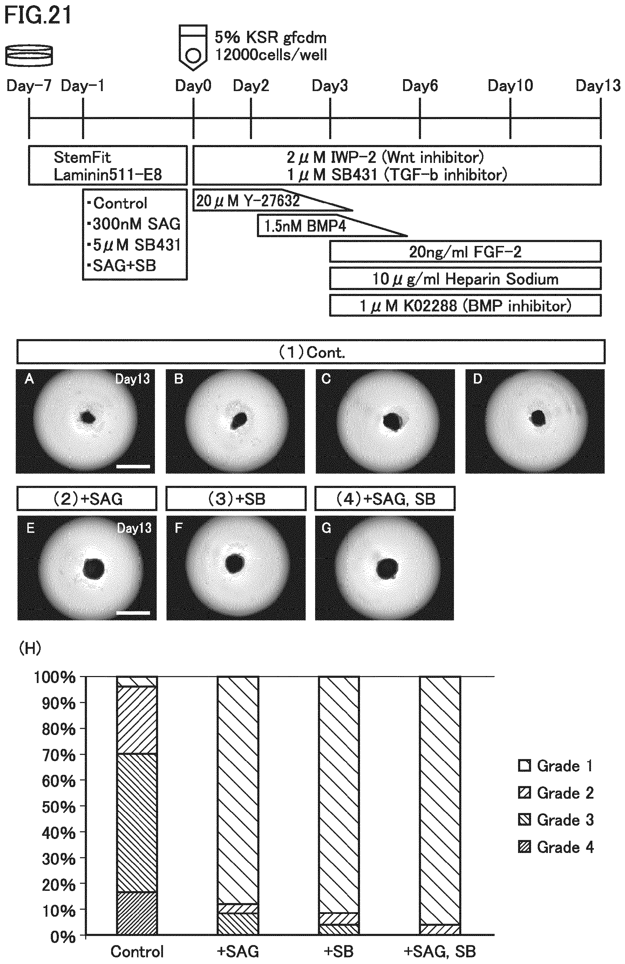

FIG. 21, in the upper section, is a diagram schematically showing the procedures for examining the effect of a compound pretreatment of human iPS cells subjected to differentiation induction in the production of a cell cluster including an olfactory receptor neuron or a precursor cell thereof from human iPS cells in Experiment 8. FIGS. 21A to 21G in the lower section are diagrams showing bright-field observation images by inverted microscopy of the aggregates after 13 days from the start of suspension culturing in Experiment 8. FIGS. 21A to 21D are diagrams showing bright-field observation images by inverted microscopy of the cell clusters on day 13 after the start of suspension culturing formed from human iPS cells in a control where only a solvent DMSO was added as a pretreatment. FIGS. 21E to 21G are diagrams showing bright-field observation images by inverted microscopy of the cell clusters on day 13 after the start of suspension culturing formed from human iPS cells that were pretreated under different conditions. The scale bars in FIGS. 21A and 21E represent 500 µm. FIG. 21H is a graphical representation of the quality evaluation results of the cell clusters formed in each pretreatment condition.

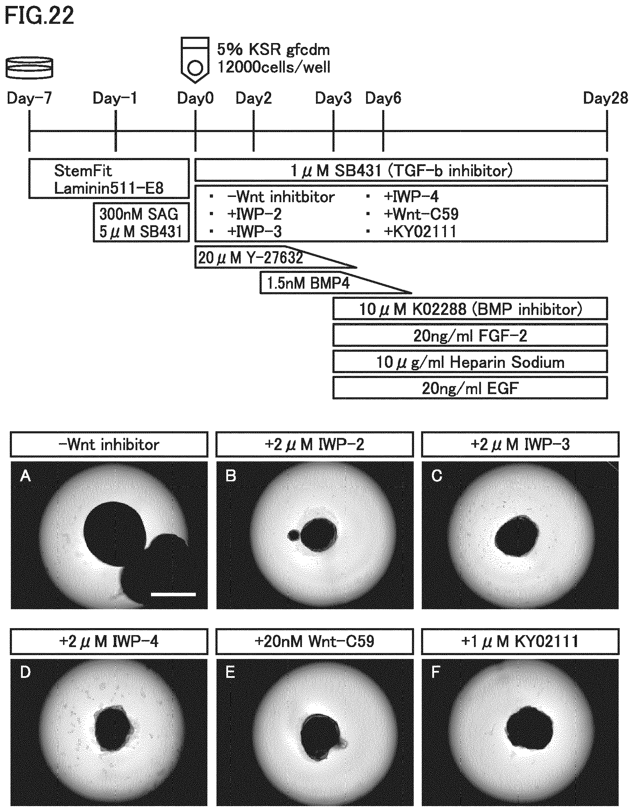

FIG. 22, in the upper section, is a diagram schematically showing the procedures for examining the effect of each first Wnt signaling pathway inhibitory substance in the production of a cell cluster including an olfactory receptor neuron or a precursor cell thereof from human iPS cells in Experiment 9. FIGS. 22A to 22F in the lower section are diagrams showing bright-field observation images by inverted microscopy of the aggregates after 28 days from the start of suspension culturing in Experiment 9. FIG. 22A is a diagram showing a bright-field observation image by inverted microscopy of the cell cluster on day 28 after the start of suspension culturing in a control where a first Wnt signaling pathway inhibitory substance was not added. FIGS. 22B to 22F are diagrams showing bright-field observation images by inverted microscopy of the cell clusters on day 28 after the start of suspension culturing when varying type of first Wnt signaling pathway inhibitory substances were each added at the start of suspension culturing. The scale bar in FIG. 22A represents 500 µm.

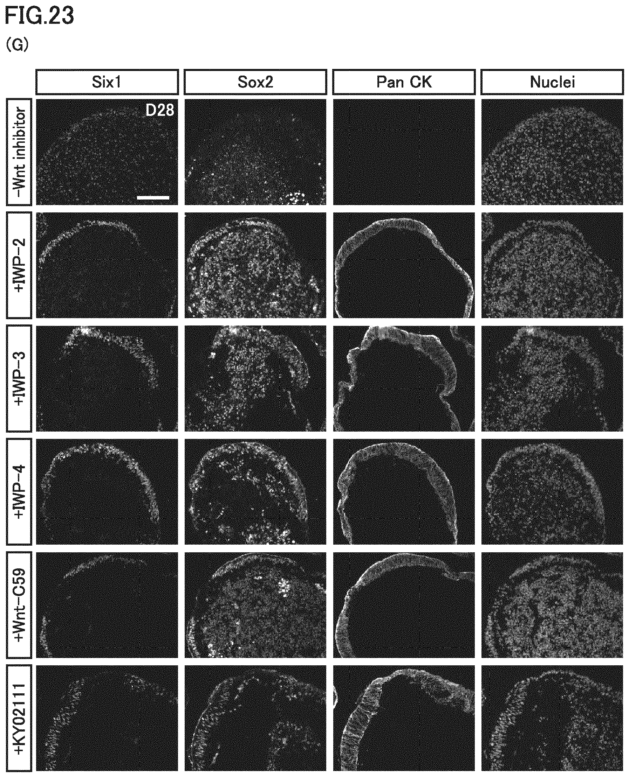

FIG. 23G is a diagram showing the results of examining the expression status of each cell marker in the cell cluster after 28 days from the start of suspension culturing in Experiment 9 by immunofluorescence staining. Panel rows show conditions of the first Wnt signaling pathway inhibitory substance, and panel columns show Six1, Sox2, pancytokeratin (PanCK), and the nuclei staining image in order from left to right, respectively. The scale bar in the upper left panel represents 100 µm.

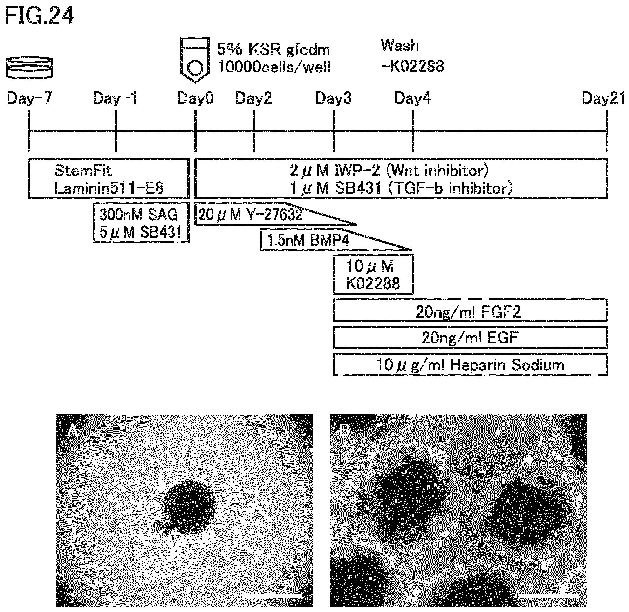

FIG. 24, in the upper section, is a diagram schematically showing the procedures for producing a cell cluster including an olfactory receptor neuron or a precursor cell thereof from human iPS cells using a three-dimensional culture vessel in Experiment 10. FIGS. 24A and 24B in the lower section are diagrams showing bright-field observation images by inverted microscopy of the aggregates after 21 days from the start of suspension culturing in Experiment 10. The scale bar in FIG. 24A represents 500 µm, and the scale bar in FIG. 24B represents 200 µm.

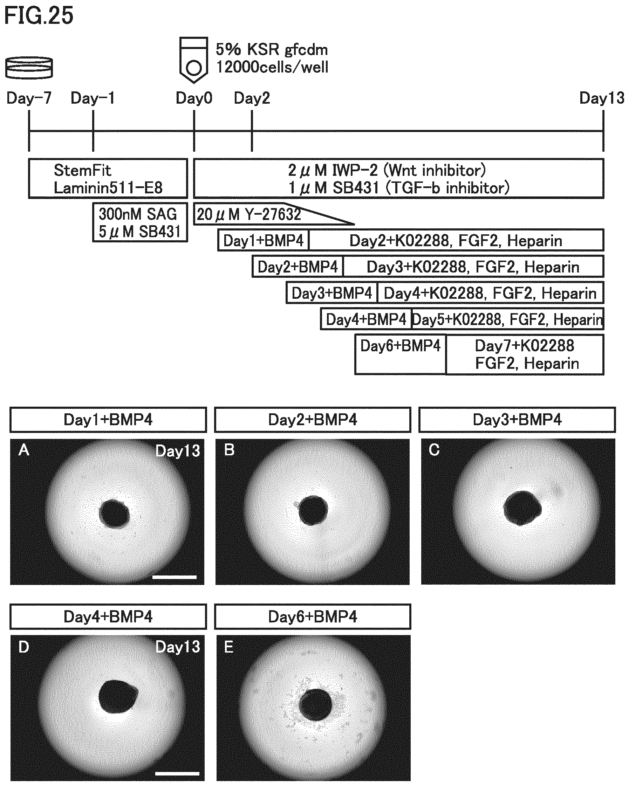

FIG. 25, in the upper section, is a diagram schematically showing the procedures for examining the effect of a difference in the time to add a BMP signaling pathway-activating substance in the production of a cell cluster including an olfactory receptor neuron or a precursor cell thereof from human iPS cells in Experiment 11. FIGS. 25A to 25E in the lower section are diagrams showing the bright-field observation image by inverted microscopy of the cell aggregates after 13 days from the start of suspension culturing in Experiment 11. The scale bars in FIGS. 25A and 25D represent 500 µm.

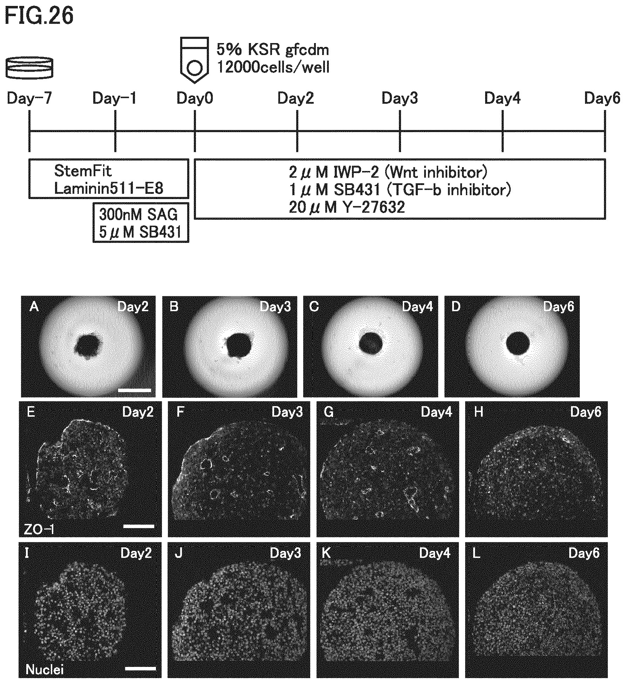

FIG. 26, in the upper section, is a diagram schematically showing the procedures for examining the optimal time to add a BMP signaling pathway-activating substance in the production of a cell cluster including an olfactory receptor neuron or a precursor cell thereof from human iPS cells in Experiment 12. FIGS. 26A to 26D in the lower section are diagrams showing bright-field observation images by inverted microscopy of the aggregates after 2 to 6 days from the start of suspension culturing in Experiment 12. FIGS. 26E to 26H show staining images with ZO-1 of the cell aggregates after 2 to 6 days from the start of suspension culturing, and FIGS. 26I to 26L are diagrams showing the contrast staining image of the respective nuclei. The scale bar in FIG. 26A represents 500 µm, and the scale bars in FIGS. 26E and 26I represent 100 µm.

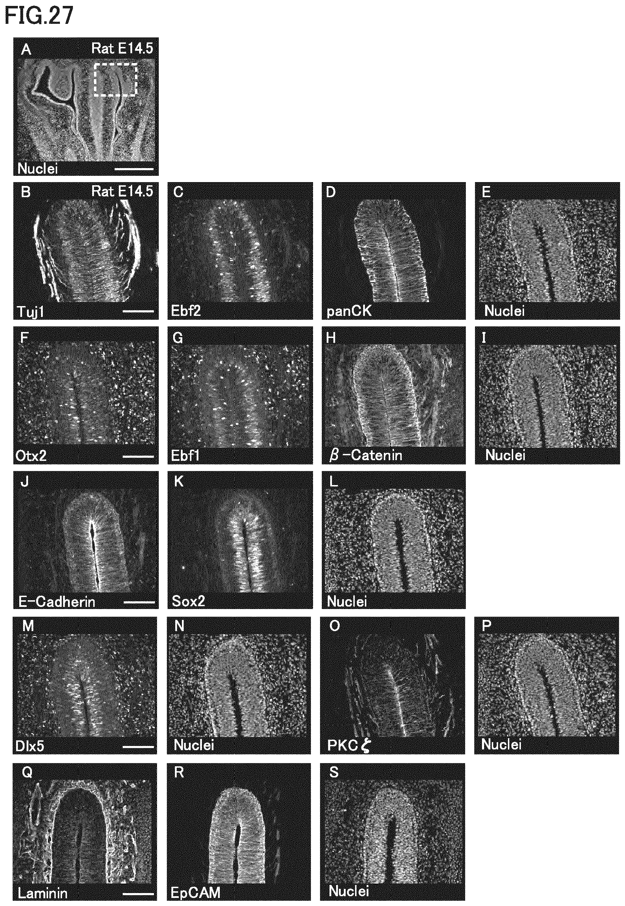

FIGS. 27A to 27P are the results of producing coronal sections of rat embryo at embryonic day 14.5 in Experiment 13 and analyzing the expression of each marker by immunostaining. FIG. 27A is a staining image at low magnification of nuclear staining. FIGS. 27B to 27S are staining images at high magnification of successive sections of the region corresponding to the region defined by dashed lines in FIG. 27A. FIGS. 27B to 27E show staining images with Tuj1, Ebf2 and PanCK, and the nuclei staining image. FIGS. 27F to 27I show staining images with Otx2, Ebf1 and β-Catenin, and the nuclei staining image. FIGS. 27J to 27L show staining images with E-Cadherin and Sox2, and the nuclei staining image. FIGS. 27M and 27N show a staining image with Dlx5, and the nuclei staining image. FIGS. 27O and 27P show a staining image with PKCζ, and the nuclei staining image. FIGS. 27Q to 27S show staining images with Laminin and EpCAM, and the nuclei staining image. The scale bar in FIG. 27A represents 500 µm, and the scale bars in FIGS. 27B, 27F, 27J, 27M, and 27Q represent 100 µm.

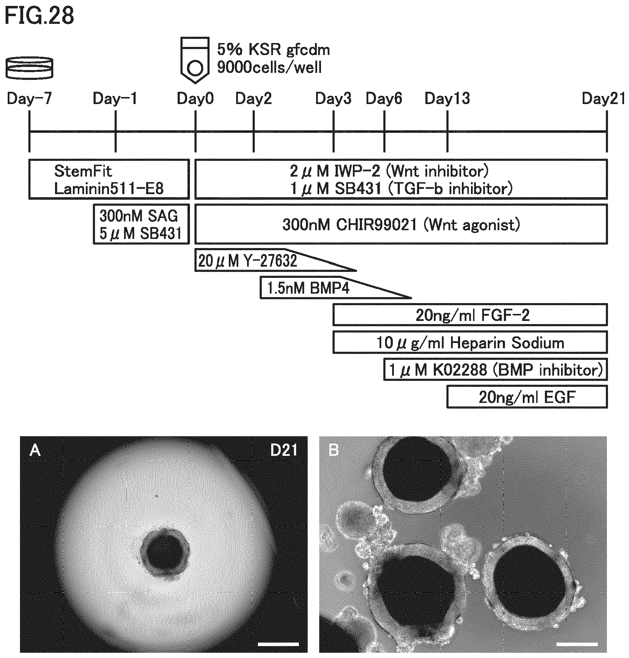

FIG. 28, in the upper section, is a diagram schematically showing conditions for combination use of a Wnt signaling pathway inhibitory substance and a Wnt signaling pathway-activating substance in the production of a cell cluster including an olfactory receptor neuron or a precursor cell thereof from human iPS cells in Experiment 14. FIGS. 28A and 28B in the lower section are diagrams showing bright-field observation images by inverted microscopy of the cell clusters on day 21 from the start of suspension culturing in Experiment 14. The scale bar in FIG. 28A represents 500 µm, and the scale bar in FIGS. 28B represents 200 µm.

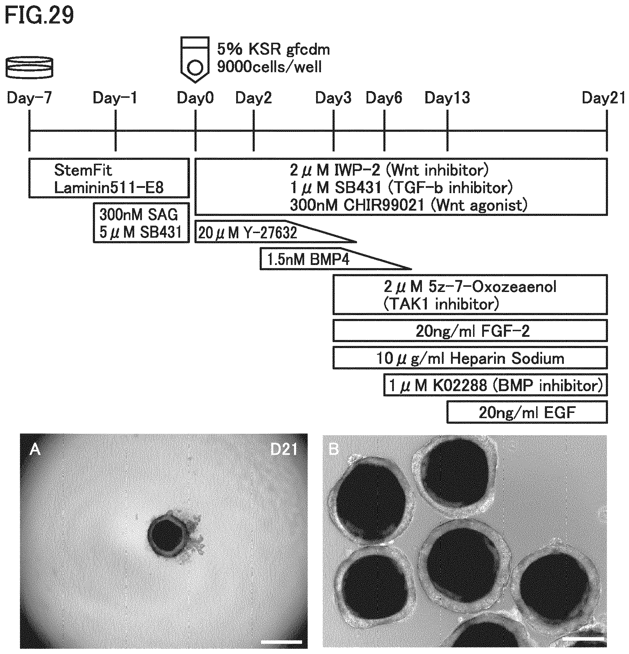

FIG. 29, in the upper section, is a diagram schematically showing conditions for use of a TAK1 inhibitory substance in the production of a cell cluster including an olfactory receptor neuron or a precursor cell thereof from human iPS cells in Experiment 15. FIGS. 29A and 29B in the lower section are diagrams showing bright-field observation images by inverted microscopy of the cell clusters on day 21 from the start of suspension culturing in Experiment 15. The scale bar in FIG. 29A represents 500 µm, and the scale bar in FIG. 29B represents 200 µm.

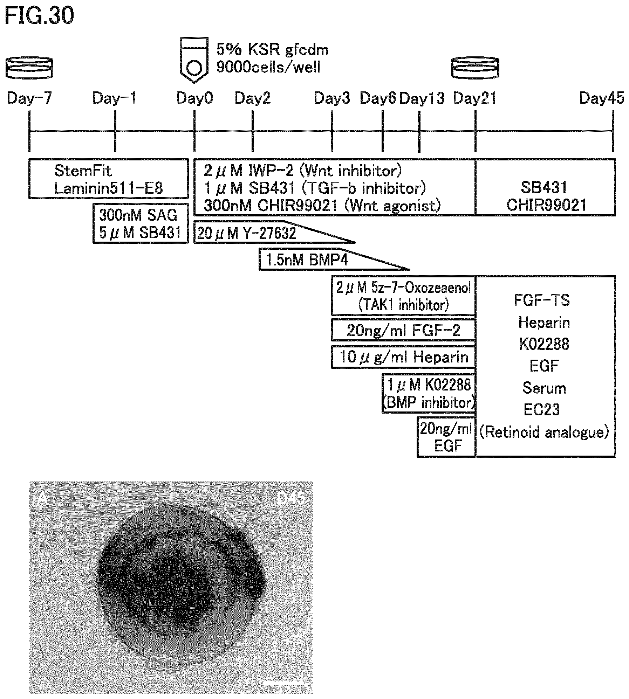

FIG. 30, in the upper section, is a diagram representing the procedures for culturing a cell cluster including an olfactory receptor neuron or a precursor cell thereof produced from human iPS cells in a viscous culture medium in Experiment 16. FIG. 30A in the lower section is a diagram showing a bright-field observation image by inverted microscopy of the cell cluster on day 45 from the start of suspension culturing in Experiment 16. The scale bar in FIG. 30A represents 200 µm.

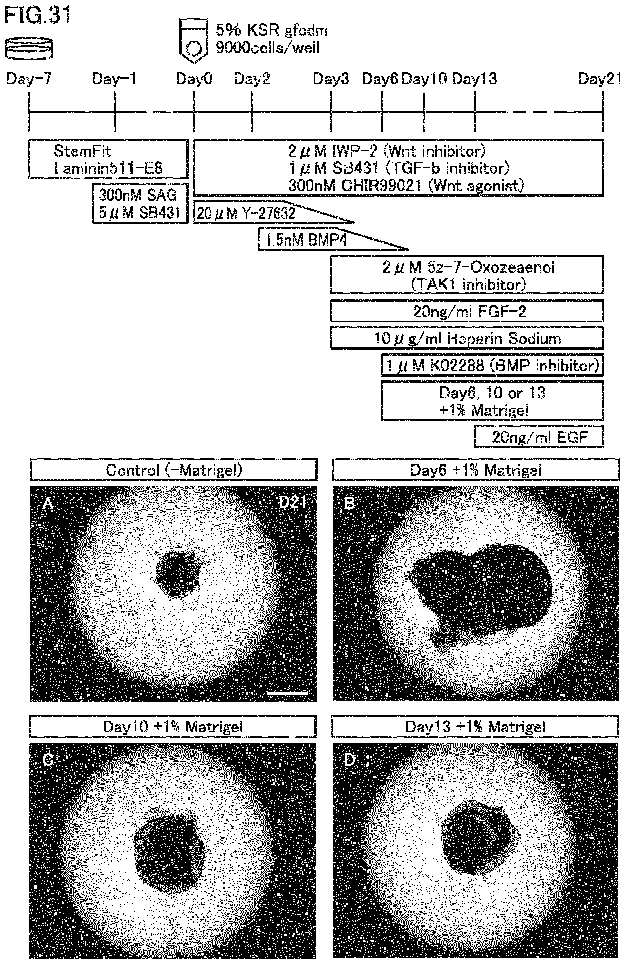

FIG. 31, in the upper section, is a diagram schematically showing conditions for addition of a basement membrane preparation in the production of a cell cluster including an olfactory receptor neuron or a precursor cell thereof from human iPS cells in Experiment 17. FIGS. 31A to 31D in the lower section are diagrams showing bright-field observation images by inverted microscopy of the cell clusters on day 21 from the start of suspension culturing in Experiment 17. The scale bar in FIG. 31A represents 500 µm.

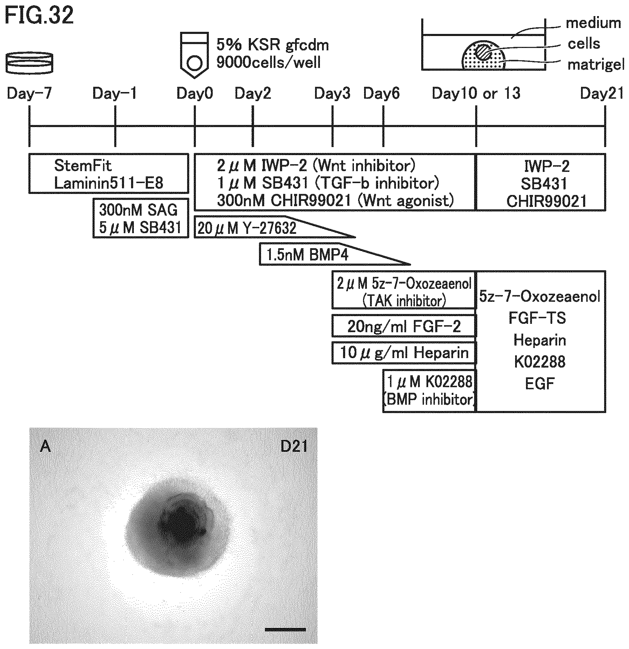

FIG. 32, in the upper section, is a diagram schematically showing the procedures for embedded culturing a cell cluster including an olfactory receptor neuron or a precursor cell thereof in a basement membrane preparation from human iPS cells in Experiment 18. FIG. 32A in the lower section is a diagram showing a bright-field observation image by inverted microscopy of the cell cluster on day 21 from the start of suspension culturing in Experiment 18. The scale bar in FIG. 32A represents 500 µm.

### DESCRIPTION OF EMBODIMENTS

### [1. Definition]

The "stem cell" means an undifferentiated cell having differentiation potency and proliferation potency (particularly self-replication potency). The stem cell includes, in accordance with the differentiation ability, subpopulations such as pluripotent stem cell, multipotent stem cell, and unipotent stem cell. The pluripotent stem cell refers to a stem cell that can be cultured *in vitro* and has an ability to differentiate to all cells (tissues derived from the triderm (ectoderm, mesoderm, endoderm)) (pluripotency) constituting a living organism. The multipotent stem cell means a stem cell that has an ability to differentiate to, not all kinds but, several kinds of tissues and cells. The unipotent stem cell means a stem cell that has an ability to differentiate to specific tissues and cells.

The pluripotent stem cell can be induced from a fertilized egg, a cloned embryo, a germline stem cell, an interstitial stem cell, a somatic cell and the like. Examples of the pluripotent stem cell include Embryonic stem cell (ES cell), EG cell (Embryonic germ cell), and induced pluripotent stem cell (iPS cell). Muse cell (Multi-lineage differentiating Stress Enduring cell) obtainable from a mesenchymal stem cell (MSC) and GS cell generated from a germ cell (for example, testicle) are also encompassed in the pluripotent stem cells.

Embryonic stem cell was established for the first time in 1981 and has been applied to knockout mouse generation since 1989. In 1998, human embryonic stem cell was established and is being utilized in regenerative medicine. ES cells can be produced by culturing an inner cell mass on feeder cells or in medium containing leukemia inhibitory factor (LIF). The production method of ES cells is described in, for example, WO96/22362, WO02/101057, U.S. Patent No. 5,843,780 specification, U.S. Patent No. 6,200,806 specification, and U.S. Patent No. 6,280,718 specification. Embryonic stem cell can be obtained from designated institutes, or a commercially available product can also be purchased. KhES-1, KhES-2, and KhES-3, that are human embryonic stem cells, can be obtainable from Institute for Frontier Life and Medical Sciences, Kyoto University. For both mouse embryonic stem cells, EB5 cell is obtainable from Institute of Physical and Chemical Research and D3 strain can be obtainable from ATCC. A method for providing human embryonic stem cell culture (cell line) without destroying a human embryo is described in, for example, Cell Stem Cell, 2008:2(2):113-117 and WO03/046141. A nuclear transfer embryonic stem cell (ntES cell), that is one of the ES cells, can be established from a cloned embryo generated by transferring the cell nucleus of a somatic cell to an egg from which a cell nucleus is removed.

EG cells can be produced by culturing primordial germ cells in medium containing mSCF, LIF, and bFGF (Cell, 70:841-847, 1992).

The "induced pluripotent stem cell" is a pluripotency-induced cell by reprogramming a somatic cell by a known method. Specifically, examples include pluripotency-induced cells obtained by reprogramming somatic cells differentiated to fibroblasts, peripheral blood mononuclear cells or the like by the expression of any combinations of a plurality of genes selected from the reprogramming gene group including Oct3/4, Sox2, Klf4, Myc (c-Myc, N-Myc, L-Myc), Glis1, Nanog, Sall4, lin28, Esrrb and the like. In 2006, induced pluripotent stem cell was established using mouse cells by Yamanaka et. al (Cell, 2006, 126(4) pp.663-676). Induced pluripotent stem cell was also established using human fibroblasts in 2007 and has pluripotency and self-replication potency like the embryonic stem cells (Cell, 2007, 131(5) pp.861-872; Science, 2007, 318(5858) pp.1917-1920; Nat. Biotechnol., 2008, 26(1) pp.101-106). Induced pluripotent stem cells can be induced from somatic cells by the addition of a compound other than by the method for producing induced pluripotent stem cells by direct reprogramming of gene expressions (Science, 2013, 341 pp.651-654).

The somatic cell used for producing induced pluripotent stem cells is not particularly limited and examples include tissue-derived fibroblasts, hematopoietic cells (for example, peripheral blood mononuclear cells and T cells), liver cells, pancreatic cells, intestinal epithelial cells, and smooth muscle cells.

When the reprogramming is carried out by the expression of several kinds of genes (for example, 4 factors of Oct3/4, Sox2, Klf4, and Myc) for producing induced pluripotent stem cells, a means for expressing genes is not particularly limited. Examples of the means include infection methods using a virus vector (for example, retroviral vector, lentiviral vector, Sendai virus vector, adenoviral vector, adeno-associated virus vector), gene transfer methods using a plasmid vector (for example, plasmid vector, episomal vector) (for example, calcium phosphate method, lipofection method, RetroNectin method, electroporation method), gene transfer methods using a RNA vector (for example, calcium phosphate method, lipofection method, electroporation method), and protein direct injections.

Induced pluripotent stem cells can be obtained from designated institutes, or a commercially available product can also be purchased. For example, human induced pluripotent stem cell line, 201B7 strain, can be obtained from Kyoto University, and HC-6#10 strain can be obtained from Institute of Physical and Chemical Research.

The pluripotent stem cell used in the present invention is preferably embryonic stem cells or induced pluripotent stem cells.

Examples of the multipotent stem cell include tissue stem cells such as hematopoietic stem cells, neural stem cells, retinal stem cells, and mesenchymal stem cells (also called histo-stem cells, tissue-specific stem cells, or somatic stem cells).

Gene modified pluripotent stem cells can be generated by, for example, using a homologous recombination technology. Examples of the gene on a chromosome to be modified include cell marker genes, histocompatibility antigen genes, and genes related to diseases due to a neural cell disorder. The modification of a target gene on a chromosome can be carried out using the methods described in Manipulating the Mouse Embryo, A Laboratory Manual, Second Edition, Cold Spring Harbor Laboratory Press (1994), Gene Targeting, A Practical Approach, IRL Press at Oxford University Press (1993), and Bio Manual Series 8, Gene targeting, Generation of mutant mouse using ES cells, YODOSHA CO., LTD. (1995).

Specifically, for example, a genomic gene of a target gene to be modified (for example, cell marker genes, histocompatibility antigen genes, and disease-related genes) is isolated, and a target vector for homologous recombination of the target gene is generated using the isolated genomic gene. The generated target vector is introduced to a stem cell and cells in which the homologous recombination occurred between the target gene and the target vector are selected thereby to generate stem cells in which the gene on the chromosome is modified.

Examples of the method for isolating a genomic gene of a target gene include known methods described in Molecular Cloning, A Laboratory Manual, Second Edition, Cold Spring Harbor Laboratory Press (1989), and Current Protocols in Molecular Biology, John Wiley & Sons (1987-1997). A genomic gene of a target gene can also be isolated using genome DNA library screening system (manufactured by Genome Systems), Universal Genome Walker Kits (manufactured by CLONTECH) and the like.

Generation of a target vector for homologous recombination of a target gene and efficient selection of homologous recombinants can be carried out in accordance with the methods described in Gene Targeting, A Practical Approach, IRL Press at Oxford University Press (1993), Bio Manual Series 8, Gene targeting, Generation of mutant mouse using ES cells, YODOSHA CO., LTD. (1995) and the like. For the target vector, either replacement type or insertion type can be used. For the selection method, a method such as positive selection, promoter selection, negative selection, or poly-A selection can be used.

Examples of the method for selecting intended homologous recombinants from the sorted cell lines include southern hybridization method and PCR method on a genomic DNA.

The "mammal" encompasses rodents, ungulates, Carnivora, and primates. Rodents encompass mouse, rat, hamster, guinea pig and the like. Ungulates encompass pig, cow, goat, horse, sheep and the like. Carnivora encompasses dog and cat. The "primate" refers to mammals that belong to Primates, and examples of the primate include Strepsirrhini such as lemur, loris, and treeshrew and Anthropoidea such as monkey, ape, and human.

The pluripotent stem cell used in the present invention is mammal's pluripotent stem cells, preferably pluripotent stem cells of rodents (for example, mouse, rat) or primates (for example human, monkey), and most preferably pluripotent stem cells of human.

The "cell adhesion" refers to a cell-to-cell adhesion and an adhesion of cells to extracellular matrices. The adhesion of cells to a culture vessel or the like caused under artificial culture environments *in vitro* is also encompassed in the cell adhesion. Examples of the cell adhesion type include anchoring junction, communicating junction, and occluding junction.

The "tight junction" indicates the occluding junction, among the intercellular adhesions, found in the vertebrates and the chordates. The tight junction is formed between epithelial cells. The presence of the tight junction in tissues derived from a living organism and in cell clusters generated by the production method of the present invention can be detected by, for example, a technique such as immunohistochemistry that uses an antibody to structural components of the tight junction (anti-Claudin antibody, anti-ZO-1 antibody or the like).

The "suspension culture" in the present invention refers to the culture that is carried out while maintaining a state in which cells, cell aggregates, or cell clusters are present as suspended in a culture medium. That is, the suspension culture is carried out by the condition under which cells, cell aggregates, or cell clusters are not allowed to adhere to a culture vessel or the like, and the culture carried out by the condition under which the adherence to a culture vessel or the like is allowed (adherent culture) is not included in the category of suspension culture. In this case, the adhesion of cells refers to the formation of a strong cell-substratum junction, which is a type of the cell adhesions, between cells, cell aggregates, or cell clusters and a culture vessel. More specifically, the suspension culture refers to the culture by the condition under which a strong cell-substratum junction is not allowed to be formed between cells, cell aggregates, or cell clusters and a culture vessel or the like, whereas the adherent culture refers to the culture by the condition under which a strong cell-substratum junction is allowed to be formed between cells, cell aggregates, or cell clusters and a culture vessel or the like.

In cell aggregates or cell clusters during the suspension culture, plane attachment occurs between cells. In cell aggregates or cell clusters during the suspension culture, the cell-substratum junction is hardly formed, or has little contribution even if formed, between cells and a culture vessel or the like. In some embodiments, in cell aggregates or cell clusters during the suspension culture, endogenous cell-substratum junctions are present in the aggregates or cell clusters but the cell-substratum junction is hardly formed, or has little contribution even if formed, between cells and a culture vessel or the like.

The plane attachment occurs between cells refers to a cell-to-cell attachment by plane. More specifically, the plane attachment occurs between cells refers that a ratio of a surface area of a certain cell adhered to the surface of another cell is, for example, 1% or more, preferably 3% or more, and more preferably 5% or more. The surface of cells can be observed by the staining using a reagent (for example, Dil) that stains a membrane, or immunostaining of cell adhesion factors (for example, E-cadherin, N-cadherin).

The culture apparatus used for carrying out the suspension culture is not particularly limited as long as "suspension culturing" is enabled and can be suitably determined by those skilled in the art. Examples of such a culture apparatus include flasks, tissue culture flasks, dishes, petri dishes, tissue culture dishes, multidishes, microplates, micro well plates, micropores, multiplates, multi well plates, chamber slides, schales, tubes, trays, culture bags, spinner flasks, and roller bottles. These culture apparatuses are preferably cell non-adherent to enable the suspension culture. The cell non-adherent culture apparatuses usable are those with the surface thereof that is not artificially treated (for example, coating treatment using a basement membrane preparation, an extracellular matrix such as laminin, entactin, collagen, or gelatin, or a polymer such as polylysine or polyornithine, or surface processing such as positive charge treatment) for the purpose of enhancing the adhesiveness to cells. The cell non-adherent culture apparatuses usable are those with the surface thereof that is artificially treated (for example, ultra-hydrophilic treatment such as 2-methacryloyloxyethyl phosphorylcholine (MPC) polymer or the like, low protein adsorption treatment or the like) for the purpose of reducing the adhesiveness to cells. Rotation culture can be carried out using a spinner flask, a roller bottle or the like. The culture surface of the culture apparatus can be flat bottom or concaved.

The suspension culture can also be carried out by embedding cell aggregates in a gel or encapsulating in a permeable capsule for the purpose of protecting the cell aggregates from physical stresses such as a shear force caused when carrying out the suspension culture, increasing local concentrations of growth factors and cytokines secreted by the cells, and promoting the development of tissues (Nature, 2013, 501.7467:373). The gel or the capsule used for embedding can be derived from a living organism or made of a synthetic polymer. Examples of the gel or capsule used for such purposes include Matrigel (manufactured by Corning), PuraMatrix (manufactured by 3D Matrix), VitroGel 3D (manufactured by TheWell Bioscience), collagen gel (manufactured by Nitta Gelatin Inc), alginate gel (manufactured by PG Research Co., Ltd.), and Cell-in-a-Box (manufactured by Austrianova).

The medium used for cell culture can be prepared using medium typically used for culturing animal cells as basal medium. Examples of the basal medium include Basal Medium Eagle (BME), BGJb medium, CMRL 1066 medium, Glasgow Minimum Essential Medium (Glasgow MEM), Improved MEM Zinc Option, Iscove's Modified Dulbecco's Medium (IMDM), Medium 199, Eagle Minimum Essential Medium (Eagle MEM), Alpha Modified Eagle Minimum Essential Medium (αMEM), Dulbecco's Modified Eagle Medium (DMEM), F-12 medium, DMEM/F12, IMDM/F12, Ham's medium, RPMI 1640, Fischer's medium, and mixed medium thereof.

For the culture of pluripotent stem cells, medium for pluripotent stem cell culture with the above basal medium as a base, preferably known medium for embryonic stem cells or induced pluripotent stem cells, medium for feeder-free pluripotent stem cell culture (feeder-free medium) and the like can be used. Examples of the feeder-free medium include Essential 8 medium, TeSR medium, mTeSR medium, mTeSR-E8 medium, and StemFit medium.

The "serum-free medium" in the present invention means the medium that does not contain non-conditioned or unpurified serum. In the present invention, even media in which purified blood-derived components or animal tissue-derived components (for example, growth factors) are mixed in are included in the serum-free media as long as non-conditioned or unpurified serum is not contained.

The serum-free medium can contain a serum substitute. Examples of the serum substitute include those suitably containing albumin, transferrin, fatty acids, collagen precursor, trace elements, 2-mercaptoethanol, 3'thiol glycerol, and equivalents thereof. Such a serum substitute can be prepared by the method described in, for example, WO98/30679. A commercially available product can also be utilized as the serum substitute. Examples of the commercially available serum substitute include Knockout Serum Replacement (manufactured by Thermo Fisher Scientific: hereinafter described also as "KSR"), Chemically-defined Lipid concentrated (manufactured by Thermo Fisher Scientific), Glutamax (manufactured by Thermo Fisher Scientific), B27 Supplement (manufactured by Thermo Fisher Scientific), and N2 Supplement (manufactured by Thermo Fisher Scientific).

The serum-free medium used for the suspension culture can suitably contain fatty acids or fats, amino acids (for example, non-essential amino acids), vitamins, growth factors, cytokines, antioxidants, 2-mercaptoethanol, pyruvic acid, buffers, and inorganic salts.

For avoidance of cumbersome preparations, serum-free medium to which a proper amount (for example, about 0.5% to about 30%, preferably about 1% to about 20%) of a commercially available KSR (manufactured by Thermo Fisher Scientific) is added (for example, medium in which 1 x chemically-defined Lipid concentrated, 5% KSR and 450 µM 1-monothioglycerol are added to a 1:1 mixed solution of F-12 medium and IMDM medium) can also be used. Additionally, examples of the KSR equivalent include medium disclosed in Japanese National Patent Publication No. 2001-508302.

The "serum medium" in the present invention means the medium containing non-conditioned or unpurified serum. Such a medium can contain fatty acids or fats, amino acids (for example, non-essential amino acids), vitamins, growth factors, cytokines, antioxidants, 2-mercaptoethanol, 1-monothioglycerol, pyruvic acid, buffers, and inorganic salts. For example, when pluripotent stem cells are induced to differentiate to retinal tissues and the like using a basement membrane preparation such as Matrigel, serum medium can be used (Cell Stem Cell, 10(6), 771-775 (2012)).

The culture in the present invention is preferably carried out under zeno-free condition. The "zeno-free" means the condition in which components derived from a biological species different from the biological species of cells to be cultured are excluded.

The medium used in the present invention is preferably the medium containing chemically defined components (Chemically defined medium: CDM) from a viewpoint of avoiding chemically undefined components from mixing in.

The "basement membrane-like structure" means a thin membranous structure constituted by extracellular matrices. The basement membrane in a living organism is formed on the basal side of epithelial cells. Examples of the component of the basement membrane include type IV collagen, laminin, heparan sulfate proteoglycan (perlecan), entactin/nidogen, cytokines, and growth factors. The presence of a basement membrane in living organism-derived tissues and in cell clusters generated by the production method of the present invention and the like can be detected by, for example, tissue staining such as PAM stain, and a technique such as immunohistochemistry that uses an antibody to structural components of the basement membrane (anti-laminin antibody, anti-type IV collagen antibody or the like).

The "basement membrane preparation" refers to, when desired cells that have a basement membrane forming ability are seeded thereon and cultured, those including basement membrane structural components that have a function to control epithelial cell-like cell form, differentiation, growth, motility, functional expression and the like. For example, when cells and tissues produced by the present invention are dispersed to further carry out adherent culture, the culture can be carried out in the presence of a basement membrane preparation. The "basement membrane structural component" herein refers to thin membranous extracellular matrix molecules present between the epithelial cell layer, the stromal cell layer or the like in animal tissues. A basement membrane preparation can be generated by, for example, removing cells that have a basement membrane forming ability and are adhering onto the support through the basement membrane from the support using a solution that has a lipid solubilizing ability of such cells or an alkali solution. Examples of the basement membrane preparation include products commercially available as basement membrane formulations (for example, Matrigel (manufactured by Corning: hereinafter also described as Matrigel)) and Geltrex (manufactured by Thermo Fisher Scientific), and those containing extracellular matrix molecules known as the basement membrane components (for example, laminin, type IV collagen, heparan sulfate proteoglycan, and entactin).

In the present invention, a basement membrane preparation such as Matrigel (manufactured by Corning), which is extracted from tissues or cells of Engelbreth-Holm-Swarm (EHS) mouse sarcoma or the like and solubilized, can be used for the culture of cells or tissues. Similarly, solubilized human amniotic membrane (manufactured by Bioresource Application Institute, Co. Ltd.), human recombinant laminin produced by HEK293 cells (manufactured by BioLamina), human recombinant laminin fragments (Nippi. Inc.), human recombinant vitronectin (manufactured by Thermo Fisher Scientific) and the like can also be used as the basement membrane component used for cell culture but, from a viewpoint of avoiding components derived from a different biological species from mixing in and a viewpoint of avoiding risks of infections, recombinant proteins with identified components are preferable.

In the present invention, the "medium containing a substance X" and "in the presence of a substance X" mean medium to which an exogenous substance X is added or medium containing an exogenous substance X, or in the presence of an exogenous substance X. That is, even when cells or tissues present in such a medium are endogenously expressing, secreting, or producing such a substance X, the endogenous substance X is distinguished from an exogenous substance X and medium that does not contain an exogenous substance X should be understood as not categorized under the "medium containing a substance X". Further, a substance X in medium can have minor changes in the concentration due to the decomposition of a substance X or evaporation of medium.

For example, the "medium containing a sonic hedgehog signaling pathway-activating substance" refers to medium to which an exogenous sonic hedgehog signaling pathway-activating substance is added or medium containing an exogenous sonic hedgehog signaling pathway-activating substance.

The start of the culture in medium in which a concentration of a substance X is Y refers preferably to a point of time at which a concentration of the substance X in medium is uniform at Y, however, when a culture container is small enough (for example, 96-well plate, or culture in a culture medium of 200 µL or less), the point of time at which a medium addition procedure to be described later or a half medium exchange procedure or a complete medium exchange procedure is carried out to achieve a concentration of Y, should be understood as the start of the culture at a concentration of Y. Further, a concentration of a substance X in medium is Y includes a case in which an average concentration of X is Y throughout a certain culture period, a case in which a period during which a substance X is contained at a concentration of Y is 50% or more of a culture period, a case in which a period during which a substance X is contained at a concentration of Y is longer than the shortest period of the culture period estimated in each step and the like.

In the present invention, the "in the absence of a substance X" means the medium to which an exogenous substance X is not added or medium not containing an exogenous substance X, or a state in which an exogenous substance X is not present.

In the present invention, the "A hours (A days) or more" refers to the inclusion of A hours (A days) and the time succeeding the A hours (A days). The "B hours (B days) or less" refers to the inclusion of B hours (B days) and the time preceding the B hours (B days).

In the present invention, the feeder cell refers to a cell that is allowed to coexist with stem cells but other than such stem cells when cultured. Examples of the feeder cell used for undifferentiated state maintenance culture of pluripotent stem cells include mouse embryonic fibroblasts (MEF), human fibroblasts, and SNL cells. For the feeder cell, growth suppressed feeder cells are preferable. Examples of the growth suppression treatment include growth suppressor (for example, mitomycin C and the like) treatment, UV irradiation and the like. The feeder cells used for undifferentiated state maintenance culture of pluripotent stem cells contribute to the undifferentiated state maintenance of pluripotent stem cells by secretion of a humoral factor (preferably undifferentiated state maintenance factor) and generation of a scaffold (extracellular substrate) for cell adhesion.

In the present invention, the absence of feeder cells (feeder-free) is to culture in the absence of feeder cells. Examples of the absence of feeder cells include a condition in which feeder cells are not added and a condition in which feeder cells are not substantially contained (for example, a ratio of the feeder cell number to the total cell number is 3% or less).

In the present invention, the "cell aggregate" refers to a cluster formed when cells dispersed in medium are grouped, in which the cells adhere to each other. Embryoid body, sphere, spheroid, and organoid are also encompassed in the cell aggregate. Preferably in the cell aggregate, plane attachment occurs between cells. In some embodiments, in a part or whole of an aggregate, cells may form a cell-cell junction or cell adhesion, such as an adherence junction. Examples of the "cell aggregate" in the present invention specifically include aggregates formed by cells that are created in step (1) in "2. Production method of cell clusters including olfactory receptor neurons or precursor cells thereof" to be described later and have been dispersed at the start of the suspension culture.

In the present invention, the "uniform aggregates" means that each aggregate has a certain size when culturing a plurality of aggregates, and in the case of evaluating the size of aggregate in terms of the maximum diameter length, the uniform aggregates mean that the dispersion of the maximum diameter length is small. More specifically, the "uniform aggregates" means that 75% or more of aggregates in the whole aggregate group is the average value of ±100%, preferably within a range of the average value of ±50%, and more preferably within a range of the average value of ±20%, of the maximum diameter in such an aggregate group.

In the present invention, the "uniform aggregates are formed" refers that when cells are allowed to group to form cell aggregates to be suspension cultured, cell aggregates in uniform sizes are formed by "quickly aggregating a certain number of dispersed cells".

The dispersion refers to the separation of cells and tissues by a dispersion treatment such as an enzymatic treatment or a physical treatment to small cell debris (2 cells or more and 100 cells or less, preferably 50 cells or less) or to single cells. The certain number of dispersed cells refers to a group of a certain number of cell debris or single cells.

Examples of the method for dispersing pluripotent stem cells include mechanical dispersion treatments, cell-dispersing liquid treatments, and cell protectant addition treatments. These treatments may be used in combination. Preferably, the cell-dispersing liquid treatment is carried out and subsequently the mechanical dispersion treatment is carried out.

Examples of the mechanical dispersion treatment method include pipetting treatments and scraping procedures using a scraper.

Examples of the cell-dispersing liquid used for the cell-dispersing liquid treatment include solutions containing either enzymes such as trypsin, collagenases, hyaluronidases, elastases, Pronase, DNase, or papain or a chelating agent such as ethylenediaminetetraacetic acid. A commercially available cell-dispersing liquid such as Accumax (manufactured by Innovative cell technologies) and TripLE Select (manufactured by Thermo Fisher Scientific) can also be used.

Examples of the cell protectant used for the cell protectant addition treatment include FGF signaling pathway-activating substances, heparin, Rho-associated protein kinase (ROCK) inhibitory substances, serum, and serum substitutes. Examples of the preferable cell protectant include ROCK inhibitory substances.

Examples of the method for dispersing pluripotent stem cells include a method in which colonies of pluripotent stem cells are treated with a cell-dispersing liquid (Accumax) in the presence of a ROCK inhibitory substance and further dispersed by pipetting.

In the production method of the present invention, it is preferable to quickly group pluripotent stem cells to form aggregates of pluripotent stem cells. When aggregates of pluripotent stem cells are formed in this way, cells to be induced to differentiate from the formed aggregates can be allowed to form the epithelium-like structure with good reproducibility. Examples of the experimental procedure for forming cell aggregates include a method in which cells are trapped in a small space using a plate with small wells (for example, a plate with wells having a bottom area of about 0.1 to 2.0 cm² in terms of flat bottom) or a micropore, and a method in which cells are aggregated by centrifuging in a short time using a small centrifuge tube. Examples of the plate with small wells include 24-well plates (an area having about 1.88 cm² in terms of flat bottom), 48-well plates (an area having about 1.0 cm² in terms of flat bottom), 96-well plates (an area having about 0.35 cm² in terms of flat bottom, an inner diameter of about 6 to 8 mm), and 384-well plates. Preferable is 96-well plates. Examples of the shape of a plate with small wells include, in terms of the bottom area shape when looking down wells from above, polygons, rectangles, ovals, and circles, with circles being preferable. Examples of the shape of a plate with small wells include, in terms of the bottom area shape when looking wells from the side, a structure having a high external periphery with an inner concave part being slightly dented is preferable such as U-bottoms, V-bottoms, and M-bottoms, with U-bottoms or V-bottoms being preferable, and V-bottoms being the most preferable. For the plate with small wells, cell culture dishes (for example, 60 mm to 150 mm dish, culture flask) with the bottom surface being concaved or dented can also be used. The bottom surface of a plate with small wells is cell non-adherent, and it is preferable that a bottom surface be coated with the above cell non-adhesiveness is used.

The formation of aggregates of pluripotent stem cells and the formation of epithelium-like structure in each cell forming aggregates can be confirmed based on the size and cell numbers, macroscopic form, microscopic form and homogeneity thereof by histostaining analyses of aggregates, expression of differentiation and undifferentiation markers and homogeneity thereof, expression control of differentiation markers and synchronicity thereof, reproducibility of differentiation efficiency between aggregates and the like.

The "tissue" refers to a structural body of a cell population having a structure in which a plurality kind of cells different in form and characteristic are arranged three-dimensionally in a certain pattern.

The "nervous tissue" means a tissue constituted by neural cells such as the formative stage or adult stage cerebrum, midbrain, cerebellum, spinal cord, retina, sensory nerves, or peripheral nerves.

In the present invention, the "neural epithelial tissue" refers to a nervous tissue that forms a layer-structured epithelial structure, and neural epithelial tissues in the nervous tissues can be evaluated for the abundance by bright field observation and the like using an optical microscope.

The "central nervous system" indicates a region where nervous tissues accumulate and plays a central role in the information process. In the vertebrates, the brain and spinal cord are included in the central nervous system.

The "neural cell" indicates a cell other than epidermal cells among the ectoderm-derived tissues. That is, the neural cell includes cells such as neural precursor cells, neurons, glia cells, neural stem cells, neuron precursor cells, and glial precursor cells. The neural cell also encompasses cells constituting retinal tissues (retinal cells) to be described later, retinal precursor cells, retinal layer-specific neurons, neuroretinal cells, and retinal pigment epithelial cells. Neural cells can be identified using Nestin, βIII tubulin (Tuj 1), PSA-NCAM, N-cadherin or the like as a marker.

The neuron is a functional cell that forms a neural circuit and contributes to the information transduction and can be identified using, as an indicator, the expression of an immature neuronal marker such as TuJ1, Dcx, or HuC/D and/or a mature neuronal marker such as Map2 or NeuN.

Examples of the glia cell include astrocytes, oligodendrocytes, and Muller glia. Examples of the marker for astrocytes include GFAP, the marker for oligodendrocytes include O4, and the marker for Muller glia include CRALBP.

The neural stem cell is a cell that has differentiation potency to neurons and glia cells (multipotency) and proliferation potency (also referred to as self-replication potency) while maintaining the multipotency. Examples of the marker for neural stem cell include Nestin, Sox2, Musashi, Hes family, and CD133 but these markers are markers for general precursor cells and not considered as markers specific to neural stem cells. The numbers of neural stem cells can be evaluated by the neurosphere assay and the clonal assay.

The neuron precursor cell is a cell that has proliferation potency, produces neurons and does not produce glia cells. Examples of the marker for neuron precursor cell include Tbr2 and Tα1. Alternatively, immature neuronal marker (TuJ1, Dcx, HuC/D)-positive and proliferation marker (Ki67, pH3, MCM)-positive cells can also be identified as neuron precursor cells.

The glial precursor cell is a cell that has proliferation potency, produces glia cells and does not produce neurons.

The neural precursor cell is a mass of precursor cells including neural stem cells, neuron precursor cells, and glial precursor cells and has proliferation potency and neuron and glia production potency. The neural precursor cells can be identified using Nestin, GLAST, Sox2, Sox1, Musashi, or Pax6 as a marker. Neural cell marker-positive and proliferation marker (Ki67, pH3, MCM)-positive cells can also be identified as neural precursor cells.

The "retinal tissue" means a retinal tissue in which cells such as visual cells, horizontal cells, bipolar cells, amacrine cells, retinal ganglion cells, and precursor cells thereof, or retinal precursor cells that constitute each retinal layer in the living retina are layered and three-dimensionally arranged in at least a plurality of kinds. Cells constituting either of the retinal layers can be confirmed respectively by a known method such as the expression presence or absence of a cell marker and a level thereof and the like.

The "retinal precursor cell" refers to a precursor cell that can differentiate to any mature retinal cells of visual cells, horizontal cells, bipolar cells, amacrine cells, retinal ganglion cells, and retinal pigment epithelial cells.

The visual cell precursor cell, horizontal cell precursor cell, bipolar cell precursor cell, amacrine cell precursor cell, retinal ganglion cell precursor cell, and retinal pigment epithelial precursor cell refer to the precursor cells that are determined to differentiate to visual cells, horizontal cells, bipolar cells, amacrine cells, retinal ganglion cells, and retinal pigment epithelial cells, respectively.

The "retinal layer-specific neuron" means a cell constituting a retinal layer and is a neuron specific to a retinal layer. Examples of the retinal layer-specific neuron include bipolar cells, retinal ganglion cells, amacrine cells, horizontal cells, visual cells, retinal pigment epithelial cells, rod cells, and pyramidal cells.

The "retinal cell" encompasses the above-mentioned retinal precursor cells and retinal layer-specific neurons.

Examples of the retinal cell marker include Rx (also referred to as Rax), Aldhla3, and Pax6 that are expressed in retinal precursor cells, Nkx2.1 that is expressed in precursor cells of hypothalamic neurons but not expressed in retinal precursor cells, Sox1 that is expressed in the hypothalamic neuroepithelium but not expressed in the retina, and Crx and Blimp 1 that are expressed in precursor cells of visual cells. Examples of the marker for retinal layer-specific neuron include Chx10, PKCα and L7 that are expressed in bipolar cells, Tuj 1 and Brn3 that are expressed in retinal ganglion cells, Calretinin that is expressed in amacrine cells, Calbindin that is expressed in horizontal cells, Rhodopsin and Recoverin that are expressed in mature visual cells, Nrl that is expressed in rod cells, Rxr-gamma that is expressed in pyramidal cells, REP65 and Mitf that are expressed in retinal pigment epithelial cells.

The "cerebral tissue" means a tissue in which cells constituting the fetal stage or adult cerebrum (for example, cortical neural precursor cells, dorsal cortical neural precursor cells, abdominal cortical neural precursor cells, cerebral layer structure-specific neurons, the first layer neurons, second layer neurons, third layer neurons, forth layer neurons, fifth layer neurons, sixth layer neurons, glia cells (astrocytes and oligodendrocytes), and precursor cells thereof) are layered and three-dimensionally arranged in one kind or a plurality of kinds. The cerebrum in the fetal stage is also called prosencephalon or telencephalon. The presence of respective cells can be confirmed by a known method such as the expression presence or absence of a cell marker and a level thereof and the like.

The "cerebral layer" means each layer constituting the adult cerebrum or fetal stage cerebrum, and specifically, examples include the molecular layer, external granular layer, external pyramidal cell layer, inner granular layer, neuron layer (inner pyramidal cell layer), multiform cell layer, first layer, second layer, third layer, forth layer, fifth layer, sixth layer, cortex zone, intermediate zone, subventricular zone, and ventricular zone.

Examples of the "cortical neural precursor cell" include neuronal precursor cells, the first layer neuronal precursor cells, second layer neuronal precursor cells, third layer neuronal precursor cells, fourth layer neuronal precursor cells, fifth layer neuronal precursor cells, sixth layer neuronal precursor cells, astrocyte precursor cells, and oligodendrocyte precursor cells. Each of the cells is a precursor cell that is determined to differentiate to the first layer neurons, second layer neurons, third layer neurons, fourth layer neurons, fifth layer neurons, sixth layer neurons, astrocytes, and oligodendrocytes.

The "cortical neural precursor cell" includes multi-potent neural stem cells that have differentiation potency (pluripotency) to at least a plurality of differentiation lineages among the first layer neurons, second layer neurons, third layer neurons, fourth layer neurons, fifth layer neurons, sixth layer neurons, astrocytes, and oligodendrocytes.

The "cerebral layer-specific neuron" means cells constituting the cerebral layers and a neuron specific to the cerebral layers. Examples of the cerebral layer-specific neuron include the first layer neurons, second layer neurons, third layer neurons, fourth layer neurons, fifth layer neurons, sixth layer neurons, cerebral excitatory neurons, and cerebral inhibitory neurons.

Examples of the cerebral cell marker include FoxG1 (also known as Bf1) that is expressed in cerebral cells, Sox2 and Nestin that are expressed in cortical neural precursor cells, Pax6 and Emx2 that are expressed in dorsal cortical neural precursor cells, Dlx1, Dlx2, and Nkx2.1 that are expressed in abdominal cortical neural precursor cells, Tbr2 that is expressed in neuronal precursor cells, Tbr1 that is expressed in Nex, Svet1, and the sixth layer neurons, Ctip2 that is expressed in the fifth layer neurons, RORβ that is expressed in the fourth layer neurons, Cux1 or Brn2 that is expressed in the third layer neurons or the second layer neurons, and Reelin that is expressed in the first layer neurons.

The "olfactory cortex" is a region of the cerebrum that receives a monosynaptic input from the olfactory bulb and involved with the olfactory information processing.

Examples of the gene and marker that are expressed in the olfactory cortex include Tbr1, FoxP2, Ctip2, Nor1 (NR4a3), DAARP-32, CUX1, Brn2, and CART.

The "basal ganglia" is a mass of nerve nuclei present in a region of the cerebrum. Examples of the nerve nucleus included in the basal ganglia include striatum, pallidum, subthalamic nucleus, and substantia nigra.

The "ganglionic eminence" is a structure consisting of neural cells formed in the cerebral ventricles of a developing embryo. The ganglionic eminence becomes the base of adult basal ganglia and additionally produces a plurality kind of neurons, and these cells migrate to various parts of the central nervous system during developing.

The "rostral migratory stream" indicates a phenomenon and stream by which new neurons produced from neural stem cells of the subventricular zone migrate to the olfactory bulb. The neurons that are migrating through the rostral migratory stream can be detected by, for example, using a migrating neuronal marker such as PSA-NCAM or Dcx.

The "olfactory bulb" means a region of the central nervous system that is present at the tip of the cerebrum, receives an input from olfactory receptor cells present in the olfactory epithelium and is involved with the olfactory information processing. Cells present in the olfactory bulb form layer structures, which are called, in order from the surface layer, the olfactory nerve layer, glomerular layer, external plexiform layer, mitral cell layer, internal plexiform layer, and granule cell layer. In neurons of the olfactory bulb, there are mitral cells and tufted cells as the excitatory neurons, and there are periglomerular cells and granule cells as the inhibitory neurons (intermediate neurons).

Examples of the gene and marker that are expressed in the olfactory bulb include Arx, Tbr1, Tbr2/EOMES, Tbx21, and Iba1.

The "non-neural epithelial tissue" indicates a tissue other than neural epithelial tissues among the tissues having the epithelial structure. The epithelial tissues are also formed from any germ layers among ectoderm, mesoderm, endoderm, and trophectoderm. The epithelial tissues include the epithelium, mesothelium, and endothelium. Examples of the tissue included in the non-neural epithelial tissue include the epidermis, corneal epithelium, nasal epithelium (including olfactory epithelium), oral epithelium, tracheal epithelium, bronchial epithelium, respiratory epithelium, renal epithelium, renal cortical epithelium, and placental epithelium.

The epithelial tissues are typically linked by various cell-cell junctions and form tissues that have a layer structure of a single layer or stratified layers. The confirmation of the presence or absence of these epithelial tissues and the quantification of abundance can be carried out by observance using an optical microscope or a technique such as immunohistochemistry that uses an antibody to an epithelial cell marker (anti-E-Cadherin antibody, anti-N-Cadherin antibody, anti-EpCAM antibody or the like).

The "placode" indicates an organ primordium formed primarily during the development process of the vertebrates when a part of the epidermal ectoderm is thickened. Examples of the tissue derived from placodes include the lens, olfactory epithelium, inner ear, trigeminal nerve, and pituitary gland. Examples of the marker for placodes or preplacode regions, which are precursor tissues thereof, include Six1, Six4, Dlx5, and Eya2.

The "olfactory epithelium" is an epithelial tissue present in the nasal cavity and indicates an olfactory organ by which a living organism senses smell information. The olfactory epithelium is one of the tissues derived from a placode, expresses an olfactory receptor and is constituted by cells such as olfactory receptor neurons that sense volatile molecules in the air, supporting cells that support olfactory receptor neurons, basal cells which are stem cells and precursor cells of the olfactory epithelium, Bowman's gland cells that secret mucus. The olfactory epithelium is morphologically classified into the surface layer, intermediate layer, and basal layer, and supporting cells are present in the surface layer, olfactory receptor neurons are present in the intermediate layer, and basal cells are present in the basal layer. The Bowman's glands form branched tubular alveolar glands in the olfactory epithelium and are scattered around.

The "precursor tissue of the olfactory epithelium" includes the olfactory epithelial placode. Examples of the gene and marker that are expressed in the olfactory epithelium and olfactory epithelial placode include, in addition to the makers of the above placode regions and preplacode regions, Pax6, Otx2, FoxG1 (also known as Bf1), Sox2, Pou2f1, Sp8, Chd7, N-Cadherin, E-Cadherin, EpCAM, CK18, and PDGFRβ.

The vomeronasal organ is one kind of the olfactory system tissues and is an olfactory organ a living organism has separately from the olfactory epithelium. The vomeronasal organ is also called Jacobson's organ. The vomeronasal organ in the mammals has a different role from the olfactory epithelium that senses general volatile materials but has abundant neurons that receive a pheromone-like substance.

The "olfactory system tissue" means a tissue in which the fetal stage or adult olfactory system tissues, such as cells constituting the olfactory epithelium (for example, olfactory receptor neurons, supporting cells, basal cells, Bowman's gland cells, olfactory ensheathing glias, and precursor cells thereof), cells constituting the olfactory bulb, and cells constituting the olfactory cortex, are layered and three-dimensionally arranged in one kind or at least a plurality of kinds. The presence of respective cells can be confirmed by a known method such as the expression presence or absence of a cell-specific gene and a level thereof.

The "olfactory receptor neuron (ORN)" indicates a neuron that is present in the olfactory epithelium intermediate layer of the nasal mucous membrane and receives the sense of smells. The olfactory receptor neurons capture volatile molecules in the air using olfactory receptors located in the surface cilia. The olfactory receptor neuron is a bipolar sensory cell, and the olfactory information captured by olfactory receptors that are expressed on the peripheral side is propagated to the nerve axes called olfactory filaments on the central side. Olfactory filaments gather by several tens to form bundles, and the olfactory nerve refers to all these bundles. The olfactory filaments reach the cerebral olfactory bulb through the ethmoidal foramina located at the cribriform plate of ethmoid bone of the skull and synaptically bond to mitral cells and the like to transduce the olfactory information to the olfactory center in the brain.

Examples of the gene and marker that are expressed in olfactory receptor neurons and precursor cells thereof include cyclic nucleotide-gated channel α2 subunit (CNGA2), cyclic nucleotide-gated channel α4 subunit (CNGA4), cyclic nucleotide-gated channel β1b subunit, olfactory-specific G protein (Golf) adenylate cyclase, Olfactory Marker protein (OMP), NCAM, OCAM (NCAM-2), Ebf1, Ebf2, Ebf3, NeuroD, PGP9.5, Neuron Specific Enolase (NSE), Growth Associated Protein 43 (GAP-43/B50), vimentin, Lhx2, Id3, β-Tubulin III (Tuj), Calretinin, TrkB, Ctip2, Uncx, and olfactory receptor (OR).

The "supporting cell" indicates a many-columnar cylindrical epithelium-like cell present on the most top end side of the olfactory epithelium. The supporting cells are involved with the function fulfillment, survival, and maintenance of the epithelial structure and the like of other cells such as olfactory receptor neurons. The supporting cells of the olfactory epithelium are constituted by two kinds of cells, sustentacular cells and microvillar cells.

Examples of the gene and marker that are expressed in the supporting cells include Notch2, Notch3, Carbonyl reductase 2 (Cbr2), S-100, Ezrin, Reep6, Sox2, Tyro3, CYP2A6, SUS-1, SUS-4, and EPAS1.

The "basal cell" indicates a cell present in the basal layer of the olfactory epithelium. The basal cells are morphologically classified into globose basal cells (GBC) and horizontal basal cells (HBC). Of these two kinds of cells, the globose basal cells are precursor cells, and stem cells in action that constantly cause the cell division and supply new olfactory receptor neurons. On the other hand, the horizontal basal cells are stem cells typically in a state in which the cell cycle of the cell division is halted and in a dormant state but activated when damages of a large scale are caused on the olfactory epithelium.

Examples of the gene and marker that are expressed in the horizontal basal cells include p63, cytokeratin 5, cytokeratin 14, and ICAM-1.

Examples of the gene and marker that are expressed in the globose basal cells include GAP43, GBC-1, Lgr5, Ascl1, LSD1, and SEC8.

The "Bowman's gland cell" indicates a cell constituting the Bowman's gland (olfactory gland). The Bowman's gland is a branched narrow tubular tissue present in the olfactory epithelium and has a function of secreting a protective mucus for the olfactory epithelium.

Examples of the gene and marker that are expressed in the Bowman's gland cells include Sox9, E-Cadherin, Aquaporin5, Ascl3, and cytokeratin 18.

The "olfactory ensheathing glia (OEG)" indicates a kind of the glia cells present in the olfactory epithelium and olfactory bulb. The olfactory ensheathing glias are cells that release neurotrophic factors such as BDNF and NGF and are involved with the constant regeneration of olfactory nerves.

Examples of the gene and marker that are expressed in the olfactory ensheathing glias include p75NTR, S100β, Sox10, GFAP, BLBP, Aquaporin1, and Integrin α7.

The "lateral olfactory epithelium" is a region around the olfactory epithelium and indicates the region continuous with non-neural epithelium other than the olfactory epithelium. The "medial olfactory epithelium" in the present invention indicates the central region of the olfactory epithelium surrounded by the lateral olfactory epithelium. It is known that a ratio of cells constituting the lateral olfactory epithelium and the medial olfactory epithelium is different (Development, 2010, 137:2471-2481). Examples of the gene that is expressed in the lateral olfactory epithelium at a high level include Pbx1/2/3, Meis1, and βIV Tubulin. Examples of the gene that is expressed in the medial olfactory epithelium at a high level include Tuj 1, Ascl1, and Sox2.

The "gonadotropin releasing hormone-positive neuron" is a neuron that is primarily present in the hypothalamus of the central nervous system and secrets gonadotropin releasing hormone (GnRH) that is associated with the control of the reproductive organs. The gonadotropin releasing hormone-positive neurons are developed in the olfactory epithelium in the prenatal stage and moved to the central nervous system.

Examples of the gene and marker that are expressed in the gonadotropin releasing hormone include gonadotropin releasing hormone (GnRH).

The "bone tissue" indicates a cell constituting bones and bone matrices constituted by calcium phosphate, type I collagen and the like. The cells constituting bones include osteocytes, osteoclasts, and osteoblasts.

The "skull" indicates bone tissues present in the head. The skull has functions of maintaining the form and structure of the face and head and protecting the central nervous system.

The "ethmoidal bone" indicates one of the bone tissues constituting the skull. The human ethmoidal bone is present between both orbits.

Examples of the gene and marker that are expressed in the cells constituting bones including the skull include Msx2, Runx2, Osterix, Osteocalcin, and Osteopontin. Additionally, the presence of bone tissues in the tissues can be confirmed by a technique such as alizarin red staining and alkaline phosphatase staining.

The "receptor protein" indicates a protein that is present in the cell membrane, cytoplasm or nucleus, binds to a substance such as hormones, cytokines, cell growth factors, and compounds, and triggers reactions of various cells.

The "olfactory receptor" indicates a receptor protein that is expressed in the olfactory receptor neurons and other cells and involved with sensing a compound or the like.

[2. Method for producing cell clusters including olfactory receptor neurons or precursor cells thereof]

The present invention provides a method for producing cell clusters including olfactory receptor neurons or precursor cells thereof. Hereinafter, it is also referred to as the production method of the present invention.

An embodiment of the production method of the present invention is a method for producing cell clusters including olfactory receptor neurons or precursor cells thereof comprising the following steps.

A step (1) of suspension culturing pluripotent stem cells in the presence of a first Wnt signaling pathway inhibitory substance to form cell aggregates;
a step (2) of suspension culturing the cell aggregates obtained in step (1) in the presence of a BMP signaling pathway-activating substance; and
a step (3a) of suspension culturing the cell aggregates obtained in step (2) in the presence of an FGF signaling pathway-activating substance to obtain the above cell clusters.

A preferable embodiment of the production method of the present invention is a method for producing cell clusters including olfactory receptor neurons or precursor cells thereof comprising the following steps.

A step (a), before a step (1), of culturing pluripotent stem cells in the absence of feeder cells in medium containing 1) at least one selected from the group consisting of TGFβ family signaling pathway inhibitory substances and sonic hedgehog signaling pathway-activating substances and 2) an undifferentiated state maintenance factor;
step (1) of suspension culturing pluripotent stem cells in the presence of a first Wnt signaling pathway inhibitory substance to form cell aggregates;
a step (2) of suspension culturing the cell aggregates obtained in step (1) in the presence of a BMP signaling pathway-activating substance; and,
a step (3a) of suspension culturing the cell aggregates obtained in step (2) in the presence of an FGF signaling pathway-activating substance to obtain the above cell clusters.

A preferable embodiment of the production method of the present invention is a method for producing cell clusters including olfactory receptor neurons or precursor cells thereof comprising the following steps.

A step (1) of suspension culturing pluripotent stem cells in the presence of a first Wnt signaling pathway inhibitory substance to form cell aggregates;
a step (2) of suspension culturing the cell aggregates obtained in step (1) in the presence of a BMP signaling pathway-activating substance;
a step (3a) of suspension culturing the cell aggregates obtained in step (2) in the presence of an FGF signaling pathway-activating substance; and
a step (3c) of culturing the cell aggregates obtained in step (3a) in the presence of an FGF signaling pathway-activating substance and a BMP signaling pathway inhibitory substance to obtain the above cell clusters.

A preferable embodiment of the production method of the present invention is a method for producing cell clusters including olfactory receptor neurons or precursor cells thereof comprising the following steps.

A step (a), before a step (1), of culturing pluripotent stem cells in the absence of feeder cells in medium containing 1) at least one selected from the group consisting of TGFβ family signaling pathway inhibitory substances and sonic hedgehog signaling pathway-activating substances and 2) an undifferentiated state maintenance factor;
step (1) of suspension culturing pluripotent stem cells in the presence of a first Wnt signaling pathway inhibitory substance to form cell aggregates;
a step (2) of suspension culturing the cell aggregates obtained in step (1) in the presence of a BMP signaling pathway-activating substance;
a step (3a) of suspension culturing the cell aggregates obtained in step (2) in the presence of an FGF signaling pathway-activating substance; and
a step (3c) of culturing the cell aggregates obtained in step (3a) in the presence of an FGF signaling pathway-activating substance and a BMP signaling pathway inhibitory substance to obtain the above cell clusters.

A further embodiment of the production method of the present invention is a method for producing cell clusters including olfactory receptor neurons or precursor cells thereof comprising the following steps.

A step (1) of suspension culturing pluripotent stem cells in the presence of a first Wnt signaling pathway inhibitory substance to form cell aggregates;
a step (2) of suspension culturing the cell aggregates obtained in step (1) in the presence of a BMP signaling pathway-activating substance; and
a step (3b) of suspension culturing the cell aggregates obtained in step (2) in the presence of a BMP signaling pathway inhibitory substance to obtain the above cell clusters.

A preferable embodiment of the production method of the present invention is a method for producing cell clusters including olfactory receptor neurons or precursor cells thereof comprising the following steps.

A step (1) of suspension culturing pluripotent stem cells in the presence of a first Wnt signaling pathway inhibitory substance to form cell aggregates;
a step (2) of suspension culturing the cell aggregates obtained in step (1) in the presence of a BMP signaling pathway-activating substance;
a step (3b) of suspension culturing the cell aggregates obtained in step (2) in the presence of a BMP signaling pathway inhibitory substance; and
a step (3d) of suspension culturing the cell aggregates obtained in step (3b) in the absence of a BMP signaling pathway inhibitory substance to obtain the above cell clusters.

A more preferable embodiment of the production method of the present invention is a method for producing cell clusters including olfactory receptor neurons or precursor cells thereof comprising the following steps.

A step (a), before a step (1), of culturing pluripotent stem cells in the absence of feeder cells in medium containing 1) at least one selected from the group consisting of TGFβ family signaling pathway inhibitory substances and sonic hedgehog signaling pathway-activating substances and 2) an undifferentiated state maintenance factor;
step (1) of suspension culturing pluripotent stem cells in the presence of a first Wnt signaling pathway inhibitory substance to form cell aggregates;
a step (2) of suspension culturing the cell aggregates obtained in step (1) in the presence of a BMP signaling pathway-activating substance; and
a step (3b) of suspension culturing the cell aggregates obtained in step (2) in the presence of a BMP signaling pathway inhibitory substance to obtain the above cell clusters.

A further preferable embodiment of the production method of the present invention is a method for producing cell clusters including olfactory receptor neurons or precursor cells thereof comprising the following steps.

A step (a), before a step (1), of culturing pluripotent stem cells in the absence of feeder cells in medium containing 1) at least one selected from the group consisting of TGFβ family signaling pathway inhibitory substances and sonic hedgehog signaling pathway-activating substances and 2) an undifferentiated state maintenance factor;
step (1) of suspension culturing pluripotent stem cells in the presence of a first Wnt signaling pathway inhibitory substance to form cell aggregates;
a step (2) of suspension culturing the cell aggregates obtained in step (1) in the presence of a BMP signaling pathway-activating substance;
a step (3b) of suspension culturing the cell aggregates obtained in step (2) in the presence of a BMP signaling pathway inhibitory substance; and
a step (3d) of suspension culturing the cell aggregates obtained in step (3b) in the absence of a BMP signaling pathway inhibitory substance to obtain the above cell clusters.

A further embodiment of the production method of the present invention is a method for producing cell clusters including olfactory receptor neurons or precursor cells thereof comprising the following steps.

A step (1) of suspension culturing pluripotent stem cells in the presence of a first Wnt signaling pathway inhibitory substance to form cell aggregates;
a step (2) of suspension culturing the cell aggregates obtained in step (1) in the presence of a BMP signaling pathway-activating substance; and
a step (3c) of suspension culturing the cell aggregates obtained in step (2) in the presence of an FGF signaling pathway-activating substance and a BMP signaling pathway inhibitory substance to obtain the above cell clusters.

A preferable embodiment of the production method of the present invention is a method for producing cell clusters including olfactory receptor neurons or precursor cells thereof comprising the following steps.

A step (1) of suspension culturing pluripotent stem cells in the presence of a first Wnt signaling pathway inhibitory substance to form cell aggregates;
a step (2) of suspension culturing the cell aggregates obtained in step (1) in the presence of a BMP signaling pathway-activating substance;
a step (3c) of suspension culturing the cell aggregates obtained in step (2) in the presence of an FGF signaling pathway-activating substance and a BMP signaling pathway inhibitory substance; and
a step (3d) of suspension culturing the cell aggregates obtained in step (3c) in the absence of a BMP signaling pathway inhibitory substance to obtain the above cell clusters.

A more preferable embodiment of the production method of the present invention is a method for producing cell clusters including olfactory receptor neurons or precursor cells thereof comprising the following steps.

A step (a), before a step (1), of culturing pluripotent stem cells in the absence of feeder cells in medium containing 1) at least one selected from the group consisting of TGFβ family signaling pathway inhibitory substances and sonic hedgehog signaling pathway-activating substances and 2) an undifferentiated state maintenance factor;
step (1) of suspension culturing pluripotent stem cells in the presence of a first Wnt signaling pathway inhibitory substance to form cell aggregates;
a step (2) of suspension culturing the cell aggregates obtained in step (1) in the presence of a BMP signaling pathway-activating substance; and
a step (3c) of suspension culturing the cell aggregates obtained in step (2) in the presence of an FGF signaling pathway-activating substance and a BMP signaling pathway inhibitory substance to obtain the above cell clusters.

A further preferable embodiment of the production method of the present invention is a method for producing cell clusters including olfactory receptor neurons or precursor cells thereof comprising the following steps.

A step (a), before a step (1), of culturing pluripotent stem cells in the absence of feeder cells in medium containing 1) at least one selected from the group consisting of TGFβ family signaling pathway inhibitory substances and sonic hedgehog signaling pathway-activating substances and 2) an undifferentiated state maintenance factor;
step (1) of suspension culturing pluripotent stem cells in the presence of a first Wnt signaling pathway inhibitory substance to form cell aggregates;
a step (2) of suspension culturing the cell aggregates obtained in step (1) in the presence of a BMP signaling pathway-activating substance;
a step (3c) of suspension culturing the cell aggregates obtained in step (2) in the presence of an FGF signaling pathway-activating substance and a BMP signaling pathway inhibitory substance; and
a step (3d) of suspension culturing the cell aggregates obtained in step (3c) in the absence of a BMP signaling pathway inhibitory substance to obtain the above cell clusters.

The cell clusters to be produced by the above production methods are as described later in "3. Cell clusters including olfactory receptor neurons or precursor cells thereof.

### <Step (a)>

Step (a) of culturing pluripotent stem cells in the absence of feeder cells in medium containing 1) at least one selected from the group consisting of TGFβ family signaling pathway inhibitory substances and sonic hedgehog signaling pathway-activating substances and 2) an undifferentiated state maintenance factor will be described.

In step (a), pluripotent stem cells are cultured in the presence of at least one selected from the group consisting of TGFβ family signaling pathway inhibitory substances and sonic hedgehog signaling pathway-activating substances and then subjected to the suspension culture of step (1), whereby the state of the pluripotent stem cells changes, thereby enabling highly efficient production of the cell aggregates that have improved efficiency in formation of non-neural epithelial tissues, enhanced quality of the aggregates, are easy to differentiate, are less likely to have cell death, and maintain undifferentiated potency inside thereof densely.

Step (a) is carried out in the absence of feeder cells. The absence of feeder cells (feeder free) in the present invention means a condition in which feeder cells are not substantially contained (for example, a ratio of the feeder cell number to the total cell number is 3% or less).

The medium used in step (a) under the feeder free condition is not particularly limited as long as medium (feeder free medium) enables undifferentiated state maintenance culture of pluripotent stem cells.

Many synthetic media are developed and commercially available as feeder free media and examples include Essential 8 medium. Essential 8 medium contains, as additives in DMEM/F12 medium, L-ascorbic acid-2-phosphate magnesium (64 mg/l), sodium selenium (14 µg/1), insulin (19.4 mg/l), NaHCO₃ (543 mg/l), transferrin (10.7 mg/l), bFGF (100 ng/mL), and a TGFβ family signaling pathway-activating substance (TGFβ1 (2 ng/mL) or Nodal (100 ng/mL)) (Nature Methods, 8, 424-429 (2011)). Examples of the commercially available feeder free medium include Essential 8 (manufactured by Thermo Fisher Scientific), S-medium (manufactured by DS Pharma Biomedical Co., Ltd.), StemPro (manufactured by Thermo Fisher Scientific), hESF9, mTeSR1 (manufactured by STEMCELL Technologies), mTeSR2 (manufactured by STEMCELL Technologies), TeSR-E8 (manufactured by STEMCELL Technologies), and StemFit (manufactured by AJINOMOTO Co., Inc.). In step (a), the present invention can be carried out easily by using these. StemFit medium contains bFGF as an undifferentiated state maintenance component (Scientific Reports (2014) 4, 3594).

The medium used in step (a) can be serum medium or serum-free medium. The medium used in step (a) is preferably serum-free medium from a viewpoint of avoiding chemically undefined components from mixing in. The medium can contain a serum substitute.

The medium used in step (a) contains an undifferentiated state maintenance factor to enable the undifferentiated state maintenance culture. The undifferentiated state maintenance factor is not particularly limited as long as a substance has the action to suppress the differentiation of pluripotent stem cells. Examples of the undifferentiated state maintenance factor commonly used by those skilled in the art include FGF signaling pathway-activating substances, TGFβ family signaling pathway-activating substances, and insulin for primed pluripotent stem cells (for example, human ES cells and human iPS cells). Examples of the FGF signaling pathway-activating substance specifically include fibroblast growth factors (for example, bFGF, FGF4, and FGF8). Additionally, examples of the TGFβ family signaling pathway-activating substance include TGFβ signaling pathway-activating substances and Nodal/Activin signaling pathway-activating substances. Examples of the TGFβ signaling pathway-activating substance include TGFβ1 and TGFβ2. Examples of the Nodal/Activin signaling pathway-activating substance include Nodal, Activin A, and Activin B. These substances can be used singly or in combination. When human pluripotent stem cells (for example, human ES cells and human iPS cells) are cultured, the medium in step (a) preferably contains bFGF as an undifferentiated state maintenance factor.

The undifferentiated state maintenance factor used in the present invention is typically a mammal's undifferentiated state maintenance factor. Examples of the mammal include those as described above. An undifferentiated state maintenance factor can have the interspecies cross reactivity among mammals, and hence an undifferentiated state maintenance factor of any mammals can be used as long as the undifferentiated state of pluripotent stem cells to be cultured can be maintained. The undifferentiated state maintenance factor used in the present invention is preferably an undifferentiated state maintenance factor of a mammal of the same species as cells to be cultured. For example, for the culture of human pluripotent stem cells, a human undifferentiated state maintenance factor (for example, bFGF, FGF4, FGF8, EGF, Nodal, Activin A, Activin B, TGFβ1, or TGFβ2) can be used. The "human protein X" used herein means that a protein X (undifferentiated state maintenance factor and the like) has an amino acid sequence of the protein X that is naturally expressed in a human living organism.

The undifferentiated state maintenance factor used in the present invention is preferably isolated.

The "isolation" means that a procedure for removing factors other than components and cells of interest is made and hence the naturally occurring state has been absent. Thus, the "isolated protein X" does not encompass an endogenous protein X contained in cells and tissues produced from the cells and tissues to be cultured and medium. The purity of the "isolated protein X" (a weight percentage of the protein X accounting for the total protein weight) is typically 70% or more, preferably 80% or more, more preferably 90% or more, further preferably 99% or more, and most preferably 100%.

The present invention comprises, in an embodiment, a step of providing an isolated undifferentiated state maintenance factor. Additionally, the present invention comprises, in an embodiment, a step of exogenously (or extrinsically) adding an isolated undifferentiated state maintenance factor to the medium used in step (a). Alternatively, an undifferentiated state maintenance factor can be added in advance to the medium used in step (a).

The undifferentiated state maintenance factor concentration in the medium used in step (a) is a concentration that is sufficient to maintain the undifferentiated state of pluripotent stem cells to be cultured and can be suitably set by those skilled in the art. For example, when bFGF is used as an undifferentiated state maintenance factor in the absence of feeder cells, a concentration thereof is typically about 4 ng/mL to about 500 ng/mL, preferably about 10 ng/mL to about 200 ng/mL, and more preferably about 30 ng/mL to about 150 ng/mL.

The culture of pluripotent stem cells in step (a) can be carried out by either condition of suspension culture or adherent culture but preferably carried out by adherent culture.

In the culture of pluripotent stem cells under the feeder free condition in step (a), suitable matrices can be used as scaffolds to provide pluripotent stem cells with scaffolds in place of feeder cells. Pluripotent stem cells are adherent cultured in a cell vessel that has the surface coated with matrices as the scaffold.

Examples of the matrix usable as the scaffold include laminin (Nat Biotechnol. 28, 611-615 (2010)), laminin fragments (Nat Commun 3, 1236 (2012)), basement membrane preparations (Nat Biotechnol 19, 971-974 (2001)), gelatins, collagen, heparan sulfate proteoglycan, entactin, and vitronectin.

The "laminins" are heterotrimeric molecules consisting of α, β, and γ chains and extracellular matrix proteins that have isoforms with different compositions of the subunit chains. Specifically, laminins are heterotrimer combinations of 5 variants of the α chain, 4 variants of the β chain, and 3 variants of the γ chain and have about 15 kinds of isoforms. The laminins are named according to the combinations of respective numbers of the α chains (α1 to α5), β chains (β1 to β4), and γ chains (γ1 to γ4). For example, a laminin composed of a combination the α5 chain, the β1 chain, and the γ1 chain is called laminin511. In the present invention, laminin511 is preferably used (Nat Biotechnol 28, 611-615 (2010)).

The laminin fragment used in the present invention is not particularly limited as long as it has the adhesiveness to pluripotent stem cells and enables the maintenance culture of pluripotent stem cells under the feeder free condition but preferably E8 fragment. The laminin E8 fragment was identified as the fragment that has strong cell adhesion activity among the fragments obtained by digesting laminin511 with elastase (EMBO J., 3:1463-1468, 1984, J. Cell Biol., 105:589-598, 1987). In the present invention, the E8 fragment of laminin511 is preferably used (Nat Commun 3, 1236 (2012), Scientific Reports 4,3549 (2014)). The laminin E8 fragment used in the present invention is not required to be an elastase digested product of the laminin and can be a recombinant. Alternatively, those produced by genetically modified animals (silkworm and the like) are acceptable. A recombinant laminin fragment is preferably used in the present invention from a viewpoint of avoiding undefined components from mixing in. The E8 fragment of laminin511 is commercially available and can be purchased from, for example, Nippi. Inc.

The laminin or laminin fragment used in the present invention is preferably isolated from a viewpoint of avoiding undefined components from mixing in.

In the culture of pluripotent stem cells under the feeder free condition in step (a), pluripotent stem cells are adherent cultured in a cell vessel that has the surface coated with preferably isolated laminin511 or the E8 fragment of laminin511, more preferably with the E8 fragment of laminin511.

The culture time of pluripotent stem cells in step (a) is not particularly limited within the range in which the effect to enhance the quality of aggregates to be formed in the subsequent step (1) is achievable and typically 0.5 to 144 hours, preferably 2 to 96 hours, more preferably 6 to 48 hours, further preferably 12 to 48 hours, particularly preferably 18 to 28 hours, and, for example, 24 hours.

That is, step (a) is started 0.5 to 144 hours, and preferably 18 to 28 hours before the start of step (1), and step (1) is carried out continuously after the completion of step (a).

The culture conditions such as the culture temperature and CO₂ concentration in step (a) can be suitably set. The culture temperature is, for example, from about 30°C to about 40°C, and preferably about 37°C. Further, the CO₂ concentration is, for example, from about 1% to about 10%, and preferably about 5%.

In a preferable embodiment, human pluripotent stem cells are adherent cultured in serum-free medium containing bFGF in the absence of feeder cells. Such an adherent culture is preferably carried out in a cell vessel that has the surface coated with preferably laminin511, the E8 fragment of laminin511, or vitronectin. Such an adherent culture is preferably carried out using StemFit as feeder free medium.

In a preferable embodiment, human pluripotent stem cells are suspension cultured in serum-free medium containing bFGF in the absence of feeder cells. In such a suspension culture, the human pluripotent stem cells can form aggregates of the human pluripotent stem cells.

The sonic hedgehog (hereinafter also referred to as "Shh") signaling pathway-activating substance is a substance that can reinforce the signaling mediated by Shh. Examples of the Shh signaling pathway-activating substance include proteins belonging to the Hedgehog family (for example, Shh and Ihh), Shh receptors, Shh receptor agonists, Smo agonists, Purmorphamine (9-cyclohexyl-N-[4-(morpholinyl)phenyl]-2-(1-naphthalenyloxy)-9H-purin-6-amine), GSA-10 (propyl 4-(1-hexyl-4-hydroxy-2-oxo-1,2-dihydroquinoline-3-carboxamido)benzoate), Hh-Ag1.5, 20(S)-Hydroxycholesterol, and SAG (Smoothened Agonist:N-Methyl-N'-(3-pyridinylbenzyl)-N'-(3-chlorobenzo[b]thiophene-2-carbonyl)-1,4-diaminocyclohexane). These substances can be used singly or in combination.

The Shh signaling pathway-activating substance preferably includes at least one selected from the group consisting of SAG, Purmorphamine, and GSA-10, and more preferably includes SAG. The concentration of the Shh signaling pathway-activating substance in medium can be suitably set to be a range in which the above-mentioned effect is achievable. SAG, in step (a), is typically used in a concentration of about 1 nM to about 2000 nM, preferably about 10 nM to about 1000 nM, more preferably about 10 nM to about 700 nM, further preferably about 50 nM to about 700 nM, particularly preferably about 100 nM to about 600 nM, and most preferably about 100 nM to about 500 nM. Further, when a Shh signaling pathway-activating substance other than SAG is used, it is desirable to use such a substance in a concentration that provides a Shh signaling acceleration activity equivalent to SAG of the concentration described previously. The sonic hedgehog signaling acceleration activity can be determined by a well-known method by those skilled in the art such as the reporter gene assay that focuses on the expression of the Gli1 gene (Oncogene (2007) 26, 5163-5168).

The TGFβ family signaling pathway (that is, TGFβ super family signaling pathway) is a signaling pathway transduced by the Smad family in a cell using a transforming growth factor β (TGFβ), Nodal/Activin, or BMP as a ligand.

The TGFβ family signaling pathway inhibitory substance indicates a substance that inhibits the TGFβ family signaling pathway, that is, the signaling pathway transduced by the Smad family, and specifically examples include TGFβ signaling pathway inhibitory substances, Nodal/Activin signaling pathway inhibitory substances, and BMP signaling pathway inhibitory substances. For the TGFβ family signaling pathway inhibitory substance, TGFβ signaling pathway inhibitory substances are preferable.

The TGFβ signaling pathway inhibitory substance is not particularly limited as long as a substance inhibits the signaling pathways that are caused by the TGFβ and can be nucleic acids, proteins or low molecular organic compounds. Examples of such a substance include substances directly act on the TGFβ (for example, proteins, antibodies, and aptamers), substances that suppress the expression of genes encoding the TGFβ (for example, antisense oligonucleotides and siRNA), substances that inhibit the binding of TGFβ receptors and the TGFβ, substances that inhibit physiological activities caused by the signaling by a TGFβ receptor (for example, TGFβ receptor inhibitors and Smad inhibitors). Examples of the protein that is known as the TGFβ signaling pathway inhibitory substance include Lefty.

For the TGFβ signaling pathway inhibitory substance, compounds well known by those skilled in the art can be used. Specifically, examples include Alk5/TGFβR1 inhibitors such as SB431542 (sometimes abbreviated as SB431 in the present description and drawings) (4-[4-(3,4-Methylenedioxyphenyl)-5-(2-pyridyl)-1H-imidazol-2-yl]benzamide), SB505124 (2-[4-(1,3-Benzodioxol-5-yl)-2-(1,1-dimethylethyl)-1H-imidazol-5-yl]-6-methylpyridine), SB525334 (6-[2-(1,1-Dimethylethyl)-5-(6-methyl-2-pyridinyl)-1H-imidazol-4-yl]quinoxaline), LY2157299 (4-[5,6-Dihydro-2-(6-methyl-2-pyridinyl)-4H-pyrrolo[1,2-b]pyrazol-3 -yl]-6-quinolinecarboxamide), LY2109761 (4-[5,6-dihydro-2-(2-pyridinyl)-4H-pyrrolo[1,2-b]pyrazol-3-yl]-7-[2-(4-morpholinyl)ethoxy]-quinoline), GW788388 (4-{4-[3-(Pyridin-2-yl)-1H-pyrazol-4-yl]-pyridin-2-yl}-N-(tetrahydro-2H-pyran-4-yl)benzamide), LY364947 (4-[3-(2-Pyridinyl)-1H-pyrazol-4-yl]quinoline), SD-208 (2-(5-Chloro-2-fluorophenyl)pteridin-4-yl)pyridin-4-yl amine), EW-7197 (N-(2-fluorophenyl)-5-(6-methyl-2-pyridinyl)-4-[1,2,4]triazolo[1,5-a]pyridin-6-yl-1H-Imidazole-2-methanamine), A83-01 (3-(6-Methylpyridin-2-yl)-4-(4-quinolyl)-1-phenylthiocarbamoyl-1H-pyrazole), and RepSox (2-[5-(6-Methylpyridin-2-yl)-1H-pyrazol-4-yl]-1,5-naphthyridine), and SMAD3 inhibitors such as SIS3 (1-(3,4-dihydro-6,7-dimethoxy-2(1H)-isoquinolinyl)-3-(1-methyl-2-phenyl-1H-pyrrolo[2,3-b]pyridin-3-yl)-2-propen-1-one). These substances can be used singly or in combination. SB431542 herein is a known compound as an inhibitor of a TGFβ receptor (ALK5) and an Activin receptor (ALK4/7) (that is, TGFβR inhibitor). SIS3 is a TGFβ signaling pathway inhibitory substance that inhibits the phosphorylation of SMAD3, which is an intracellular signaling factor under the control of TGFβ receptor.

The TGFβ signaling pathway inhibitory substance used in the present invention preferably includes an Alk5/TGFβR1 inhibitor. The Alk5/TGFβR1 inhibitor preferably includes at least one selected from the group consisting of SB431542, SB505124, SB525334, LY2157299, GW788388, LY364947, SD-208, EW-7197, A83-01, and RepSox, and further preferably includes SB431542 or A83-01.

The concentration of the TGFβ signaling pathway inhibitory substance in medium can be suitably set in a range in which the above-mentioned effect is achievable. When SB431542 is used as a TGFβ pathway inhibitory substance in step (a), SB431542 is used typically in a concentration of about 1 nM to about 100 µM, preferably about 10 nM to 100 µM, more preferably about 10 nM to about 50 µM, further preferably about 100 nM to about 50 µM, and particularly preferably about 1 µM to about 10 µM. When a TGFβ signaling pathway inhibitory substance other than SB431542 is used, it is desirable to use such an inhibitory substance in a concentration that provides a TGFβ signaling pathway inhibitory activity equivalent to SB431542 of the concentration described previously.

The culture conditions such as the culture temperature and CO₂ concentration in each of step (a) and steps (1) to (3) below can be suitably set. The culture temperature is about 30°C to about 40°C, and preferably about 37°C. The CO₂ concentration is about 1% to about 10%, and preferably about 5%.

### <Step (1)>

Step (1) of suspension culturing pluripotent stem cells maintained in an undifferentiated state, preferably pluripotent stem cells cultured in step (a), in the presence of a first Wnt signaling pathway inhibitory substance to form a cell aggregate will be described.

In the present invention, to discriminate from a second Wnt signaling pathway inhibitory substance described later, the Wnt signaling pathway inhibitory substance added in step (1) is also referred to as the first Wnt signaling pathway inhibitory substance.

Wnt signaling pathways are signaling pathways including a Wnt family protein as a ligand and primarily including Frizzled as a receptor. Examples of such signaling pathways include a canonical Wnt pathway, in which β-catenin transfers signals, and non-canonical Wnt pathways. Examples of non-canonical Wnt pathways include a Planar Cell Polarity (PCP) pathway, Wnt/Calcium pathway, Wnt-RAP1 pathway, Wnt-Ror2 pathway, Wnt-PKA pathway, Wnt-GSK3MT pathway, Wnt-aPKC pathway, Wnt-RYK pathway, and Wnt-mTOR pathway. In non-canonical Wnt pathways, there exist common signaling factors that are activated in other non-Wnt signaling pathways, and these factors are regarded as constituent factors for Wnt signaling pathways in the present invention, and inhibitory substances for those factors are also included in Wnt signaling pathway inhibitory substances.

In the present invention, the Wnt signaling pathway inhibitory substance is not limited as long as the inhibitory substance can suppress signaling evoked by Wnt family proteins. The inhibitory substance can be nucleic acids, proteins or low molecular organic compounds. Examples of such a substance can include substances that inhibit processing of Wnt and extracellular secretion thereof, substances that directly act on Wnt (for example, proteins, antibodies, aptamers), substances that suppress expression of a gene encoding Wnt (for example, antisense oligonucleotides, siRNA), substances that inhibit binding between a Wnt receptor and Wnt, and substances that inhibit physiological activity due to signaling by a Wnt receptor.

Examples of proteins known as Wnt signaling pathway inhibitory substances include proteins belonging to the secreted Frizzled Related Protein (sFRP) class (sFRP1 to sFRP5, Wnt Inhibitory Factor-1 (WIF-1), Cerberus), and proteins belonging to the Dickkopf (Dkk) class (Dkk1 to Dkk4, Kremen).

Compounds known to those skilled in the art can be used as a Wnt signaling pathway inhibitory substance. Examples of such a Wnt signaling pathway inhibitory substance include a Porcupine (PORCN) inhibitor, Frizzled inhibitor, Dishevelled (Dvl) inhibitor, Tankyrase (TANK) inhibitor, casein kinase 1 inhibitor, catenin responsive transcription inhibitor, p300 inhibitor, CREB-binding protein (CBP) inhibitor, and BCL-9 inhibitor (Am J Cancer Res. 2015; 5(8): 2344-2360). Examples of inhibitors for non-canonical Wnt pathways include a Calcium/calmodulin-dependent protein kinase II (CaMKII) inhibitor, TGF-β-activated kinase 1 (TAK1) inhibitor, Nemo-Like Kinase (NLK) inhibitor, LIM Kinase inhibitor, mammalian target of rapamycin (mTOR) inhibitor, c-Jun NH 2-terminal kinase (JNK) inhibitor, protein kinase C (PKC) inhibitor, Methionine Aminopeptidase 2 (MetAP2) inhibitor, Calcineurin inhibitor, nuclear factor of activated T cells (NFAT) inhibitor, and ROCK inhibitor. Examples of Wnt signaling pathway inhibitory substances action mechanisms of which have not been reported include KY02111 (N-(6-Chloro-2-benzothiazolyl)-3,4-dimethoxybenzenepropanamide) and KY03-I (2-(4-(3,4-dimethoxyphenyl)butanamide)-6-Iodobenzothiazole). These substances can be used singly or in combination.

Examples of PORCN inhibitors include IWP-2 (N-(6-Methyl-2-benzothiazolyl)-2-[(3,4,6,7-tetrahydro-4-oxo-3-phenylthieno[3,2-d]pyrimidin-2-yl)thio]-acetamide), IWP-3 (2-[[3-(4-fluorophenyl)-3,4,6,7-tetrahydro-4-oxothieno[3,2-d]pyrimidin-2-yl]thio]-N-(6-methyl-2-benzothiazolyl)-acetamide), IWP-4 (N-(6-methyl-2-benzothiazolyl)-2-[[3,4,6,7-tetrahydro-3-(2-methoxyphenyl)-4-oxothieno[3,2-d]pyrimidin-2-yl]thio]-acetamide), IWP-L6 (N-(5-phenyl-2-pyridinyl)-2-[(3,4,6,7-tetrahydro-4-oxo-3-phenylthieno[3,2-d]pyrimidin-2-yl)thio]-acetamide), IWP-12 (N-(6-Methyl-2-benzothiazolyl)-2-[(3,4,6,7-tetrahydro-3,6-dimethyl-4-oxothieno[3,2-d]pyrimidin-2-yl)thio]acetamide), LGK-974 (2-(2',3-Dimethyl-2,4'-bipyridin-5-yl)-N-(5-(pyrazin-2-yl)pyridin-2-yl)acetamide), Wnt-C59 (2-[4-(2-Methylpyridin-4-yl)phenyl]-N-[4-(pyridin-3-yl)phenyl]acetamide), ETC-159 (1,2,3,6-Tetrahydro-1,3-dimethyl-2,6-dioxo-N-(6-phenyl-3-pyridazinyl)-7H-purine-7-acetamide), and GNF-6231 (N-[5-(4-Acetyl-1-piperazinyl)-2-pyridinyl]-2'-fluoro-3-methyl[2,4'-bipyridine]-5-acetamide). These substances can be used singly or in combination.

The first Wnt signaling pathway inhibitory substance used in the present invention preferably contains at least one selected from the group consisting of PORCN inhibitors, KY02111, and KY03-I, and more preferably contains a PORCN inhibitor. In addition, it is preferable for the first Wnt signaling pathway inhibitory substance to contain a substance having an inhibitory activity on a non-canonical Wnt pathway of Wnt. The PORCN inhibitor used in the present invention preferably contains at least one selected from the group consisting of IWP-2, IWP-3, IWP-4, IWP-L6, IWP-12, LGK-974, Wnt-C59, ETC-159, and GNF-6231, more preferably contains IWP-2 or Wnt-C59, and further preferably contains IWP-2.

The concentration of the first Wnt signaling pathway inhibitory substance in medium can be suitably set in a range in which the above-mentioned effect is achievable. For example, when IWP-2, one of PORCN inhibitors, is used as the first Wnt signaling pathway inhibitory substance, the concentration thereof is typically about 10 nM to about 50 µM, preferably about 10 nM to about 30 µM, further preferably about 100 nM to about 10 µM, and most preferably about 2 µM from a viewpoint of efficiency in production of a cell cluster including an olfactory receptor neuron or a precursor cell thereof. When Wnt-C59, one of PORCN inhibitors, is used, the concentration thereof is typically about 10 nM to about 30 µM, preferably about 20 nM to about 10 µM, and more preferably about 500 nM. When KY02111 is used, the concentration thereof is typically about 10 nM to about 50 µM, preferably 10 nM to about 30 µM, more preferably about 100 nM to about 10 µM, and further preferably about 5 µM.

The time to add the first Wnt signaling pathway inhibitory substance is typically 48 hours or less, preferably 24 hours or less, more preferably 12 hours or less, after the start of suspension culture of pluripotent stem cells in step (1), and further preferably the same as the start of suspension culture.

It is preferable that a TGFβ signaling pathway inhibitory substance be further present in medium in step (1).

Those exemplified in step (a) can be used as a TGFβ signaling pathway inhibitory substance to be used step (1). The TGFβ signaling pathway inhibitory substance in step (a) and that in step (1) may be the same or different, but preferably are the same.

The concentration of the TGFβ signaling pathway inhibitory substance in medium can be suitably set in a range in which the above-mentioned effect is achievable. When SB431542 is used as a TGFβ pathway inhibitory substance, SB431542 is used typically in a concentration of about 1 nM to about 100 µM, preferably about 10 nM to about 100 µM, more preferably about 100 nM to about 50 µM, and further preferably about 500 nM to about 10 µM. When a TGFβ signaling pathway inhibitory substance other than SB431542 is used, it is desirable to use such an inhibitory substance in a concentration that provides a TGFβ signaling pathway inhibitory activity equivalent to SB431542 of the concentration described previously.

The medium used in step (1) is not particularly limited as long as the medium is that as described in the above section of definition. The medium used in step (1) can be serum medium or serum-free medium. From a viewpoint of avoiding chemically undetermined components from mixing in, serum-free medium is preferably used in the present invention. For avoidance of cumbersome preparations, for example, it is preferable to use serum-free medium to which a proper amount of a serum replacement such as commercially available KSR is added (for example, medium in which 5% KSR, 450 µM 1-monothioglycerol, and 1 x Chemically Defined Lipid Concentrate are added to a 1:1 mixed solution of IMDM and F-12, or medium in which 5% to 20% KSR, NEAA, pyruvic acid, and 2-melcaptoethanol are added to GMEM). The amount of KSR to be added to the serum-free medium, for example, in the case of human ES cells is typically about 1% to about 30%, and preferably about 2% to about 20%.

It is preferable that pluripotent stem cells have been dispersed into single cells at the start of step (1). To achieve this, it is preferable to carry out a procedure for dispersing pluripotent stem cells obtained in step (a) to single cells (also referred to as step (b)) before the start of step (1). Examples of "dispersed into single cells" include a state in which 70% or more of all cells are single cells and 30% or less of all cells are in clusters of 2 to 50 cells. Examples of cells dispersed into single cells include those in a state in which 80% or more of cells are preferably single cells and 20% or less of cells are in clusters of 2 to 50 cells. Cells dispersed into single cells are, for example, those in a state in which almost no intercellular adhesion (for example, plane attachment) is present. In some embodiments, cells dispersed into single cells are those in a state in which almost no cell-cell junction (for example, adherence junction) is present.

The procedure for dispersing pluripotent stem cells obtained in step (a) may include the above-mentioned mechanical dispersion treatment, cell-dispersing liquid treatment, or cell protectant addition treatment. These treatments may be used in combination. Preferably, the cell-dispersing liquid treatment is carried out simultaneously with the cell protectant addition treatment and subsequently the mechanical dispersion treatment is carried out.

Examples of the cell protectant used for the cell protectant addition treatment include FGF signaling pathway-activating substances, heparin, ROCK inhibitory substances, myosin inhibitory substances, serum, and serum replacements. Examples of the preferable cell protectant include ROCK inhibitory substances. To prevent cell death of pluripotent stem cells (in particular, human pluripotent stem cells) induced by dispersion, a ROCK inhibitory substance may be added at the start of culturing in step (1). Examples of the ROCK inhibitory substance include Y-27632 ((R)-(+)-trans-4-(1-Aminoethyl)-N-(4-pyridyl)cyclohexanecarboxamide dihydrochloride), Fasudil (HA1077) (1-(5-Isoquinolinylsulfonyl)homopiperazine hydrochloride), H-1152 (5-[[(2S)-hexahydro-2-methyl-1H-1,4-diazepin-1-yl]sulfonyl]-4-methyl-isoquinoline dihydrochloride), and HA-1100 (Hydroxyfasudil) ([1-(1-Hydroxy-5-isoquinolinesulfonyl) homopiperazine, hydrochloride). A ready-to-use cell protectant can be used for the cell protectant. Examples of the ready-to-use cell protectant include RevitaCell Supplement (manufactured by Thermo Fisher Scientific) and CloneR (manufactured by Stemcell Technologies Inc.). These substances can be used singly or in combination.

Examples of the cell-dispersing liquid used for the cell-dispersing liquid treatment include a solution containing at least one of enzymes such as trypsin, collagenase, hyaluronidase, elastase, Pronase, DNase, and papain and chelating agents such as ethylenediaminetetraacetic acid. A commercially available cell-dispersing liquid such as TripLE Select (manufactured by Thermo Fisher Scientific), TripLE Express (manufactured by Thermo Fisher Scientific), and Accumax (manufactured by Innovative Cell Technologies, Inc.) can also be used.

Examples of the mechanical dispersion treatment method include pipetting treatments and scraping procedures using a scraper. Dispersed cells are suspended in the above medium.

Then, the suspension of the dispersed pluripotent stem cells is seeded in the above culture apparatus, and the dispersed pluripotent stem cells are cultured under conditions non-adherent to the culture apparatus to group and aggregate a plurality of cells, thereby forming a cell aggregate.

At this time, the dispersed pluripotent stem cells may be seeded in a relatively large culture container such as a 10-cm dish to form a plurality of cell aggregates in one culture apparatus at the same time; however, it is preferable from a viewpoint of achievement of less size variation among aggregates to seed a certain number of dispersed pluripotent stem cells in each well of a multi-well plate (U-bottom, V-bottom) such a cell non-adherent 96-well microplate. If being subjected to static culture, the cells quickly aggregate and thus one cell aggregate can be formed in each well. Examples of the multi-well plate include a PrimeSurface 96-well V-bottom plate (MS-9096V, manufactured by Sumitomo Bakelite Co., Ltd.). Centrifugal procedures may be carried out to more quickly form a cell aggregate. By recovering an aggregate formed in each of a plurality of wells, a population of uniform aggregates can be obtained.

It is also preferable to use for the culture container a three-dimensional cell culture container that allows exchange of media in the entire plate at a time with a cell aggregate or cell cluster remaining in each well. Examples of such a three-dimensional cell culture container include a PrimeSurface 96 slit-well plate (manufactured by Sumitomo Bakelite Co., Ltd.). This plate is provided with a narrow opening (slit) that allows medium to enter and exit in the upper part of each of the 96 wells. Since the slit is designed to have a width difficult for cell aggregates or cell clusters to pass through, media in the entire plate can be exchanged at a time while the adhesion of cell aggregates or cell clusters is prevented, and hence the efficiency in the procedure and the quality of cell clusters can be improved.

The concentration of the pluripotent stem cells in step (1) can be suitably set so as to uniformly and efficiently form cell aggregates. When human pluripotent stem cells (for example, human iPS cells obtained in step (a)) are subjected to suspension culture with a 96-well microwell plate, a solution prepared to attain typically about 1 × 10³ to about 1 × 10⁵ cells, preferably about 3 × 10³ to about 5 × 10⁴ cells, more preferably about 4 × 10³ to about 2 × 10⁴ cells, further preferably about 4 × 10³ to about 1.6 × 10⁴ cells, particularly preferably about 8 × 10³ to about 1.2 × 10⁴ cells, per well is added to each well, and the plate is left to stand to form aggregates. The number of cells can be determined by counting with a hemocytometer.

Examples of medium exchange procedures if being carried out in any of step (1) and the subsequent steps include a procedure for adding a fresh medium without discarding the existing medium (medium addition procedure), a procedure for discarding about half the amount of the existing medium (about 30 to 90%, for example, about 40 to 60% of the volume of the existing medium) and adding a fresh medium in about half the amount of the existing medium (about 30 to 90%, for example, about 40 to 60% of the volume of the existing medium) (half medium exchange procedure), and a procedure for discarding about the whole amount of the existing medium (90% or more of the volume of the existing medium) and adding a fresh medium in about the whole amount of the existing medium (90% or more of the volume of the existing medium) (complete medium exchange procedure).

If a specific component is added at a certain time point, for example, a final concentration thereof is calculated in advance, and a procedure for discarding about half the amount of the existing medium and adding a fresh medium containing the specific component in a concentration higher than the final concentration in about half the amount of the existing medium (half medium exchange procedure) may be carried out.

If a component contained in the existing medium is diluted to lower the concentration at a certain time point, for example, a medium exchange procedure may be carried out multiple times per day, preferably multiple times within 1 hour (for example, twice or three times). If a component contained in the existing medium is diluted to lower the concentration at a certain time point, cells or aggregates may be transferred into another culture container.

Examples of the tool used for medium exchange procedures include, but are not particularly limited to, a pipetter, a Pipetman (R), a multichannel pipette, and a continuous dispenser. When a 96-well plate is used as a culture container, for example, a multichannel pipette may be used.

The duration of suspension culture required to form cell aggregates can be suitably determined according to pluripotent stem cells used, but it is desirable for formation of uniform cell aggregates that the duration be as short as possible. Steps until cell aggregates are formed from dispersed cells are divided into a step in which cells group and a step in which cells that have grouped form aggregates. From the time point of seeding dispersed cells (that is, at the start of suspension culture) until cells group, in the case of human pluripotent stem cells (such as human iPS cells), for example, cells that have grouped are formed preferably within about 24 hours, more preferably within about 12 hours. In the step from the time point of seeding dispersed cells (that is, at the start of suspension culture) until cell aggregates are formed, in the case of human pluripotent stem cells (such as human iPS cells), for example, aggregates are formed preferably within about 72 hours, more preferably within about 48 hours. The duration until the formation of aggregates can be appropriately controlled with an instrument to aggregate cells or by adjusting, for example, centrifugation conditions.

The formation of aggregates can be confirmed based on the size and cell numbers, macroscopic form, microscopic form and homogeneity thereof by histostaining analyses of aggregates, expression of differentiation and undifferentiation markers and homogeneity thereof, expression control of differentiation markers and synchronicity thereof, reproducibility of differentiation efficiency between aggregates and the like.

After cell aggregates are formed, culture of the aggregates may be directly continued. The duration of suspension culture in step (1) is typically about 8 hours to 6 days, and preferably about 12 hours to 48 hours.

From a viewpoint of promotion of the survival and proliferation of neural cells or a tissue in the inside of a cell cluster, any one or more steps of steps (1) to (3) can be also carried out in the presence of a Wnt signaling pathway-activating substance. As described above, examples of Wnt signaling pathways include the canonical Wnt pathway and non-canonical Wnt pathways. Examples of non-canonical Wnt pathways include the Planar Cell Polarity (PCP) pathway, Wnt/Calcium pathway, Wnt-RAP1 pathway, Wnt-Ror2 pathway, Wnt-PKA pathway, Wnt-GSK3MT pathway, Wnt-aPKC pathway, Wnt-RYK pathway, and Wnt-mTOR pathway.

The Wnt signaling pathway-activating substance is not limited as long as the substance is capable of activating signaling evoked by Wnt family proteins. The Wnt signaling pathway-activating substance can be nucleic acids, proteins or low molecular organic compounds. Examples of such a substance include substances that promote autocrine secretion of Wnt, substances that stabilize Wnt to suppress decomposition thereof, recombinant proteins of Wnt, partial sequence peptides of Wnt and derivates and derivatives thereof, substances that act on a Wnt receptor to activate it, substances that activate the intracellular signaling mechanism of Wnt, intracellular signaling factors for Wnt and variants thereof (such as β-Catenin S33Y), and substances that activate gene expression downstream of a Wnt responsive element. Examples of proteins known as Wnt signaling pathway-activating substances include Wnt and R-Spondin.

Compounds well-known as Wnt signaling pathway-activating substances to those skilled in the art can also be used. Examples of compounds having activity as a Wnt signaling pathway-activating substance include lithium chloride, AMBMP hydrochloride, SGC AAK1 1, Foxy 5, CHIR99021, CHIR98014, TWS119, SB216763, SB415286, BIO, AZD2858, AZD1080, AR-A014418, TDZD-8, LY2090314, IM-12, Indirubin, Bikinin, A 1070722, 3F8, Kenpaullone, 10Z-Hymenialdisine, Indirubin-3'-oxime, NSC 693868, TC-G 24, TCS 2002, TCS 21311, CP21R7, BML-284, SKL2001, WAY 262611, IIIC3a, Methyl Vanillate, IQ-1, and derivatives of these compounds.

While the first Wnt signaling pathway inhibitory substance is added in step (1), use of a Wnt signaling pathway-activating substance and a Wnt signaling pathway inhibitory substance with different active sites in combination can activate or inhibit only a specific pathway of the above-mentioned multiple Wnt signaling pathways. The Wnt signaling pathway-activating substance is preferably a substance that acts on a factor downstream of the first Wnt signaling pathway inhibitory substance added to step (1). It is also preferable that the Wnt signaling pathway-activating substance be a substance that activates the canonical Wnt pathway, and it is further preferable that the Wnt signaling pathway-activating substance be a substance that activates the Wnt signaling pathway through the inhibiting and stabilizing action on the decomposition of β catenin, an intracellular Wnt signaling factor. Examples of the substance that exhibits inhibiting and stabilizing action on the decomposition of β catenin include a GSK3 inhibitor, BML-284, and SKL2001. It is also preferable that the Wnt signaling pathway-activating substance added be a substance that promotes or activates β catenin responsive transcription (CRT).

Examples of the GSK3 inhibitor include CHIR99021, CHIR98014, TWS119, SB216763, SB415286, BIO, AZD2858, AZD1080, AR-A014418, TDZD-8, LY2090314, IM-12, Indirubin, Bikinin, A 1070722, 3F8, Kenpaullone, 10Z-Hymenialdisine, Indirubin-3'-oxime, NSC 693868, TC-G 24, TCS 2002, TCS 21311, CP21R7, and derivatives of these compounds.

An example of preferred combinations of the Wnt signaling pathway-activating substance and the first Wnt signaling pathway inhibitory substance is a GSK3 inhibitor and a PORCN inhibitor. Use of a GSK3 inhibitor and a PORCN inhibitor in combination activates the canonical Wnt pathway and inhibits non-canonical Wnt pathways. When a GSK3 inhibitor and a PORCN inhibitor are used in combination, the GSK3 inhibitor preferably contains at least one selected from the group consisting of CHIR99021, CHIR98014, SB216763, SB415286, and BIO, and further preferably contains CHIR99021. When a GSK3 inhibitor and a PORCN inhibitor are used in combination, the PORCN inhibitor preferably contains at least one selected from the group consisting of IWP-2, IWP-3, IWP-4, IWP-L6, IWP-12, LGK-974, ETC-159, GNF-6231, and Wnt-C59, and further preferably contains IWP-2. Alternatively, a GSK3 inhibitor and KY02111 or a derivative or the like thereof may be used in combination. Examples of substances that exhibit inhibiting and stabilizing action on the decomposition of β catenin through an action mechanism differing from that of GSK3 inhibitors include BML-284 and SKL2001, and these compounds and derivatives thereof may be used in combination with a PORCN inhibitor, KY02111, or a derivative or the like thereof.

The concentration of the Wnt signaling pathway-activating substance in medium can be suitably set in a range in which the above-mentioned effect is achievable. From a viewpoint of promotion of the survival and proliferation of neural cells or a tissue in the inside of a cell cluster, when CHIR99021 is used as a Wnt signaling pathway-activating substance, CHIR99021 is used in a concentration of typically about 10 pM to about 10 mM, preferably about 100 pM to about 1 mM, more preferably about 1 nM to about 100 µM, further preferably about 10 nM to about 30 µM, and most preferably about 100 nM to about 3 µM. When a Wnt signaling pathway-activating substance other than CHIR99021 is used, it is desirable to use such a substance in a concentration that provides a Wnt signaling pathway-promoting activity equivalent to CHIR99021 of the above concentration.

### <Step (2)>

Step (2) of suspension culturing the cell aggregate obtained in step (1) in the presence of a BMP signaling pathway-activating substance will be described.

The BMP signaling pathway-activating substance is a substance capable of enhancing a signaling pathway mediated by BMP (bone morphogenetic protein). Examples of the BMP signaling pathway-activating substance include BMP proteins such as BMP2, BMP4, and BMP7, GDF proteins such as GDF7, an anti-BMP receptor antibody, and BMP partial peptides. These substances can be used singly or in combination. Examples of the BMP signaling pathway-activating substance according to a definition from the standpoint of biological activity include substances that have ability to induce differentiation into osteoblast-like cells for cells such as the mouse precursor chondrocyte cell line ATDC5, the mouse calvaria-derived cell line MC3T3-E1, and the mouse striated muscle-derived cell line C2C12, and ability to induce production of alkaline phosphatase. Examples of substances having such abilities include BMP-2, BMP-4, BMP-5, BMP-6, BMP-7, BMP-9, BMP-10, BMP-13/GDF-6, BMP-14/GDF-5, and GDF-7.

BMP2 protein and BMP4 protein are available, for example, from R&D Systems, Inc., BMP7 protein is available, for example, from BioLegend, and GDF7 protein is available, for example, from FUJIFILM Wako Pure Chemical Corporation. The BMP signaling pathway-activating substance preferably contains at least one protein selected from the group consisting of BMP2, BMP4, BMP7, BMP13, and GDF7, and more preferably contains BMP4.

The concentration of the BMP signaling pathway-activating substance in medium can be suitably set in a range in which the above-mentioned effect is achievable. From a viewpoint of efficiency in production of a cell cluster including an olfactory receptor neuron or a precursor cell thereof, when BMP4 is used as a BMP signaling pathway-activating substance, BMP4 is used in a concentration of typically about 1 pM to about 100 nM, preferably about 10 pM to about 50 nM, more preferably about 25 pM to about 25nM, further preferably about 25 pM to about 5 nM, particularly preferably about 100 pM to about 5 nM, and most preferably about 500 pM to about 2 nM. When a BMP signaling pathway-activating substance other than BMP4 is used, it is desirable to use such a substance in a concentration that provides a BMP signaling pathway-promoting activity equivalent to BMP4 of the above-mentioned concentration. When a commercially available recombinant BMP protein is used as a BMP signaling pathway-activating substance, those skilled in the art could determine the concentration of the BMP signaling pathway-activating substance to be added easily by comparing the activity described in a document attached to the product, for example, the ED₅₀ value for the ability to induce production of alkaline phosphatase against the mouse precursor chondrocyte cell line ATDC5, with the activity at the above-mentioned concentration of BMP4.

The medium used in step (2) is not particularly limited as long as the medium contains the BMP signaling pathway-activating substance. The medium used in step (2) can be serum medium or serum-free medium. From a viewpoint of avoiding chemically undetermined components from mixing in, serum-free medium is preferably used. For avoidance of cumbersome preparations, for example, it is preferable to use serum-free medium to which a proper amount of a serum replacement such as commercially available KSR is added (for example, medium in which 5% KSR, 450 µM 1-monothioglycerol, and 1 x Chemically Defined Lipid Concentrate are added to a 1:1 mixed solution of IMDM and F-12, or medium in which 5% to 20% KSR, NEAA, pyruvic acid, and 2-melcaptoethanol are added to GMEM). The amount of KSR to be added to the serum-free medium, for example, in the case of human ES cells is typically about 1% to about 30%, and preferably about 2% to about 20%.

From a viewpoint of efficiency in production of a cell cluster including an olfactory receptor neuron or a precursor cell thereof, the start time of step (2) is preferably 0.5 hours or more and 6 days or less, more preferably 0.5 hours or more and 72 hours or less, and further preferably 24 hours or more and 60 hours or less, after the start of suspension culture in step (1).

From a viewpoint of efficiency in production of a cell cluster including an olfactory receptor neuron or a precursor cell thereof, the start time of step (2) is a time at which preferably 10% or more and 100% or less, more preferably 30% or more and 100% or less, and further preferably 50% or more and 100% or less of cells on the surface layer of an aggregate formed in step (1) are forming a tight junction with each other.

At the start of culture in the presence of the BMP signaling pathway-activating substance in step (2), any of the above-mentioned medium exchange procedures (for example, a medium addition procedure, a half medium exchange procedure, a complete medium exchange procedure) may be carried out with use of the culture container with which step (1) was carried out, and the cell aggregates may be transferred into another culture container.

The period of suspension culture in medium containing the BMP signaling pathway-activating substance in step (2) can be suitably set. The duration of suspension culture in step (2) is typically about 8 hours to 6 days, preferably about 10 hours to 96 hours, more preferably about 12 hours to 72 hours, further preferably about 14 hours to 48 hours, and most preferably about 16 hours to 36 hours.

It is also preferable to add the additive used in step (a) or step (1), such as the Shh signaling pathway-activating substance, first Wnt signaling pathway inhibitory substance, Wnt signaling pathway-activating substance, and TGFβ signaling pathway inhibitory substance, again in step (2). The Shh signaling pathway-activating substance, first Wnt signaling pathway inhibitory substance, Wnt signaling pathway-activating substance, or TGFβ signaling pathway inhibitory substance to be added in step (2) may be the same as or different from the substance used in any of the preceding steps, but they are preferably the same. The concentration and type of the additive can be suitably adjusted. The time to add the substance may be the same as or different from the start of step (2).

### <Step (3)>

Step (3) of obtaining a cell cluster including an olfactory receptor neuron or a precursor cell thereof will be described, wherein step (3) includes a step of suspension culturing the cell aggregate obtained in step (2). Step (3) includes at least one step selected from the group consisting of: step (3a) of culturing in the presence of an FGF signaling pathway-activating substance; step (3b) of culturing in the presence of a BMP signaling pathway inhibitory substance; and step (3c) of culturing in the presence of an FGF signaling pathway-activating substance and a BMP signaling pathway inhibitory substance.

### <Step (3a)>

Step (3a) of suspension culturing the cell aggregate obtained in step (2) in the presence of an FGF signaling pathway-activating substance will be described. Step (3a) is carried out in the absence of an exogenous BMP signaling pathway inhibitory substance. If this step is carried out further in the presence of an exogenous BMP signaling pathway inhibitory substance, the case will be described later as step (3c). Here, "exogeneous" means being due to artificial addition.

The FGF signaling pathway-activating substance is not particularly limited as long as a substance is capable of enhancing a signaling pathway mediated by FGF (fibroblast growth factor). Examples of the FGF signaling pathway-activating substance include FGF proteins such as FGF1, FGF2 (also referred to as bFGF), and FGF8, an anti-FGF receptor antibody, and FGF partial peptides. These substances can be used singly or in combination.

FGF2 protein and FGF8 protein are available, for example, from FUJIFILM Wako Pure Chemical Corporation. The FGF signaling pathway-activating substance preferably contains at least one selected from the group consisting of FGF2 and FGF8 and variants thereof, more preferably contains FGF2, and further preferably contains recombinant human FGF2.

The concentration of the FGF signaling pathway-activating substance in medium can be suitably set in a range in which the above-mentioned effect is achievable. From viewpoints of formation of the epithelial structure of a nervous tissue part, differentiation into placodes, and promotion of the survival and proliferation of cells, when FGF2 is used as an FGF signaling pathway-activating substance, FGF2 is used in a concentration of typically about 1 pg/ml to about 100 µg/ml, preferably about 10 pg/ml to about 50 µg/ml, more preferably about 100 pg/ml to about 10 µg/ml, further preferably about 500 pg/ml to about 1 µg/ml, and most preferably about 1 ng/ml to about 200 ng/ml. When an FGF signaling pathway-activating substance other than FGF2 is used, it is desirable to use such a substance in a concentration that provides an FGF signaling pathway-promoting activity equivalent to FGF2 of the above concentration.

It is also preferable to add heparin or heparan sulfate to the medium containing FGF protein for the purpose of retaining the activity of the FGF protein in the medium. Heparin is available, for example, as its sodium salt from FUJIFILM Wako Pure Chemical Corporation. The concentration of heparin or heparan sulfate in medium can be suitably set in a range in which the above-mentioned effect is achievable. The concentration of heparin sodium in medium is typically about 1 ng/ml to about 100 mg/ml, preferably about 10 ng/ml to about 50 mg/ml, more preferably about 100 ng/ml to about 10 mg/ml, further preferably about 500 ng/ml to about 1 mg/ml, and most preferably about 1 µg/ml to about 200 µg/ml. When heparan sulfate is used, it is preferable that heparan sulfate have a concentration that provides an activity to protect FGF protein equivalent to heparin of the above concentration. It is also preferable to use an FGF variant such as Thermostable FGF2 described in U.S. Patent No. US8772460B2 or FGF2 controlled-release beads such as StemBeads FGF2 with FGF2 bound to biodegradable polymer for the purpose of retaining the activity of FGF protein in a cell culture environment, for example, at 37°C. Thermostable FGF2 is available, for example, from HumanZyme, Inc. StemBeads FGF2 is available, for example, from StemCulture, LLC.

### <Step (3b)>

Step (3b) of suspension culturing the cell aggregate obtained in step (2) in the presence of a BMP signaling pathway inhibitory substance will be described. Step (3b) is carried out in the absence of an exogenous FGF signaling pathway-activating substance. If the step is carried out further in the presence of an exogenous FGF signaling pathway-activating substance, the case will be described later as step (3c).

The BMP signaling pathway inhibitory substance is not limited as long as the inhibitory substance can suppress signaling evoked by BMP family proteins. The BMP signaling pathway inhibitory substance can be nucleic acids, proteins or low molecular organic compounds. Examples of such a substance can include substances that inhibit processing of BMP and extracellular secretion thereof, substances that directly act on BMP (for example, proteins, antibodies, aptamers), substances that suppress expression of a gene encoding BMP (for example, antisense oligonucleotides, siRNA), substances that inhibit binding between a BMP receptor and BMP, and substances that inhibit physiological activity due to signaling by a BMP receptor. There exist type I BMP receptors and type II BMP receptors, and BMPR1A, BMPR1B, and ACVR are known as type I BMP receptors, and TGF-beta R-II, ActR-II, ActR-IIB, BMPR2, and MISR-II are known as type II BMP receptors.

Examples of proteins known as BMP signaling pathway inhibitory substances include secretory proteins belonging to the Noggin, Chordin, Follistatin, Gremlin, Inhibin, Twisted Gastrulation, Coco, and DAN families. Because a BMP signaling pathway-activating substance has been added to culture medium in step (2) above, from a viewpoint of effectively inhibiting the subsequent BMP signaling pathway, the BMP signaling pathway inhibitory substance in step (3b) preferably contains a substance that inhibits the signaling pathway subsequent after extracellular secretion of BMP such as substances that inhibit binding between a BMP receptor and BMP and substances that inhibit physiological activity due to signaling by a BMP receptor, and more preferably contains a type I BMP receptor inhibitor.

Compounds well-known as BMP signaling pathway inhibitory substances to those skilled in the art can also be used. Examples of BMP signaling pathway inhibitory substances include K02288 (3-[(6-Amino-5-(3,4,5-trimethoxyphenyl)-3-pyridinyl]phenol,3-[6-Amino-5-(3,4,5-trimethoxyphenyl)-3-pyridinyl]-phenol), Dorsomorphin (6-[4-[2-(1-Piperidinyl)ethoxy]phenyl]-3-(4-pyridinyl)pyrazolo[1,5-a]pyrimidine), LDN-193189 (4-[6-[4-(1-Piperazinyl)phenyl]pyrazolo[1,5-a]pyrimidin-3-yl]quinoline dihydrochloride), LDN-212854 (5-[6-[4-(1-Piperazinyl)phenyl]pyrazolo[1,5-a]pyrimidin-3-yl]quinoline), LDN-214117 (1-(4-(6-methyl-5-(3,4,5-trimethoxyphenyl)pyridin-3-yl)phenyl)piperazine), ML347 (5-[6-(4-Methoxyphenyl)pyrazolo[1,5-a]pyrimidin-3-yl]quinoline)), DMH1 (4-(6-(4-Isopropoxyphenyl)pyrazolo[1,5-a]pyrimidin-3-yl)quinoline), and DMH2 (4-[6-[4-[2-(4-Morpholinyl)ethoxy]phenyl]pyrazolo[1,5-a]pyrimidin-3-yl]-quinoline). These substances can be used singly or in combination.

The BMP signaling pathway inhibitory substance used in the present invention is preferably a type I BMP receptor inhibitor, more preferably contains at least one selected from the group consisting of K02288, Dorsomorphin, LDN-193189, LDN-212854, LDN-214117, ML347, DMH1, and DMH2, and further preferably contains K02288.

The concentration of the BMP signaling pathway inhibitory substance in medium can be suitably set in a range in which the above-mentioned effect is achievable. From a viewpoint of the efficiency in formation of an olfactory epithelial-like tissue, when K02288 is used as a BMP signaling pathway inhibitory substance in step (3b), K02288 is used in a concentration of typically about 1 nM to about 100 µM, preferably about 10 nM to about 50 µM, more preferably about 100 nM to about 50 µM, and further preferably about 500 nM to about 25 µM. When LDN-193189 is used as a BMP pathway inhibitory substance, LDN-193189 is used in a concentration of typically about 1 nM to about 100 µM, preferably about 10 nM to about 10 µM, more preferably about 25 nM to about 1 µM, and further preferably about 100 nM to about 500 nM. When LDN-212854 is used as a BMP pathway inhibitory substance, LDN-212854 is used in a concentration of typically about 1 nM to about 100 µM, preferably about 10 nM to about 10 µM, more preferably about 25nM to about 5 µM, and further preferably about 250 nM to about 3 µM. When ML-347 is used as a BMP pathway inhibitory substance, ML-347 is used in a concentration of typically about 1 nM to about 100 µM, preferably about 10 nM to about 50 µM, more preferably about 100 nM to about 50 µM, and further preferably about 1 µM to about 25 µM. When DMH2 is used as a BMP pathway inhibitory substance, DMH2 is used in a concentration of typically about 1 nM to about 100 µM, preferably about 10 nM to about 10 µM, more preferably about 25nM to about 5 µM, and further preferably about 250 nM to about 3 µM. When a BMP signaling pathway inhibitory substance other than K02288 is used, it is desirable to use such an inhibitory substance in a concentration that provides a BMP signaling pathway inhibitory activity equivalent to K02288 of the above concentration.

### <Step (3c)>

Step (3c) of suspension culturing the cell aggregate obtained in step (2) in the presence of an FGF signaling pathway-activating substance and a BMP signaling pathway inhibitory substance will be described.

For the type, concentration, and so on of the FGF signaling pathway-activating substance and BMP signaling pathway inhibitory substance used in step (3c), conditions the same as or different from those in step (3a) or (3b) can be used. When FGF2 is used as an FGF signaling pathway-activating substance in step (3c), FGF2 is used in a concentration of typically about 1 pg/ml to about 100 µg/ml, preferably about 10 pg/ml to about 50 µg/ml, more preferably about 100 pg/ml to about 10 µg/ml, further preferably about 500 pg/ml to about 1 µg/ml, and most preferably about 1 ng/ml to about 200 ng/ml. When K02288 is used as a BMP pathway inhibitory substance in step (3c), K02288 is used in a concentration of typically about 1 nM to about 100 µM, preferably about 10 nM to about 50 µM, more preferably about 50 nM to about 50 µM, and further preferably about 100 nM to about 25 µM.

Step (3c) may be carried out after step (3a) or step (3b), and only step (3c) may be carried out. In the case that formation of a retinal tissue in a cell cluster is not intended, it is preferable to carry out step (3c) with steps (3a) and (3b) skipped. In the case that formation of a retinal tissue in a cell cluster is intended, it is preferable to carry out step (3a) first and then carry out step (3c). In the case that step (3a) is carried out and then step (3c) is carried out, the culture period of step (3a) is typically 1 day to 40 days, preferably 2 days to 30 days, more preferably 3 days to 25 days, and further preferably 6 days to 25 days. For the type, concentration, and so on of the FGF signaling pathway-activating substance used at the culture, conditions the same as or different from those in the case that only step (3a) is carried out may be used.

In the case that formation of a retinal tissue in a cell cluster is intended and step (3a) is carried out and step (3c) is then carried out, it is preferable with respect to the culture period of step (3a) that step (3a) be carried out until a retinal tissue is formed in a cell cluster. Examination on whether a retinal tissue was formed in a cell cluster can be made, for example, by means of observation with a microscope or the like on whether an epithelial-like tissue was formed, or a method in which a section of a cell cluster is prepared and immunofluorescence staining is carried out for a marker that is expressed in retinal tissues such as Rx, Chx10, and N-Cadherin.

From a viewpoint of the efficiency in formation of an olfactory epithelial-like tissue, the start time of step (3a), step (3b), or step (3c) is preferably 0.5 hours or more and 96 or less, more preferably 12 hours or more and 72 hours or less, and further preferably 12 hours or more and 48 hours or less, after addition of the BMP signaling pathway-activating substance in step (2).

With respect to the start time of step (3a), step (3b), or step (3c), it is preferable from a viewpoint of the efficiency in formation of an olfactory epithelial-like tissue to start before a non-neural epithelial tissue is formed on the surface of a cell cluster after addition of the BMP signaling pathway-activating substance in step (2). Examination on whether a non-neural epithelial tissue was formed on the surface of a cell cluster can be made, for example, by means of observation with a microscope or the like on whether an epithelial-like tissue was formed on the surface of a cell cluster, or a method in which a section of a cell cluster is prepared and immunofluorescence staining is carried out for a marker that is expressed in non-neural epithelial tissues such as cytokeratin, E-Cadherin, and EpCAM.

At the start of culture in step (3a), step (3b), or step (3c), any of the above-mentioned medium exchange procedure (for example, a medium addition procedure, a half medium exchange procedure, a complete medium exchange procedure) may be carried out with use of the culture container with which step (2) was carried out, and the cell aggregates may be transferred into another culture container.

The period of suspension culture in step (3a), step (3b), or step (3c) can be suitably set. The duration of suspension culture in step (3a), step (3b), or step (3c) is typically about 8 hours to 200 days, preferably about 10 hours to 180 days, more preferably about 12 hours to 150 days, further preferably about 14 hours to 120 days, and most preferably 20 hours to 100 days. In the case that step (3d) described in the following is carried out after step (3b) or step (3c), the duration of suspension culture in step (3b) or step (3c) is typically about 8 hours to 12 days, and preferably about 10 hours to 6 days.

### <Step (3d)>

Step (3) may further include step (3d) of suspension culturing in the absence of a BMP signaling pathway inhibitory substance after step (3b) or step (3c). That is, step (3d) of suspension culturing the cell aggregate obtained in step (3b) or step (3c) in the absence of a BMP signaling pathway inhibitory substance to obtain a cell cluster will be described. The cell cluster obtained in step (3b) or step (3c) may be further subjected to suspension culture in the absence of a BMP signaling pathway inhibitory substance.

In order to carry out suspension culture in the absence of a BMP signaling pathway inhibitory substance in step (3d), the BMP signaling pathway inhibitory substance added in step (3b) or step (3c) is needed to be removed from the culture environment. Examples of procedures therefor include a half medium exchange procedure and a complete medium exchange procedure. The medium exchange procedure may be carried out multiple times per day, and preferably multiple times within 1 hour (for example, twice or three times). From a viewpoint of cost reduction for production of a cell cluster, medium or the like containing no additive such as physiological saline, KSR, and a growth factor may be used, except for medium for culturing after step (3d) at the final procedure in carry outing multiple medium exchange procedures.

Examples of other procedures for suspension culture in the absence of a BMP signaling pathway inhibitory substance include procedures in which the cell aggregate is recovered into another sterilized container (such as a 15-ml tube and a petri dish for suspension culture), washed with medium, physiological saline, or the like, and transferred into a culture environment in the absence of a BMP signaling pathway inhibitory substance (a procedure for transferring the cell aggregate). The culture container used for culturing after the transfer may be the same container (such as a 96-well plate) as used until step (3b) or step (3c), or a different container (such as a dish for suspension culture).

In carry outing suspension culture in the absence of a BMP signaling pathway inhibitory substance in step (3d), it is also preferable from the viewpoint of avoiding cumbersome media exchange procedures or procedures for transferring cell clusters to use a three-dimensional cell culture container that allows exchange of media in the entire plate at a time with a cell aggregate or cell cluster remaining in each well.

By suspension culturing the cell aggregate obtained in step (3b) or step (3c) in the absence of a BMP signaling pathway inhibitory substance in step (3d), a cell cluster that is less likely to undergo cell death with more promoted cell proliferation, improved efficiency in formation of an olfactory epithelial-like tissue, and improved efficiency in formation of the epithelial structure of a nervous tissue part can be obtained.

Preferably, an FGF signaling pathway-activating substance is present in step (3d). The FGF signaling pathway-activating substance added in step (3d) may be the same as or different from the substance used in step (3a) or (3c), but is preferably the same. The concentration and type of the FGF signaling pathway-activating substance can be suitably adjusted.

From viewpoints of formation of the epithelial structure of a nervous tissue and promotion of proliferation, the start time of step (3d) is preferably 0.5 hours or more and 60 days or less, more preferably 0.5 hours or more and 30 days or less, and further preferably 12 hours or more and 96 hours or less after addition of the BMP signaling pathway inhibitory substance in step (3b) or (3c).

The period of suspension culture in the absence of a BMP signaling pathway inhibitory substance in step (3d) can be suitably set. The duration of suspension culture in step (3d) is typically about 8 hours to 200 days, preferably about 10 hours to 180 days, more preferably about 12 hours to 150 days, further preferably about 14 hours to 120 days, and most preferably about 20 days to 100 days.

The medium used in step (3) is not particularly limited, and can be serum medium or serum-free medium. From a viewpoint of avoiding chemically undetermined components from mixing in, serum-free medium is preferably used. For avoidance of cumbersome preparations, for example, it is preferable to use serum-free medium to which a proper amount of a serum replacement such as commercially available KSR is added. In the case of human ES cells, for example, the amount of KSR added to the serum-free medium is typically about 1% to about 30%, and preferably about 2% to about 20%. Examples of the medium used in step (3) include medium in which 5% KSR, 450 µM 1-monothioglycerol, and 1 x Chemically Defined Lipid Concentrate are added to a 1:1 mixed solution of IMDM and F-12, medium in which 5% to 20% KSR, NEAA, pyruvic acid, and 2-melcaptoethanol are added to GMEM, medium in which B27 Supplement is added to Neurobasal, medium in which N2 Supplement is added to DMEM/F-12, and ready-to-use neuron culture medium such as StemPro NSC SFM serum-free human neural stem cell culture medium (manufactured by Thermo Fisher Scientific).

It is also preferable to add the additive used in any of step (a), step (1), and step (2) for production of a cell cluster, such as the Shh signaling pathway-activating substance, first Wnt signaling pathway inhibitory substance, Wnt signaling pathway-activating substance, and TGFβ signaling pathway inhibitory substance, again in step (3). The Shh signaling pathway-activating substance, first Wnt signaling pathway inhibitory substance, Wnt signaling pathway-activating substance, and TGFβ signaling pathway inhibitory substance added in step (3) may be the same as or different from the substances used in step (a), step (1), and step (2), but preferably are the same. The concentration and type of the additive can be suitably adjusted. The additive used in step (3) more preferably contains the first Wnt signaling pathway inhibitory substance, and further preferably contains the first Wnt signaling pathway inhibitory substance, Wnt signaling pathway-activating substance, and TGFβ signaling pathway inhibitory substance.

From viewpoints of formation of the epithelial structure of a nervous tissue part and promotion of the survival and proliferation of cells, it is also preferable to carry out step (3) in the presence of an EGF signaling pathway-activating substance. The EGF signaling pathway-activating substance is a substance capable of enhancing a signaling pathway mediated by an epidermal growth factor (EGF). Examples of the EGF signaling pathway-activating substance include EGFR ligand proteins such as EGF, TGF-α, AR (amphiregulin), EPG, HB-EGF (heparin-binding EGF-like growth factor), BTC (betacellulin), and EPR (epiregulin), ErbB3/4 (epidermal growth factor receptor, HRE) ligand proteins of NRG (neuregulin)-1 to -4, and low molecular compounds such as alprenol and carvedilol. These substances can be used singly or in combination. EGF protein is available, for example, from Thermo Fisher Scientific and PrimeGene. HB-EGF protein is available, for example, from R&D Systems, Inc. The EGF signaling pathway-activating substance preferably contains EGF.

The concentration of the EGF signaling pathway-activating substance in medium can be suitably set in a range in which the above-mentioned effect is achievable. When EGF is used as an EGF signaling pathway-activating substance, EGF is used in a concentration of typically about 1 pg/ml to about 100 µg/ml, preferably about 10 pg/ml to about 50 µg/ml, more preferably about 100 pg/ml to about 10 µg/ml, further preferably about 500 pg/ml to about 1 µg/ml, and most preferably about 1 ng/ml to about 200 ng/ml. When an EGF signaling pathway-activating substance other than EGF is used, it is desirable to use such a substance in a concentration that provides an EGF signaling pathway-promoting activity equivalent to EGF of the above concentration.

The above substances may be present in at least one step selected from the group consisting of steps (3a) to (3d) in step (3), or in all the steps of steps (3a) to (3d). The time to add the above substances may be the same as or different from the start of step (3a), step (3b), step (3c), or step (3d).

In step (3), a more mature cell cluster including highly differentiated cells can be obtained by long-term culture of the produced cell cluster. Such culture is also referred to as maturation culture. From a viewpoint of avoiding chemically undetermined components from mixing in, serum-free medium is preferably used in the process of maturation culture in step (3). The same medium as used in step (1) to step (3) can be used as medium in maturation culture in step (3). For example, known medium for culture of neurons or the like such as medium in which 0.5% N2 Supplement and 1% B27 supplement are added to a 1:1 mixed solution of DMEM/F-12 medium and Neurobasal medium (N2B27 medium) and medium in which StemPro NSC SFM Supplement is added to Knockout DMEM/F-12 medium (StemPro NSC SFM medium) can also be used. In carrying out maturation culture, culture can also be continued with the culture container used at the start of suspension culture. Culture can be carried out after transferring the cell cluster into a new culture container such as a dish for suspension culture and a flask for suspension culture. In particular, the case that the culture conditions are largely changed in the process of maturation culture will be described later as step (3e).

### <Step (3e)>

Step (3) may further include step (3e) of further culturing after step (3a), step (3b), or step (3c). Step (3) may include step (3e) after step (3d). Through step (3e), olfactory receptor neurons in a more advanced stage of differentiation can be obtained.

The same medium as used in step (1) to step (3), or in the maturation culture in step (3) can be used as medium in step (3e). For example, known medium for culture of neurons or the like such as medium in which 0.5% N2 Supplement and 1% B27 supplement are added to a 1:1 mixed solution of DMEM/F-12 medium and Neurobasal medium (N2B27 medium), medium in which StemPro NSC SFM Supplement is added to Knockout DMEM/F-12 medium (StemPro NSC SFM medium), medium for central nervous system organoids (STEMdiff Cerebral Organoid Maturation Kit), and medium for culture of epithelial cells (PneumoCult ALI medium, CnT-Prime, Epithelial Culture Medium) can also be used.

It is also preferable to add the additive used for production of a cell cluster in any of the above steps, such as the BMP signaling pathway inhibitory substance, TGFβ signaling pathway inhibitory substance, Shh signaling pathway-activating substance, first Wnt signaling pathway inhibitory substance, Wnt signaling pathway-activating substance, FGF signaling pathway-activating substance, and EGF signaling pathway-activating substance, again in step (3e). These substances added in step (3e) may be the same as or different from the substances used in the above steps, but are preferably the same. The concentration and type of the additive can be suitably adjusted. The additive used in step (3e) more preferably contains at least one selected from the group consisting of a BMP signaling pathway inhibitory substance, TGFβ signaling pathway inhibitory substance, Wnt signaling pathway-activating substance, FGF signaling pathway-activating substance, and EGF signaling pathway-activating substance.

From a viewpoint of promoting maturation of an olfactory epithelial-like tissue, preferably, a retinoic acid signaling pathway-activating substance is further present in step (3e). Examples of the retinoic acid signaling pathway-activating substance include all-trans-retinoic acid, isotretinoin, 9-cis-retinoic acid, TTNPB, Ch55, EC19, EC23, Fenretinide, Acitretin, Trifarotene, and Adapalene. These substances can be used singly or in combination.

The retinoic acid signaling pathway-activating substance preferably contains EC23. The concentration of the retinoic acid signaling pathway-activating substance in medium is not particularly limited as long as the concentration is in a range in which the above-mentioned effect is achievable, and when EC23 is used as a retinoic acid signaling pathway-activating substance, the concentration of EC23 is, for example, about 10 pM to about 10 µM, preferably about 100 pM to about 5 µM, more preferably about 1 nM to about 1 µM, and further preferably about 10 nM to about 500 nM. When a retinoic acid signaling pathway-activating substance other than EC23 is used, it is desirable to use such a substance in a concentration that provides a retinoic acid signaling pathway-promoting activity equivalent to EC23 of the above concentration.

From a viewpoint of promoting maturation of an olfactory epithelial-like tissue, preferably, serum is further present in step (3e). The serum is not particularly limited, and serum typically used for cell culture can be used. The concentration of the serum in medium is not particularly limited as long as the concentration is in a range in which the above-mentioned effect is achievable, and, for example, about 1% to 20%, and preferably about 3% to about 15%.

From viewpoints of cell protection and improvement of the efficiency in production of an olfactory epithelial-like tissue by simulating a physical environment in a living organism, it is preferable to culture a cell cluster in a medium containing a thickener and having viscosity.

The means to impart viscosity to medium is not particularly limited, and, for example, viscosity can be imparted by adding to medium an appropriate concentration of a substance commonly known as a thickener. Any thickener can be used which can impart such an appropriate viscosity to medium and does not adversely affect cells (no cytotoxicity) in a concentration range that can impart the viscosity. Examples of the thickener include methylcellulose, pectin, guar gum, xanthan gum, tamarind gum, carrageenan, locust bean gum, gellan gum, dextrin, diutan gum, starch, tara gum, alginic acid, curdlan, casein sodium, carob bean gum, chitin, chitosan, glucosamine, pullulan, agarose, dietary fibers and chemically modified substances or derivatives thereof; polysaccharides such as cellulose and agarose; ethers of polysaccharides such as ethylcellulose, hydroxyethylcellulose, hydroxypropylcellulose, hydroxyethylmethylcellulose, hydroxypropylmethylcellulose, hydroxyethyl ethylcellulose, hydroxypropyl ethyl cellulose, ethylhydroxyethylcellulose, dihydroxypropylcellulose, and hydroxyethylhydroxypropylcellulose; synthetic polymers such as polyacrylamide, polyethylene oxide, polyvinylpyrrolidone, ethylene glycol/propylene glycol copolymer, polyethyleneimine polyvinylmethyl ether, polyvinyl alcohol, polyacrylic acid, and maleic acid copolymer; biopolymers such as collagen, gelatin, hyaluronic acid, dextran, and carrageenan; and artificial polymers that mimic them (for example, elastin-like peptide). Preferably, these thickeners can be used singly or as a mixture of some types of thickeners. In addition, a copolymer of water-soluble polymers used as thickeners may be used. Preferably, methylcellulose, polyethylene glycol, polyvinylpyrrolidone, carboxymethylcellulose, or a mixture of them can be used, and more preferably methylcellulose can be used.

For the medium having viscosity, medium that exhibits a viscosity of typically 100 mPa·S or higher, preferably 500 mPa·S or higher, more preferably 1000 mPa·S or higher, and further preferably 2000 mPa·S or higher under conditions of 37°C, which is preferably used as a culture environment, is used.

The concentration of the thickener to be added to medium can be suitably adjusted as long as the concentration can impart viscosity and does not cause toxicity to cells. When methylcellulose (4000 cP) is used as a thickener, methylcellulose is used in a concentration of typically 0.5% to 10%, preferably 1% to 7.5%, and further preferably 2% to 5%.

From a viewpoint of preventing the thinning of a tissue including olfactory receptor neurons in the cell cluster formed and improving the efficiency in production of a tissue, it is also preferable in step (3e) to carry out adherent culture of the cell aggregate formed.

As a culture vessel to adhere cells, any form of a planar cell culture dish and a cell culture vessel in a thin-film form such as a Transwell can be used. Such a cell culture dish or cell culture vessel may be coated, for example, with a basement membrane preparation, an extracellular matrix such as laminin, or synthesized cell adhesion molecules such as poly-D-lysine to promote cell adhesion.

From a viewpoint of promotion of the survival and maturation of a tissue including olfactory receptor neurons in the cell cluster in step (3e), it is also preferable to culture the cell aggregate by air liquid interface culture. The air liquid interface culture refers to culture under such conditions that at least one surface of cells or a tissue is exposed to the air or positioned in the extreme vicinity of the air. To carry out the air liquid interface culture, for example, cells or a tissue is cultured on a porous membrane or in a culture insert, the culture medium in the insert (lumen side) is extracted for removal, and culture is carried out only with the culture medium in the well in the outer side of the insert; or a hydrogel such as agarose is placed in a culture dish, cells or a tissue or the like is placed thereon and cultured under such conditions that the cells or tissue or the like is exposed from the medium in the dish without being dried. For the purpose of mitigating the toxicity of exposure to the air primarily because of oxygen, exposure conditions may be controlled by placing an oxygen-permeable membrane or sheet such as PDMS on cells or a tissue or the like.

From a viewpoint of promotion of the maturation and proliferation of an olfactory epithelial-like tissue, the start time of step (3e) is, for example, 60 days or less, preferably 30 days or less, and, for example, 12 hours or more, preferably 96 hours or more after the start of step (3).

The period of culture in step (3e) can be suitably set. The culture period in step (3e) is typically about 8 hours to 200 days, preferably about 1 day to 150 days, further preferably about 2 days to 120 days, and most preferably about 4 days to 100 days.

From a viewpoint of promotion of the maturation of olfactory receptor neurons and central nervous system cells, step (3) may include a step of culturing a cell aggregate that is embedded in a gel. Examples of the gel include gels using agarose, methylcellulose, collagen, Matrigel, or the like, and use of Matrigel is preferred.

From a viewpoint of avoiding xenogeneic components and undetermined factors from mixing in, it is preferable to carry out at least one step selected from the consisting of step (1), step (2), and step (3) in the absence of a mouse sarcoma-derived basement membrane preparation (Matrigel). From a viewpoint of promotion of the growth of an olfactory epithelial-like tissue, step (3) may be carried out in the presence of Matrigel. When Matrigel is used, the concentration of the basement membrane preparation in medium is preferably 0.5% or more and 4% or less, and, from a viewpoint of experimental procedures, more preferably 0.5% or more and 1.5% or less. The time to add the basement membrane preparation depends on the state of a cell aggregate, and is, for example, 7 days or more, and more preferably 9 days or more after the start of step (1). The time to add the basement membrane preparation is, for example, 4 days or more or 6 days or more after the start of step (3a), (3b), or (3c).

From a viewpoint of prevention of the thinning of an olfactory epithelial-like tissue formed on the surface of a cell cluster and transdifferentiation into a tissue other than placode-derived tissues, a Wnt signaling pathway inhibitory substance differing from the first Wnt signaling pathway inhibitory substance added in step (1), step (2), and/or step (3) (also referred to as the second Wnt signaling pathway inhibitory substance in the present invention) may be present in step (3). The second Wnt signaling pathway inhibitory substance preferably has an action mechanism differing from that of the first Wnt signaling pathway inhibitory substance, and if a PORCN inhibitor was used as the first Wnt signaling pathway inhibitory substance in step (1), the second Wnt signaling pathway inhibitory substance preferably contains a substance having an inhibitory activity on the canonical Wnt pathway, and more preferably contains a TANK inhibitor. In adding the second Wnt signaling pathway inhibitory substance, it is preferable that no Wnt signaling pathway-activating substance have been added in step (1).

Examples of substances having an inhibitory activity on the intracellular signaling mechanism of the canonical Wnt pathway include a Frizzled inhibitor, Dvl inhibitor, TANK inhibitor, casein kinase 1 inhibitor, catenin responsive transcription inhibitor, p300 inhibitor, CBP inhibitor, and BCL-9 inhibitor.

Examples of the TANK inhibitor include IWR1-endo (4-[(3aR,4S,7R,7aS)-1,3,3a,4,7,7a-hexahydro-1,3-dioxo-4,7-methano-2H-isoindol-2-yl]-N-8-quinolinyl-benzamide), XAV939 (3,5,7,8-Tetrahydro-2-[4-(trifluoromethyl)phenyl]-4H-thiopyrano[4,3-d]pyrimidin-4-one), MN-64 (2-[4-(1-methylethyl)phenyl]-4H-1-benzopyran-4-one), WIKI4 (1-[(1S)-1-(4-Chloro-3-fluorophenyl)-2-hydroxyethyl]-4-[2-[(2-methylpyrazol-3-yl)amino]pyrimidin-4-yl]pyridin-2-one), TC-E 5001 (3-(4-methoxyphenyl)-5-[[[4-(4-methoxyphenyl)-5-methyl-4H-1,2,4-triazol-3-yl]thio]methyl]-1,2,4-oxadiazole), JW 55 (N-[4-[[[[Tetrahydro-4-(4-methoxyphenyl)-2H-pyran-4-yl]methyl]amino]carbonyl]phenyl]-2-furancarboxamide), and AZ6102 (rel-2-[4-[6-[(3R,5S)-3,5-Dimethyl-1-piperazinyl]-4-methyl-3-pyridinyl]phenyl]-3,7-dihydro-7-methyl-4H-pyrrolo[2,3-d]pyrimidin-4-one). These substances can be used singly or in combination.

The TANK inhibitor to be used as the second Wnt signaling pathway inhibitory substance preferably contains at least one selected from the group consisting of IWR1-endo, XAV939, and MN-64, and more preferably contains XAV939. The concentration of the second Wnt signaling pathway inhibitory substance in medium can be suitably set in a range in which the above-mentioned effect is achievable. From a viewpoint of retention of a placode-like tissue and prevention of thinning, when XAV939, one of TANK inhibitors, is used as a second Wnt signaling pathway inhibitory substance, for example, the concentration is typically about 0.01 µM to about 30 µM, preferably about 0.1 µM to about 30 µM, and more preferably about 0.3 µM.

The time to add the second Wnt signaling pathway inhibitory substance may be any stage before the occurrence of the thinning and transdifferentiation of a placode-like tissue on the surface of a cell cluster in step (3), and is typically 12 hours or more and typically 28 days or less, preferably 24 days or less, and more preferably 21 days or less after the start of suspension culture of pluripotent stem cells in step (1).

In step (3), the first Wnt signaling pathway inhibitory substance and the second Wnt signaling pathway inhibitory substance may be simultaneously present in medium, and only the second Wnt signaling pathway inhibitory substance may be added to medium. Alternatively, the first Wnt signaling pathway inhibitory substance may be serially diluted by adding only the second Wnt signaling pathway inhibitory substance in half medium exchange.

The second Wnt signaling pathway inhibitory substance may be present in at least one step selected from the group consisting of step (3a) to (3d) in step (3), or in all the steps of steps (3a) to (3d). The time to add the second Wnt signaling pathway inhibitory substance may be the same as or different from the start of step (3a), step (3b), step (3c), or step (3d).

From a viewpoint of prevention of differentiation into mesendoderms and improvement of the efficiency in differentiation into placodes and efficiency in production of an olfactory epithelial-like tissue, it is also preferable to add an inhibitory substance for Transforming growth factor-β-activated kinase 1 (TAK1). TAK1 is a serine/threonine-protein kinase that mediates signaling activated by TGFβ, bone morphogenetic protein (BMP), interleukin 1 (IL-1), TNF-α, and so on and belongs to the MAP kinase kinase kinase (MAPKKK) family.

The TAK1 inhibitory substance is not limited as long as the inhibitory substance is capable of suppressing signaling mediated by TAK1. The TAK1 inhibitory substance can be nucleic acids, proteins or low molecular organic compounds. Examples of such a substance include substances that inhibit binding between TAK1 and the substrate, substances that inhibit phosphorylation of TAK1, substances that promote dephosphorylation of TAK1, substances that inhibit transcription or translation for TAK1, and substances that promote decomposition of TAK1.

Examples of the TAK1 inhibitory substance include (5Z)-7-Oxozeaenol ((3S,5Z,8S,9S,11E)-3,4,9,10-tetrahydro-8,9,16-trihydroxy-14-methoxy-3-methyl-1H-2-benzoxacyclotetradecin-1,7(8H)-dione), N-Des(aminocarbonyl) AZ-TAK1 inhibitor (3-Amino-5-[4-(4-morpholinylmethyl)phenyl]-2-thiophenecarboxamide), Takinib (N1-(1-Propyl-1H-benzimidazol-2-yl)-1,3-benzenedicarboxamide), NG25 (N-[4-[(4-Ethyl-1-piperazinyl)methyl]-3-(trifluoromethyl)phenyl]-4-methyl-3-(1H-pyrrolo[2,3-b]pyridin-4-yloxy)-benzamide trihydrochloride), and derivatives and analogs of these compounds. These substances can be used singly or in combination.

The TAK1 inhibitory substance used in the present invention is preferably (5Z)-7-Oxozeaenol. When (5Z)-7-Oxozeaenol is used as a TAK1 inhibitory substance in step (3), (5Z)-7-Oxozeaenol is used in a concentration of typically about 1 nM to about 100 µM, preferably about 10 nM to about 50 µM, more preferably about 100 nM to about 25 µM, and further preferably about 500 nM to about 10 µM. When a TAK1 inhibitory substance other than (5Z)-7-Oxozeaenol is used, it is desirable to use such an inhibitory substance in a concentration that provides a TAK1 inhibitory activity equivalent to (5Z)-7-Oxozeaenol of the above concentration.

From a viewpoint of prevention of cell death and promotion of cell proliferation, it is also preferable to add an additional growth factor into medium in step (3). The type of the growth factor to be added is not particularly limited as long as the above purpose is achievable. Examples of the growth factor to be used for such a purpose include Insulin-like growth factor (IGF), Nerve growth factor (NGF), Brain-derived neurotrophic factor (BDNF), neurotrophin 3, neurotrophin 4/5, Ciliary neurotrophic factor (CNTF), Vesicular endothelial growth factor (VEGF), Pigment epithelium-derived factor (PEDF), and Hepatocyte growth factor (HGF). Any of these growth factors can be suitably added in a concentration that allows achievement of the above purpose.

From a viewpoint of prevention of cell death and promotion of cell proliferation, it is also preferable to add a platelet-derived growth factor receptor-activating substance such as Platelet-derived growth factor (PDGF) into medium in step (3). Four genes of A, B, C, and D are present for PDGF, and PDGF forms a homo dimer of AA, BB, CC, or DD, or a hetero dimer with a specific combination to function as a ligand. Two genes of α and β are present for PDGF receptors, and a PDGF receptor forms a homo or hetero dimer with any combination of αα, αβ, and ββ to function as a receptor. Among them, PDGFRβ is well expressed in non-neuronal ectoderms including placodes, and the platelet-derived growth factor-activating substance used in the present invention preferably exhibits an action on PDGFRββ or PDGFRαβ, more preferably contains at least one selected from the group consisting of PDGF-AB, PDGF-BB, PDGF-CC, and PDGF-DD, and further preferably contains at least one selected from the group consisting of PDGF-BB and PDGF-CC. PDGF-AB, PDGF-BB, PDGF-CC, and PDGF-DD are available as recombinant proteins from R&D Systems, Inc., GenScript Biotech Corporation, and so on.

From a viewpoint of improvement of the efficiency in production of an olfactory epithelial-like tissue in step (1), step (2), and/or step (3), it is also preferable to add a compound that promotes differentiation into a placode region. Examples of compounds that exhibit the function as above include BRL-54443 described in U.S. Patent No. US20160326491A1, Phenanthroline, and Parthenolide. When BRL-54443 is used as a compound that promotes differentiation into a placode region, BRL-54443 is used in a concentration of typically 10 nM to 100 µM; when Phenanthroline is used, Phenanthroline is used in a concentration of typically 10 nM to 100 µM; and when Parthenolide is used, Parthenolide is used in a concentration of typically 10 nM to 100 µM.

From a viewpoint of prevention of cell death and promotion of cell proliferation in step (3), it is also preferable to culture under a high-oxygen atmosphere. High-oxygen conditions during culture can be achieved, for example, by artificially supplying oxygen from an oxygen cylinder connected to an incubator to culture cells. The oxygen concentration for such a purpose is typically 25% to 80%, more preferably 30% to 60%.

From a viewpoint of increasing the supply of oxygen into medium to culture a cell cluster in step (3), a culture vessel with a high gas exchange efficiency can also be used. Examples of such a culture vessel include a Lumox dish (manufactured by Sarstedt K.K.), in which the bottom of a cell culture dish or plate is a gas-permeable film, and a VECELL 96 well plate (manufactured by Vessel Inc.).

### [3. Cell clusters including olfactory receptor neurons or precursor cells thereof]

In the present invention, the terms "cell" and "tissue" are those that can be confirmed for the presence thereof by immunostaining using the same marker as corresponding cells and tissues respectively present in a living organism or by other specific methods but the functions and structures of the "cell" and "tissue" are not always identical with cells and tissues present in the living organism. For example, the "olfactory receptor neurons" included in the cell clusters of the present invention can be stained using the same marker as the olfactory receptor neurons present in a living organism but a state of the gene expression thereof and synaptic bond are not always an identical state with the olfactory receptor neurons in the living organism.

The cell cluster in the present invention refers to an aggregate that includes
1) a non-neural epithelial tissue part including olfactory receptor neurons or precursor cells thereof; and
2) a nervous tissue part including neural cells or precursor cells thereof,
wherein the above neural cells or precursor cells thereof include neural cells or precursor cells thereof constituting the central nervous system, and
at least a part of the surface of the above nervous tissue part is covered with the above non-neural epithelial tissue part.

The cell cluster is an artificially formed cell population and has a certain cell species, cell number, shape and structure. The number of cells constituting the cell cluster of the present invention is not particularly limited but, from a viewpoint of forming a cell cluster consisting of a plurality of cell species, preferably 30 cells or more, and more preferably 500 cells or more. The shape of the cell cluster of the present invention is not particularly limited and can be spherical, elliptical, or sheet-like but spherical is preferable. The cell cluster of the present invention can be preferably produced by the above production method of the present invention.

Hereinafter, the cell cluster according to the present invention will be described suitably with reference to FIG 1. An embodiment of the cell cluster according to the present invention is shown in FIG. 1 A to E. The cell cluster includes a non-neural epithelial tissue part and a nervous tissue part. At least a part of the nervous tissue part is covered with the non-neural epithelial tissue part. The surface of the nervous tissue part is covered in preferably 30% or more, more preferably 60% or more, further preferably 80% or more, and most preferably entirely, with the non-neural epithelial tissue part. A gap can be formed in at least a part of between the non-neural epithelial tissue part and the nervous tissue part. The non-neural epithelial tissue part includes olfactory receptor neurons or olfactory receptor precursor cells. The nervous tissue part includes neural cells or precursor cells thereof constituting the central nervous system and can also include neural cells or precursor cells thereof constituting the retina.

### <Non-neural epithelial tissue part including olfactory receptor neurons or precursor cells thereof>

The cell cluster of the present invention includes a non-neural epithelial tissue part. The non-neural epithelial tissue part refers to a tissue that has an epithelial structure and does not include a neural epithelial tissue. The epithelial structure refers to a structure in which cells are linked by a cell-cell junction to form a layer. The layer can be a single layer or multilayers but is preferably 2 or more layers and 5 or less layers, and more preferably 3 or more layers and 4 or less layers. Further, a part of the epithelial structure can be multilayers. The non-neural epithelial tissue part according to the cell cluster of the present invention has the epithelial structure in preferably 50% or more and 90% or less, more preferably 80% or more and 100% or less, and most preferably entirely, among the non-neural epithelial tissue part. The non-neural epithelial tissue part can include an extracellular substrate.

The non-neural epithelial tissue part includes olfactory receptor neurons or precursor cells thereof. The olfactory receptor neurons or precursor cells thereof can be defined as cells that are simultaneously expressing at least one selected from the group consisting of Tuj 1, NCAM, N-Cadherin, and Calretinin which are markers of neural cells and at least one selected from the group consisting of EpCAM, E-Cadherin, and cytokeratin which are markers of non-neural cells, and preferably further expressing at least one selected from the group consisting of Ebf1, Ebf2, Ebf3, NeuroD, Lhx2, and Ascl1 which are transcription factors expressed specifically in the olfactory receptor neurons or precursor cells thereof. The olfactory receptor neurons included in the non-neural epithelial tissue part are preferably expressing an olfactory receptor and more preferably receive olfactory information as do the olfactory receptor neurons locally present in the olfactory epithelium in a living organism and can transduce the information to the central nervous system. The precursor cell of the olfactory receptor neuron refers to a cell that becomes an olfactory receptor neuron when matures and includes cells that have differentiation potency to olfactory receptor neurons. The precursor cells of the olfactory receptor neurons preferably demonstrate the same characteristic as the neurons present in the olfactory epithelial placode. Of the cells constituting the non-neural epithelial tissue part, preferably 1% or more, more preferably 5% or more, further preferably 10% or more and 50% or less of the cells are olfactory receptor neurons or precursor cells thereof.

The non-neural epithelial tissue part preferably further includes a basement membrane-like structure, and the basement membrane-like structure is more preferably formed between the non-neural epithelial tissue part and the nervous tissue part, and further preferably cells included in the non-neural epithelial tissue part are adhered onto the basement membrane-like structure. The basement membrane-like structure refers to a thin membranous structure constituted by extracellular matrices and can be confirmed by laminin immunostaining. The basement membrane-like structure preferably contributes to, as does the basement membrane in a living organism, structure maintenance of the non-neural epithelial tissue part, mechanical separation of the non-neural epithelial tissue part from the nervous tissue part, selective permeation of substances, and the like.

The non-neural epithelial tissue part preferably forms pseudostratified epithelium or stratified epithelium, and more preferably forms pseudostratified epithelium. The pseudostratified epithelium is an epithelial structure in which cells form a plurality of layers on the basement membrane-like structure but all the cellular extensions are in contact with the basement membrane-like structure and thus also called pseudostratified epithelium. The stratified epithelium refers to an epithelial structure in which cells form a plurality of layers on the basement membrane-like structure. The non-neural epithelial tissue part can partially form pseudostratified epithelium or stratified epithelium or can entirely form pseudostratified epithelium or stratified epithelium.

The non-neural epithelial tissue part includes olfactory epithelial-like tissues, and the olfactory receptor neurons or precursor cells thereof are preferably included in the olfactory epithelial-like tissues. The olfactory epithelial-like tissue refers to a region that is expressing at least one selected from the group consisting of Six1, Sp8, Sox2, Sox3, Dlx5, Emx2, Pax6, Otx2, PDGFRβ, cytokeratin, EpCAM, E-Cadherin, and N-Cadherin which are expressed in the olfactory epithelium or precursor tissues thereof (the olfactory epithelial placode), and preferably expressing Otx2, Sp8, Sox2, EpCAM, E-Cadherin, and cytokeratin.

The olfactory epithelial-like tissue preferably further includes at least two kinds of cells selected from the group consisting of supporting cells, basal cells and Bowman's gland cells, and precursor cells thereof, and more preferably includes basal cells or precursor cells thereof. The supporting cell or precursor cell thereof refers to a cell that is expressing at least one selected from the group consisting of HIF2α, Sox2, Hes1, Hes5, and Six1. The basal cell or precursor cell thereof refers to a cell that is expressing at least one selected from the group consisting of p63, Wt1, and Lgr5. The Bowman's gland cell or precursor cell thereof refers to a cell that is expressing at least one selected from the group consisting of cytokeratin and Ascl3.

Further, the olfactory epithelial-like tissue preferably includes olfactory ensheathing glias or precursor cells thereof. The olfactory ensheathing glia or precursor cell thereof refers to a cell that is expressing at least one selected from the group consisting of S100, Sox10, and vimentin. For the marker of the olfactory ensheathing glias or precursor cells thereof, for example, the marker described in Brain Structure and Function 222.4 (2017): 1877-1895 can also be used.

The supporting cells, basal cells, Bowman's gland cells, or olfactory ensheathing glias or precursor cells thereof preferably demonstrate the same characteristic as the supporting cells, basal cells, Bowman's gland cells, or olfactory ensheathing glias or precursor cells thereof present in the olfactory epithelium or olfactory epithelial placode in a living organism.

The olfactory epithelial-like tissue preferably includes the basement membrane-like structure and more preferably form a many-columnar epithelium structure or a stratified epithelium structure. The olfactory epithelial-like tissue preferably has a basal surface opposing to the nervous tissue part and a top end surface positioned on the opposite side of the basal surface, and the base surface includes olfactory receptor precursor cells and basal cells, and the top end surface includes the supporting cells and the olfactory receptor neurons. The basal surface (or olfactory receptor precursor cells) is preferably in contact with the basement membrane, which is a laminin-positive and has an acellular structure. The cells present in the top end surface are preferably expressing PKCζ.

An enlarged non-neural epithelial tissue part of an embodiment of the cell cluster according to the present invention is shown in FIG 1 B. With reference to FIG 1 B, the non-neural epithelial tissue part preferably has a basal surface opposing to the nervous tissue part and a top end surface positioned on the opposite side of the basal surface. In the basal surface, precursor cells of the olfactory receptor neurons (olfactory receptor precursor cells) are preferably present.

The olfactory epithelial-like tissue includes a medial olfactory epithelium and a lateral olfactory epithelium provided around the medial olfactory epithelium and preferably a Lateral-Medial region is caused. The olfactory epithelial-like tissue can also consist of the medial olfactory epithelium and the lateral olfactory epithelium. The medial olfactory epithelium refers to a region in which cells that are expressing at least one selected from the group consisting of Sox2, Tuj1, and Ascl1 are locally present. The lateral olfactory epithelium refers to a region in which cells that are expressing at least one selected from the group consisting of Pbx, Meis1, Pax6, and βIV tubulin, preferably Pax6 and Pbx are locally present.

The non-neural epithelial tissue part can include non-neural epithelial tissues other than the olfactory epithelial-like tissue. The non-neural epithelial tissue refers to a region in which the olfactory receptor neurons or precursor cells thereof are not included, at least one selected from the group consisting of cytokeratin, E-Cadherin, and EpCAM is expressed and Ebf1, Ebf2, Ebf3, NeuroD, Lhx2, and Ascl1 are not expressed. The non-neural epithelial tissue preferably includes the respiratory epithelium and corneal cells, and precursor cells thereof.

An embodiment of the cell cluster according to the present invention is shown in FIG 1 C. With reference to FIG 1 C, the cell cluster can have the nervous tissue part being entirely covered with the non-neural epithelial tissue part and have the olfactory epithelial-like tissue being formed at a part of the non-neural epithelial tissue part. The olfactory epithelial-like tissue can also include a medial olfactory epithelium and a lateral olfactory epithelium provided around the medial olfactory epithelium. Sixty percent or more and 80% or less of the surface of the cell cluster covered with the non-neural epithelial tissue part can be the medial olfactory epithelium. The structure, size, and positional relation of the olfactory epithelial-like tissue and non-neural epithelial tissues are not particularly limited. A gap can be formed between the olfactory epithelial-like tissue and the non-neural epithelial tissue part. Further, as shown in FIG 1 D, a part of the nervous tissue part can be covered with the non-neural epithelial tissue part.

### <Nervous tissue part including neural cells or precursor cells thereof>

The cell cluster of the present invention includes a nervous tissue part. The nervous tissue part includes neural cells or precursor cells thereof. The neural cells or precursor cells thereof include neural cells or precursor cells thereof constituting the central nervous system. The neural cells or precursor cells thereof constituting the central nervous system can be defined as cells that are expressing at least one selected from the group consisting of Pax6, Bf1, Sp8, Sox1, Sox2, Emx2, Tuj1, and N-Cadherin, and are not expressing cytokeratin, and preferably expressing Pax6, Sox2, and Tuj 1. The neural cells or precursor cells thereof constituting the central nervous system preferably demonstrate the same characteristic as the neurons, glia cells, and neural stem cells or precursor cells thereof in the central nervous system in a living organism.

The nervous tissue part can form an epithelial structure. An embodiment of the cell cluster according to the present invention is shown in FIG 1 E. The nervous tissue part is covered with the non-neural epithelial tissue part, and the nervous tissue part forms an epithelial structure. The epithelial structure can be formed in a part or can be formed entirely of the nervous tissue part. A gap can be formed in at least a part of between the non-neural epithelial tissue part and the nervous tissue part.

The neural cell or precursor cell thereof preferably includes neural cells or precursor cells thereof constituting the retina. The neural cells or precursor cells thereof constituting the retina can be defined as cells that are expressing at least one selected from the group consisting of Rax, Six3, Chx10, Pax6, Lhx2, and Aldh1a3, and preferably expressing Rax and Chx10. The neural cells or precursor cells thereof constituting the retina preferably demonstrate the same characteristic as the retinal cells, retinal precursor cells, retina specific neurons, or precursor cells thereof in a living organism.

The central nervous system preferably includes the cerebrum. The cerebrum (including cells constituting the cerebrum) refers to tissues that are expressing at least one selected from the group consisting of Emx family genes, Otx2, Bf1, and Pax6. Further, the cerebrum preferably has a layer structure.

The cerebrum preferably includes the olfactory cortex. The olfactory cortex refers to tissues that are expressing at least one selected from the group consisting of Tbr1, Ctip2, and FoxP2.

The cerebrum preferably includes the basal ganglia or ganglionic eminences thereof. The basal ganglia or ganglionic eminences thereof refer to tissues that are expressing at least one selected from the group consisting of Dlx family genes, Gsh2, Nkx2.1, and Islet1.

The basal ganglia or ganglionic eminences thereof are preferably lateral ganglionic eminence. The lateral ganglionic eminence refers to tissues that are expressing at least one selected from the group consisting of Er81 and Islet1.

The cerebrum preferably includes the olfactory bulb or precursor tissues thereof. The olfactory bulb or precursor tissues thereof refer to tissues that are expressing at least one selected from the group consisting of Arx, Tbr1, and Tbx21.

The cell clusters preferably include gonadotropin releasing hormone-positive neurons. The gonadotropin releasing hormone-positive neuron refers to a cell and precursor cells thereof that are expressing the gonadotropin releasing hormone (GnRH).

Unlike nasal cavity tissues of a living organism, the cell cluster of the present invention does not typically form bone tissues but can include bone tissues therein. Bone tissues, osteocytes, or precursor cells thereof are preferably expressing at least one selected from the group consisting of Runx2, Osterix, and ATF. The formation of bone tissues in the cell clusters formed can be confirmed by a method well known by those skilled in the art, for example, a technique such as alizarin red staining, alkaline phosphatase staining, or immunostaining to the above bone tissue-specific genes.

The technique for detecting cells and tissues included in the cell clusters produced is not particularly limited but a technique that is reliable, highly reproductive and easily carried out is preferable. Some examples include optical observations by a microscope, immunostainings using antibodies to various cell or tissue markers, gene expression analyses such as real-time PCR to a marker gene, functional assays using olfactory receptor-activating substances, transplanting to mouse, rat or the like, with the immunostainings using antibodies to the markers described above being preferable.

Interpretation of the immunostaining result can be carried out by visual conclusion by those skilled in the art, or quantitative analysis using an image analysis software or the like of the images taken, or a method well known by those skilled in the art. For interpreting the results of immunostaining, false-positives caused by autofluorescence, nonspecific adsorption by a secondary antibody, fluorescence leakage during multiple staining or the like must be eliminated with reference to, for example, Protein, nucleic acid and enzyme Vol. 54 No. 2 (2009) P185-192 and the like. In the present invention, the presence or absence of the protein expression was confirmed in accordance with the method shown in Preliminary Experiment 1 to be described later.

### [4. Method for using as a reagent for olfactory receptor-activating substance evaluation]

The present invention can provide an evaluation method for olfactory receptor-activating substances using the above-mentioned "cell clusters including olfactory receptor neurons or precursor cells thereof".

The evaluation method for olfactory receptor-activating substances is, for example, an evaluation method for olfactory receptor-activating substances comprising a step of allowing olfactory receptor neurons or olfactory epithelial-like tissues prepared from the above cell clusters to contact a test substance, and a step of verifying impacts of the test substance on the cells or the tissues and is an identification method for a specific olfactory receptor that reacts the compound to be evaluated. An embodiment of the method for carrying out the olfactory receptor-activating substance evaluation of the present invention can be carried out with reference to, for example, the method described in Scientific Reports 6, 19934 (2016).

An embodiment of the method for carrying out the olfactory receptor-activating substance evaluation of the present invention is a method for carrying out an olfactory receptor-activating substance evaluation comprising the following steps (A) to (D).
(A) A step of separating cells that are expressing olfactory receptors from the cell clusters including olfactory receptor neurons or precursor cells thereof;
(B) a step of allowing a test substance to contact the separated cells that are expressing olfactory receptors;
(C) a step of identifying cells activated by the contact with the test substance; and
(D) a step of identifying olfactory receptors that are expressed in the cells activated by the contact with the test substance.

### <Step (A)>

The step of separating the cells that are expressing olfactory receptors from the cell clusters including olfactory receptor neurons or precursor cells thereof will be described.

In the cell clusters including olfactory receptor neurons or precursor cells thereof, it is olfactory receptor neurons that are expressing olfactory receptors, and thus the separation method in step (A) can be a method that can separate olfactory receptor neurons from the cell clusters. The separation method has desirably little damage on cells and little mixed-in of unintentional cells. An embodiment of the separation method in step (A) can include a step of treating tissues and dispersing to be single cells. Examples of the above dispersion method include enzymatic treatment dispersions using a protease, dispersions by calcium chelating agent treatment, and mechanical dispersions, with the enzymatic treatment dispersions being preferable. An embodiment of the separation method can also purify and separate only cells of interest using a cell surface marker. Examples of the above separation method of cells of interest include fluorescence activated cell sorting (FACS) using an antibody to an olfactory receptor neuron-specific cell surface marker, and magnetic cell sorting (MACS). Examples of the olfactory receptor neuron-specific cell surface marker include OCAM Alternatively, with reference to PloS one, 10(1), e0113170 and the like, proteins, glycans, lipids and the like that are expressed in the cell membrane of olfactory receptor neurons can also be used as markers. The separated cells can be cultured in medium typically used for cell culture. During this procedure, a growth factor and a cell protectant can also be added to suppress cell death.

Further, the olfactory epithelial-like tissue is separated from a cell cluster and can be used for the evaluation. The olfactory epithelial-like tissue formed on outside the cell cluster can be peeled and collected from the above-mentioned cell cluster using tweezers or the like under a microscope observation. The olfactory epithelial-like tissue can be confirmed as a semi-transparent thin epithelium present in the surface layer of the obtained cell cluster as described in, for example, Nature communications, 2016, 7. For the collection method of the olfactory epithelial-like tissue, freezing and thawing, preferably slow freezing method, can also be used. In such a method, the cell cluster having the olfactory epithelial-like tissue on the outside and the neural epithelial tissue inside is frozen and thawed whereby outside the olfactory epithelial-like tissue is peeled from the cell cluster without carrying out the mechanical treatment.

### <Step (B)>

The step of allowing cells or tissues that are expressing the separated olfactory receptors to contact a test substance will be described.

The cells or tissues allowed to contact a test substance in step (B) can be in a suspended state or an adhered state but, from a viewpoint of enhancing the survival rate of the olfactory receptor neurons and enabling later steps such as washing to be easily carried out, the cells in an adhered state are preferable. For the culture vessel to which cells are adhered, either flat cell culture dishes or thin membranous cell culture vessels such as Transwell can be used. These cell culture dishes or cell culture vessels can be coated with an extracellular substrate such as laminin or a synthetic substrate such as Poly-D-Lysine for accelerating the adhesion of cells.

The test substance used in step (B) can be used directly as an active ingredient or used as diluted in a solvent. For such an instance, the solvent used for dilution is preferably those that do not affect test results and, for example, medium for cell culture such as physiological saline, phosphate buffered saline (PBS), Hanks' Balanced Salt Solution (HBSS), and DMEM can be used. When a test substance is insoluble in a culture medium and fails to obtain good suspendability, DMSO, ethanol, a mineral oil or the like can be used as a solubilizing solvent as necessary.

The culture conditions such as the culture temperature and CO₂ concentration for the exposure condition of a test substance in step (B) can be suitably set. The culture temperature is, for example, from about 30°C to about 40°C, and preferably about 37°C. Further, the CO₂ concentration is, for example, from about 1% to about 10%, and preferably about 5%. The exposure time of a test substance in step (B) can also be suitably set. The exposure time is, for example, from 30 seconds to 2 days, and preferably from 1 minute to 1 day.

### <Step (C)>

The step of identifying the cells activated by the contact with the test substance will be described.

The technique that is carried out in step (C) is not limited as long as the cells activated by the contact with a test substance can be identified but a technique that is simple, low cost, and capable of treating a large amount of specimen at a time is preferable. Some examples include a technique for detecting membrane potential changes, a technique for detecting gene expression changes, and a technique for detecting intracellular ion concentration changes, with a technique for detecting intracellular ion concentration changes using a calcium indicator such as Fluo4 AM being preferable.

### <Step (D)>

The step of identifying the olfactory receptors expressed in the cells activated by the contact with a test substance will be described.

The technique that is carried out in step (D) is not limited as long as the olfactory receptors expressed in the activated cells can be identified but a technique that is simple, low cost, and capable of treating a large amount of specimen at a time is preferable. Some examples include the single-cell RNA sequencing.

### [5. Olfactory receptor-activating substance evaluation kit or olfactory receptor screening kit]

The present invention can provide an olfactory receptor-activating substance evaluation kit or an olfactory receptor screening kit to carry out the above-mentioned [4. Method for using as a reagent for olfactory receptor-activating substance evaluation].

This reagent or kit includes the cell clusters of the present invention or olfactory receptor neurons or olfactory epithelial-like tissues separated from the cell clusters by the above-mentioned step (A) and the like, and at least one of a reagent, a culture medium, a culture container, and an analysis program to be used to carry out the above-mentioned steps (B) to (D), and preferably include a reagent to be used as a positive control or a negative control, or a reagent for identifying the cells activated by the contact with a test substance. The present kit can further include a written instruction or a manual that describes the procedure for the screening.

The present invention can provide a method and a kit for evaluating neurotoxicity or drug efficacy by allowing the neurons or the nervous tissues included in the cell clusters to contact a test substance as in the above-mentioned [4. Method for using as a reagent for olfactory receptor-activating substance evaluation] and [5. Olfactory receptor-activating substance evaluation kit or olfactory receptor screening kit].

### [6. Therapeutic drug and treatment method for diseases]

The present invention can provide a therapeutic drug for diseases due to a nervous tissue disorder or sensory organ disorder, preferably diseases due to an olfactory disorder and comprising the above cell clusters, or cells or tissues included in the above cell clusters. Examples of the nervous tissue disorder include disorders of the central nervous system such as spinal cord injuries, brain damages, and neurodegenerative diseases. Examples of the brain damage include hemorrhagic strokes, ischemic attacks, brain damages by encephalopathy, traumatic brain injuries, and brain damages by cerebral infarction and cerebral hemorrhage. Examples of the neurodegenerative disease include Alzheimer's disease, multiple sclerosis (MS), peripheral nerve disorder, Huntington's disease, amyotrophic lateral sclerosis, and Parkinson's disease. These diseases can be of non-human animals. The cells present in the olfactory epithelium (including olfactory receptor neurons and precursor cells thereof) have higher regenerative ability than other nervous tissue-derived cells and are hence prominent as a therapeutic drug for nerve injuries.

Examples of the therapeutic drug include suspensions including the cell clusters of the present invention or cells or tissues included in the cell clusters, and grafts including these (transplant transporter). Examples of the suspension include solutions in which the cell clusters are suspended in artificial tears or physiological saline. The suspension can also include non-neural epithelial cells isolated from the cell clusters and can also include a factor that accelerates adhesion of cells such as extracellular substrates and hyaluronic acid.

Further, a treatment method for diseases comprising a step of transplanting cells or tissues included in the cell clusters of the present invention in an effective dose required for the treatment to a subject in need of the transplant can be provided. The transplant of a therapeutically effective dose of cells can recover nervous tissues and typically alleviate disease-related symptoms.

### Examples

Hereinafter, the present invention is described in more detail with Examples, but these are not limiting of the scope of the present invention. In addition, reagents and materials used are commercially available unless otherwise limited.

### [Preliminary Experiment 1: Quantification and determination criteria for immunostaining result]

Quantification of fluorescence intensity was performed to determine positive and negative of fluorescence immunostained specimens. As the method for quantifying staining intensity, a linear fluorescence intensity profile of the region of interest shown in line A-A' was output to the image of the immunofluorescence staining result with an anti-Lhx2 antibody of a frozen section of the cell cluster on day 28 of culturing prepared in Test Example 2 described later (upper section of FIG. 2) (maximum excitation wavelength 490 nm, maximum fluorescence wavelength 525 nm), and the fluorescence intensities of the region in which the frozen section of tissue was present and the region in which the tissue was absent were compared. In the analysis results shown in the lower section of FIG. 2, the fluorescence intensity of the region in which the tissue was absent was 219, while the numerical value of the region with strong fluorescence intensity that was visually determined to be positive was 4053.333, and the numerical value of the positive region with weaker fluorescence intensity than above was 2043.667. Thus, as shown by dashed lines in the graph in the lower section of FIG. 2, it was found that a positive or negative determination of staining results of the antigen can be quantitatively performed by setting a region showing a numerical value greater than or equal to 5 times the average fluorescence intensity of the region in which a tissue was confirmed to be absent in the bright-field as positive. The following experiments were performed in the same manner as in this preliminary experiment to determine whether an expression was positive or negative.

### [Comparative Experiment 1: Production of cell aggregate including nervous tissue]

In accordance with the procedures shown in the upper section of FIG. 3, the pluripotent stem cells were suspension-cultured in the presence of a Wnt signaling pathway inhibitory substance to produce cell aggregates. Human ES cells (KhES-1 strain, obtained from Kyoto University) were cultured under feeder-free conditions in accordance with the method described in Scientific Reports, 4, 3594 (2014). StemFit medium (AK02N, manufactured by Ajinomoto Co., Inc.) was used as the feeder-free medium, and Laminin 511-E8 (manufactured by Nippi. Inc.) was used as the feeder-free scaffold.

As specific maintenance culturing manipulations, human ES cells that became sub-confluent were first washed with PBS, then enzymatically treated with Accumax (manufactured by Innovative Cell Technologies, Inc.). StemFit medium was then added, and the cells were peeled off from the surface of the culture dish with a cell scraper, and dispersed into single cells by pipetting. The human ES cells dispersed into single cells were then seeded into a Laminin 511-E8-coated plastic culture dish, and cultured in the presence of Y27632 (ROCK inhibitory substance, manufactured by FUJIFILM Wako Pure Chemical Corporation, 10 µM) in the StemFit medium under feeder-free conditions. When a 6-well plate (manufactured by Corning, plate for cell culture, culture area 9.5 cm²) was used as the plastic culture dish, the number of seeded cells of the human ES cells dispersed into single cells was set to 1.2 x 10⁴. One day after the seeding, the medium was fully exchanged with StemFit medium being free of Y27632. Thereafter, once every 1 to 2 days, the medium was fully exchanged with StemFit medium being free of Y27632. For 7 days after seeding, the cells were cultured until sub-confluent (approximately 60% of the culture area was covered with the cells).

When the cultured cells were used for differentiation induction, SB-431542 (TGF-β signaling pathway inhibitory substance, manufactured by FUJIFILM Wako Pure Chemical Corporation, final concentration 5 µM) and SAG (Shh signal pathway-activating substance, manufactured by Enzo Life Sciences, final concentration 300 nM) were added at the same time with the culture medium exchange with StemFit medium after 6 days from seeding, and the cells were cultured for 24 hours (step (a)).

The prepared sub-confluent human ES cells were washed with PBS, then enzymatically treated with Accumax. A serum-free medium for differentiation induction was then added, and the cells were peeled off from the surface of the culture dish with a cell scraper, and dispersed into single cells by pipetting.

Then, the human ES cells dispersed into single cells were suspended into 100 µl of a serum-free medium in a non-cell-adhesive 96-well culture plate (PrimeSurface 96 V-bottom plate, MS-9096V, manufactured by Sumitomo Bakelite Co., Ltd.) at 1.2 x 10⁴ cells per well, and the cells were suspension-cultured under conditions of 37°C, 5%CO₂. The serum-free medium (gfCDM + KSR) used was a serum-free medium of a mixture of F-12 + Glutamax medium (manufactured by Thermo Fisher Scientific) and IMDM + Glutamax medium (manufactured by Thermo Fisher Scientific) in a volume ratio of 1 : 1 supplemented with 5% Knockout Serum Replacement (manufactured by Thermo Fisher Scientific), 450 µM 1-monothioglycerol (manufactured by FUJIFILM Wako Pure Chemical Corporation), 1x Chemically defined lipid concentrate (manufactured by Thermo Fisher Scientific), and 50 unit/ml penicillin-50 µg/ml streptomycin (manufactured by NACALAI TESQUE, INC.)

At the start of suspension culturing (day 0 after the start of suspension culturing, start of step (1)), Y27632 (final concentration 20 µM), IWP-2 (first Wnt signaling pathway inhibitory substance, manufactured by Tocris Biosciences, final concentration 2 µM), SB-431542 (TGF signaling pathway inhibitory substance, manufactured by FUJIFILM Wako Pure Chemical Corporation, final concentration 1 µM) were added to the serum-free medium described above.

Thereafter, a serum-free medium being free of Y27632 and containing IWP-2 and SB-431542 (also abbreviated as SB431) was added at 100 µl per well on day 3 after the start of suspension culturing. Thereafter, on days 6, 10, 13, 17, 21, and 24 after the start of suspension culturing, the medium was half exchanged with a serum-free medium being free of Y27632 and containing IWP-2 and SB-431542.

On day 28 after the start of suspension culturing, aggregates were collected into a dish, and bright-field observation was performed with an inverted microscope (manufactured by KEYENCE CORPORATION, BIOREVO) (FIG. 3A). As a result, human pluripotent stem cell aggregates were formed by the differentiation induction method described above.

The cell aggregates on day 28 after the start of suspension culturing were collected into a tube for culturing with a 200 µl wide bore tip, washed twice with PBS, and then fixed with 4% paraformaldehyde phosphate buffer (manufactured by FUJIFILM Wako Pure Chemical Corporation) for 15 minutes at room temperature. After fixation, the cell aggregates were washed three times with PBS, and then immersed in 20% sucrose/PBS overnight at 4°C for cryoprotective treatment. The aggregate after the cryoprotective treatment was transferred to cryomold No. 3 (manufactured by Sakura Finetek), and the excess 20% sucrose/PBS around the cell aggregate was removed with a micropipetter. The cells were then embedded in an O. C. T compound (manufactured by Sakura Finetek), and rapidly frozen on an aluminum heat block cooled with dry ice to prepare blocks for frozen section preparation. Frozen sections with a thickness of 10 µm were prepared with Lycra CM1950 cryostat (manufactured by Leica) from the blocks embedding the above aggregate, and attached onto PLATINUM PRO coated glass slides (manufactured by Matsunami Glass Co., Ltd.). The surroundings of the frozen sections on the glass slides were enclosed with ultra pap pens (manufactured by Bio Medical Science Inc.) and then permeabilized with 0.2% Triton-X 100/TBS for 10 minutes at room temperature, followed by blocking treatment with a mixture solution of a blocking reagent N-102 (manufactured by NOF corporation.) and SuperBlock (TBS) Blocking Buffer (manufactured by Thermo Fisher Scientific) in a volume ratio of 1 : 4 for 30 minutes at room temperature.

To the frozen sections after the blocking treatment, 300 µl of a primary antibody diluted with Antibody Diluent OP Quanto (manufactured by Thermo Fisher Scientific) per glass slide was added, and the mixture was allowed to react overnight at 4°C in a wet box. The frozen sections after the primary antibody treatment were subjected to three washing manipulations of treating with 0.05% Tween-20/TBS for 10 minutes at room temperature. Secondary antibodies were diluted with a buffer of a mixture of Blocking One Histo (manufactured by NACALAI TESQUE, INC.) and 0.2% Triton-X 100/TBS in a volume ratio of 1 : 19. The diluent was then added to the frozen sections at 300 µl per glass slide, and allowed to react at room temperature for 1 hour. The frozen sections after the secondary antibody treatment were subjected to three washing manipulations of treating with 0.05% Tween-20/TBS for 10 minutes, then washed once with pure water. The frozen sections were then mounted with NEO cover glass (manufactured by Matsunami Glass Industries, Ltd.) and 30 µl of Prolong Diamond (manufactured by Thermo Fisher Scientific) per glass slide. The mounted specimens were allowed to stand overnight in the dark at room temperature to solidify Prolong Diamond. The surroundings of the cover glass were then coated with a clear varnish, then the varnish was air-dried in the dark at room temperature, and then observation by fluorescence microscopy was performed. For observation of the specimens and acquisition of the images, an upright fluorescence microscope Axio Imager M2 (manufactured by Carl Zeiss) and the affiliated software Axio Vision were used.

As primary antibodies, an anti-Dlx5 antibody staining placode and cells of central nervous system, an anti-Sox1 antibody staining cells of central nervous system, a pan-cytokeratin (PanCK) antibody staining non-nervous tissues including placode, and an anti-Tuj 1 antibody staining neurons, described in Table 1, were used. As fluorescently labeled secondary antibodies, an Alexa488-labeled donkey anti-rabbit antibody, a CF555-labeled donkey anti-goat antibody, a CF555-labeled donkey anti-mouse antibody, and an Alexa647-labeled donkey anti-mouse antibody, described in Table 2, were used to perform multiple staining. For contrast staining of the nucleus, 1 µg/ml of Hoechst 33342 (manufactured by Sigma Aldrich, Inc.) was added to the secondary antibody diluent.

The staining results are shown in FIG. 3. The cell aggregate included cells of central nervous system expressing Sox1 and Dlx5 (FIGS. 3B, 3C). Furthermore, since pan-cytokeratin-positive tissue could not be observed on the surface of the cell aggregate and the cell aggregate was Tuj 1-positive, it was found that the formed cell aggregate did not contain non-neural epithelial tissues and consisted only of the cells of central nervous system (FIGS. 3D, 3F). A schematic diagram of the cell aggregate assumed from the staining results is shown in FIG. 3H.

### [Comparative Experiment 2: Production of cell aggregate including nervous tissue and non-neural epithelial tissue]

Cell aggregates were produced by adding a step of culturing in the presence of a BMP signaling pathway-activating substance (step (2)) to Comparative Experiment 1 and in accordance with the procedures shown in the upper section of FIG. 4. First, human ES cells (KhES-1 cell line) were subjected to maintenance culturing and step (a) under the same manipulations as Comparative Experiment 1, then suspension culturing was started in the presence of Y27632 (final concentration 20 µM), IWP-2 (final concentration 2 µM) and SB-431542 (final concentration 1 µM) (day 0 after the start of suspension culturing, start of step (1)).

On day 2 after the start of suspension culturing, a serum-free medium being free of Y27632 and containing IWP-2, SB-431542 and BMP4 (BMP signaling pathway-activating substance, manufactured by R&D Systems) was added at 100 µl per well (start of step (2)). BMP4 was added to the medium at 3 nM to be a final concentration in the well of 1.5 nM. Thereafter, on days 6, 10, 13, 17, 21, and 24 after the start of suspension culturing, the medium was half exchanged with a serum-free medium being free of Y27632 and BMP4 and containing IWP-2 and SB-431542.

On day 28 after the start of suspension culturing, the cell aggregates were collected into a dish, and bright-field observation was performed with an inverted microscope (FIG. 4A). As a result, it was found that aggregates including nervous tissues and non-neural epithelial tissues were formed from human ES cells by the differentiation induction method described above. It was also found that the external non-neural epithelial tissues rapidly expanded from day 21 to day 28 of the suspension culturing, and a space was formed between the external non-neural epithelial tissues and the internal nervous tissues.

The cell aggregates on day 28 after the start of suspension culturing were subjected to immunofluorescence staining in accordance with the same method as in Comparative Experiment 1. As primary antibodies, an anti-Tuj 1 antibody staining neurons; an anti-Sox2 antibody staining placode and central nervous system; an anti-PanCK antibody, an anti-Sixl antibody and an anti-EpCAM antibody staining non-neural epithelial tissues including placode; an anti-Sp8 antibody staining olfactory epithelial placode and central nervous system; an anti-N-Cadherin antibody staining placode and neural epithelial tissues; an anti-Pax6 antibody staining central nervous system including retina, and placode and lateral olfactory epithelium; an anti-ChxlO antibody staining retina; an anti-Proxl antibody, an anti-C-Maf antibody and an anti-Crystalline αA antibody staining crystalline placode; and an anti-Sox 1 antibody staining crystalline placode and central nervous system, described in Table 1, were used. As fluorescently labeled secondary antibodies, an Alexa488-labeled donkey anti-rabbit antibody, a CF555-labeled donkey anti-mouse antibody, a CF555-labeled donkey anti-goat antibody, an Alexa647-labeled donkey anti-mouse antibody, and an Alexa647-labeled donkey anti-goat antibody, described in Table 2, were used to perform multiple staining. Hoechst 33342 was used for contrast staining of the nucleus.

The staining results are shown in FIGS. 4 and 5. It was found that the internal of the cell aggregate on day 28 after the start of suspension culturing produced by the culture method described above was Tuj1, N-Cadherin, Pax6, Chx10-positive epithelial tissues of neural retina (FIGS. 4B, 4H, 4K, 4L), and the external thereof was EpCAM, Six1, pan-cytokeratin-positive non-neural epithelial tissues of cornea or ocular surface ectoderm (FIGS. 4J, 4F, 4D). In addition, since a portion of the non-neural epithelial tissues was thickened and the thickened portion was C-Maf, Sox1, Prox1, Crystalline aA-positive (FIGS. 5O to 5U), it was found that crystalline placode was formed in the cell aggregate by the culture method described above. It was also found that pan-cytokeratin-positive mesenchymal cells that did not form epithelium were present between-Chx10-positive internal retinal tissues and pan-cytokeratin-positive external non-neural epithelial tissues (FIGS. 4D, 4E). A schematic diagram of the cell aggregate assumed from the above staining results is shown in FIG. 5V.

### [Experiment 1: Production of cell cluster including olfactory receptor neuron or precursor cell thereof from ES cell]

Cell clusters were produced by adding a step (3b) of culturing in the presence of a BMP signaling pathway inhibitory substance to Comparative Experiment 2, and in accordance with the procedures shown in the upper section of FIG. 6. In Experiment 1, cell aggregates on day 13 after the start of suspension culturing were observed.

First, human ES cells (KhES-1 cell line) were subjected to maintenance culturing and step (a) under the same manipulations as in Comparative Experiment 1, then suspension culturing was started in the presence of Y27632 (final concentration 20 µM), IWP-2 (final concentration 2 µM) and SB-431542 (final concentration 1 µM) using a PrimeSurface 96 V-bottom plate (day 0 after the start of suspension culturing, start of step (1)).

On day 2 after the start of suspension culturing, a serum-free medium being free of Y27632 and containing IWP-2, SB-431542 and BMP4 was added at 100 µl per well (start of step (2)). BMP4 was added to the medium at 3 nM to be a final concentration in the well of 1.5 nM.

In addition, on day 3 after the start of suspension culturing, the medium was half exchanged with a serum-free medium being free of Y27632 and BMP4 and containing IWP-2, SB-431542 and K02288 (BMP signaling pathway inhibitory substance, manufactured by AdooQ Biosciences) (start of step (3b)). K02288 was added to the medium at 20 µM to be a final concentration in the well of 10 µM. Thereafter, on days 6 and 10 after the start of suspension culturing, the medium was half exchanged with a serum-free medium being free of Y27632 and BMP4 and containing IWP-2, SB-431542 and K02288.

On day 13 after the start of suspension culturing, bright-field observation was performed with an inverted microscope (FIG. 6A). As a result, it was found that spherical cell aggregates of around 600 µm in diameter were formed from human ES cells by the differentiation induction method described above.

The cell aggregates on day 13 after the start of suspension culturing were subjected to immunofluorescence staining in accordance with the same method as in Comparative Experiment 1. As primary antibodies, an anti-Dlx5 antibody, an anti-Sixl antibody, an anti-pan-cytokeratin (PanCK) antibody and an anti-E-Cadherin antibody staining placode and non-neural epithelial tissues; an anti-N-Cadherin antibody staining placode and neural epithelial tissues; an anti-Sox1 antibody, an anti-Sox2 antibody, an anti-Sox3 antibody, an anti-Pax6 antibody and an anti-Sp8 antibody staining placode and neural cells; an anti-AP2α antibody staining ectoderm at early stage in embryogenesis; and an anti-Otx2 antibody staining anterior regions of embryo, described in Table 1, were used. As fluorescently labeled secondary antibodies, the antibodies described in Comparative Experiment 2 were used to perform multiple staining, and Hoechst 33342 was used for contrast staining of the nucleus. The positive and negative determinations of the staining results were performed in the same manner as in Preliminary Experiment 1.

The staining results are shown in FIGS. 6 and 7. It was found that the internal of the cell aggregates on day 13 after the start of suspension culturing induced by the differentiation induction method described above was tissues composed of Sox1, Sox2, Sox3, Sp8, N-Cadherin-positive central nervous system cells or precursor cells thereof, and the external thereof was Dlx5, Pax6, AP2α, Six1, Sp8, Sox2, Sox3, pan-cytokeratin, E-Cadherin, N-Cadherin-positive non-neural epithelial tissues or placode (FIGS. 6B to 6L, FIGS. 7M to 7R). Since the epithelial tissues formed on the external were almost monolayers, and no thickening was observed, it was found that the states at day 13 of the culturing were those of preplacode region and placode precursor cells. It was also found that the anterior regions of the embryo were induced since both of the cells were Otx2-positive (FIG. 6J). No osteoprecursor cell-like cells could be observed between the internal Sox1-positive central nervous system cells or precursor cells thereof and the external pan-cytokeratin-positive non-neural epithelial tissues. A schematic diagram of the cell aggregate formed by the above culture method is shown in FIG. 7S.

### [Experiment 2: Production of cell cluster including olfactory receptor neuron or precursor cell thereof from ES cell]

In Experiment 2, a cell cluster including an olfactory receptor neuron or a precursor cell thereof was produced by performing the same experimental manipulations as in Experiment 1, but extending the culturing period over Experiment 1, as shown in the upper section of FIG. 8. In the same manner as in Experiment 1, steps (a), (1) and (2) were performed, and then, on day 3 after the start of suspension culturing, the medium was half exchanged with a serum-free medium being free of Y27632 and BMP4 and containing IWP-2, SB-431542, and K02288 (start of step (3b)). Thereafter, on days 6, 10, 13, 17, 21, and 24 after the start of suspension culturing, the medium was half exchanged with a serum-free medium being free of Y27632 and BMP and containing IWP-2, SB-431542, and K02288.

On day 28 after the start of suspension culturing, bright-field observation was performed with an inverted microscope (FIG. 8A). As a result, it was found that spherical cell clusters of around 600 µm in diameter were formed from human ES cells, as in Experiment 1.

The cell clusters on day 28 after the start of suspension culturing were subjected to immunofluorescence staining in accordance with the same method as in Comparative Experiment 1. As primary antibodies, an anti-Dlx5 antibody, an anti-Sixl antibody, an anti-pan-cytokeratin (PanCK) antibody, an anti-E-Cadherin antibody and an anti-Otx2 antibody staining olfactory epithelial placode; an anti-Pax6 antibody and a Pbx1/2/3/4 staining lateral olfactory epithelium; an anti-N-Cadherin antibody, an anti-Sox2 antibody, an anti-Sp8 antibody and an anti-Emx2 antibody staining olfactory epithelial placode and cells of central nervous system; an anti-Ebfl antibody, an anti-NCAM antibody, an anti-Calretinin antibody and an anti-NeuroD antibody staining olfactory receptor neurons and precursor cells thereof; an anti-Bfl antibody staining cells of central nervous system; an anti-Tuj 1 antibody staining cells of central nervous system and olfactory receptor neurons; an anti-ChxlO antibody staining neural retina; and an anti-Lhx2 antibody staining neural retina and olfactory receptor neurons, described in Table 1, were used. As fluorescently labeled secondary antibodies, the antibodies described in Comparative Experiment 2 were used to perform multiple staining, and Hoechst 33342 was used for contrast staining of the nucleus. The positive and negative determinations of the staining results were performed in the same manner as in Preliminary Experiment 1.

The staining results are shown in FIGS. 8 and 9. On day 28 from the start of suspension culturing, a cell cluster was formed in which the external was an E-Cadherin, N-Cadherin, pan-cytokeratin, Sox2, Six1, Dlx5, Sp8, Pax6, Otx2-positive non-neural epithelial tissue part of placode-like (olfactory epithelial-like tissue), and the internal was an E-Cadherin, pan-cytokeratin-negative and N-Cadherin, Sox2, Sp8, Bf1, Emx2, Tuj 1-positive nervous tissue part including neural cells or precursor cells thereof constituting central nervous system (cerebrum). In addition, some cells in the external placode-like epithelial tissue (olfactory epithelial-like tissue) were Lhx2, Ebf2, Tuj1, NCAM, Calretinin-positive olfactory receptor neurons or immature precursor cells thereof. On the external of the cell cluster, a Pax6-positive lateral olfactory epithelium was formed. NeuroD-positive cells were also observed on the basal surface of the olfactory epithelial-like tissue (FIGS. 8B-M, FIGS. 9N-AI). From the above, it was found that, by subjecting pluripotent stem cells to suspension culturing in the presence of a first Wnt signaling pathway inhibitory substance, culturing the resulting cell aggregate in the presence of a BMP signaling pathway-activating substance, and then further culturing in the presence of a BMP signaling pathway inhibitory substance, a cell cluster including: 1) a non-neural epithelial tissue part including an olfactory receptor neuron or a precursor cell thereof; and 2) a nervous tissue part including a neural cell or a precursor cell thereof, wherein the neural cell or a precursor cell thereof includes a neural cell or a precursor cell thereof constituting a central nervous system, and at least a part of the surface of the nervous tissue part is covered with the non-neural epithelial tissue part, can be produced. In addition, the non-neural epithelial tissue part included an olfactory epithelial-like tissue in which an olfactory receptor neuron or a precursor cell thereof was present, and the olfactory epithelial-like tissue included a medial olfactory epithelium and a lateral olfactory epithelium. A schematic diagram of the cell cluster formed by the above production method is shown in FIG. 10.

### [Experiment 3: Production of cell cluster including olfactory receptor neuron or precursor cell thereof from human iPS cell]

In Experiment 3, as shown in the upper section of FIG. 11, a cell cluster including an olfactory receptor neuron or a precursor cell thereof was produced by performing a step (3a) of culturing in the presence of an FGF signaling pathway-activating substance, FGF2, in place of a step (3b) of culturing in the presence of a BMP signaling pathway inhibitory substance performed in Experiment 2. Human iPS cells (HC-6#10 cell line, obtained from the Institute of Physical and Chemical Research) were used as pluripotent stem cells. In addition, the culturing period was set to 21 days. The same manipulations as in Experiment 1 were performed except for those specifically indicated.

Specifically, steps (a), (1) and (2) were performed with maintenance-cultured human iPS cells, and then, on day 3 after the start of suspension culturing, the medium was half exchanged with a serum-free medium being free of Y27632 and BMP4 and containing IWP-2, SB-431542, FGF2 and Heparin Sodium (start of step (3a)). FGF2 (FGF signal pathway-activating substance, manufactured by FUJIFILM Wako Pure Chemical Corporation) and Heparin Sodium (manufactured by FUJIFILM Wako Pure Chemical Corporation) were added to the medium at 40 ng/ml and 20 µg/ml, respectively, to be final concentrations in the well of 20 ng/ml and 10 µg/ml. Thereafter, on days 6, 10, 13, and 17 after the start of suspension culturing, the medium was half exchanged with a serum-free medium being free of Y27632 and BMP4 and containing IWP-2, SB-431542, FGF2, and Heparin Sodium.

On day 21 after the start of suspension culturing, bright-field observation was performed with an inverted microscope (FIGS. 11A, 11B). As a result, it was found that spherical cell clusters of around 600 µm in diameter were formed from human iPS cells, as in the case formed from human ES cells in Experiment 1.

The cell clusters on day 21 after the start of suspension culturing were subjected to immunofluorescence staining in accordance with the same method as in Comparative Experiment 1. As primary antibodies, an anti-Tuj1 antibody and an anti-NCAM antibody staining neural cells; an anti-ChxlO antibody staining neural retina and precursor cells thereof; an anti-Sixl antibody, an anti-pan-cytokeratin (PanCK) antibody, an anti-E-Cadherin antibody and an anti-EpCAM antibody staining placode and non-neural epithelial tissues; an anti-N-Cadherin antibody staining placode and neural epithelial tissues; an anti-Sox2 antibody and an anti-Pax6 antibody staining placode and neural cells; an anti-Sp8 antibody staining olfactory epithelial placode and cells of central nervous system; an anti-Otx2 antibody staining anterior regions of embryo; and an anti-Ebf1 antibody and an anti-Ebf2 antibody staining olfactory receptor neurons and precursor cells thereof, described in Table 1, were used. The antibodies described in Comparative Experiment 2 were used as fluorescently labeled secondary antibodies to perform multiple staining. Hoechst 33342 was used for contrast staining of the nucleus. The positive and negative determinations of the staining results were performed in the same manner as in Preliminary Experiment 1.

The staining results are shown in FIGS. 11 and 12. On day 21 from the start of suspension culturing, a cell cluster was formed in which the external was an E-Cadherin, N-Cadherin, pan-cytokeratin, EpCAM, Sox2, Six1, Sp8-positive non-neural epithelial tissue part of placode-like (olfactory epithelial-like tissue), and the internal was an E-Cadherin, pan-cytokeratin, EpCAM-negative and N-Cadherin, NCAM, Tuj1, Chx10-positive neural epithelial tissue of neural retina. It was found that a tissue in the anterior region of embryo was formed since it was Otx2-positive. In addition, some cells in the external placode-like epithelial tissue (olfactory epithelial-like tissue) were Ebf1, Ebf2, Tuj1, NCAM-positive olfactory receptor neurons or immature precursor cells thereof (FIGS. 11C to 11N, FIGS. 12O to 12Z). From the above, it was found that, by subjecting pluripotent stem cells to suspension culturing in the presence of a first Wnt signaling pathway inhibitory substance, culturing the resulting cell aggregate in the presence of a BMP signaling pathway-activating substance, and then further culturing in the presence of an FGF signaling pathway-activating substance, a cell cluster including: 1) a non-neural epithelial tissue part including an olfactory receptor neuron or a precursor cell thereof, and 2) a nervous tissue part including a neural cell or a precursor cell thereof, wherein the neural cell or a precursor cell thereof includes a neural cell or a precursor cell thereof constituting a central nervous system, and at least a part of the surface of the nervous tissue part is covered with the non-neural epithelial tissue part, can be produced. It was also found that the nervous tissue part on the internal of the cell cluster forms an epithelial structure, and that the neural cell or a precursor cell thereof includes a cell constituting a retina. A schematic diagram of the cell cluster formed by the above production method is shown in FIG. 12AA.

### [Experiment 4: Production of cell cluster including olfactory receptor neuron or precursor cell thereof from human iPS cell]

In Experiment 4, as shown in the upper section of FIG. 13, in step (3), a cell cluster including an olfactory receptor neuron or a precursor cell thereof were produced by performing a step (3a) of culturing in the presence of an FGF signaling pathway-activating substance, and then performing a step (3c) of culturing in the presence of an FGF signaling pathway-activating substance and a BMP signaling pathway inhibitory substance. The culturing period was 28 days. The same manipulations as in Experiment 3 were performed except for those specifically indicated.

Specifically, steps (a), (1) and (2) were performed with maintenance-cultured human iPS cells, and then, on day 3 after the start of suspension culturing, the medium was half exchanged with a serum-free medium being free of Y27632 and BMP4 and containing IWP-2, SB-431542, FGF2 and Heparin Sodium (start of step (3a)). FGF2 and Heparin Sodium were added to the medium at 40 ng/ml and 20 µg/ml, respectively, to be final concentrations in the well of 20 ng/ml and 10 µg/ml. Thereafter, on days 6 and 10 after the start of suspension culturing, the medium was half exchanged with a serum-free medium being free of Y27632 and BMP4 and containing IWP-2, SB-431542, FGF2, and Heparin Sodium. In addition, on day 13 after the start of suspension culturing, the culturing was started with a medium containing K02288 at a final concentration of 1 µM in addition to the above substances (start of step (3c)), and on days 17, 21, and 24, the medium was half exchanged with a medium containing IWP-2, SB-431542, FGF2, Heparin Sodium, and K02288.

On day 28 after the start of suspension culturing, bright-field observation was performed with an inverted microscope (FIG. 13A). As a result, it was found that spherical cell clusters of around 600 µm in diameter were formed from human iPS cells, as in the case formed from human ES cells in Experiment 1.

The cell clusters on day 28 after the start of suspension culturing were subjected to immunofluorescence staining in accordance with the same method as in Comparative Experiment 1. As primary antibodies, antibodies against Dlx5, NeuroD1, NCAM, p63, Sox2, E-Cadherin, Pax6, Chx10, N-Cadherin, Six1, Sp8, EpCAM, Tuj1, Ebf2, and pan-cytokeratin, described in Table 1, were used. The antibodies described in Comparative Experiment 2 were used as fluorescently labeled secondary antibodies to perform multiple staining. Hoechst 33342 was used for contrast staining of the nucleus. The positive and negative determinations of the staining results were performed in the same manner as in Preliminary Experiment 1.

The staining results are shown in FIGS. 13 and 14. On day 28 from the start of suspension culturing, a cell cluster was formed in which the external was an E-Cadherin, N-Cadherin, pan-cytokeratin, EpCAM, Sox2, Six1, Sp8-positive non-neural epithelial tissue part of placode-like (olfactory epithelial-like tissue), and the internal was an E-Cadherin, pan-cytokeratin, EpCAM-negative and N-Cadherin, NCAM, Tuj1, Chx10-positive neural epithelial tissue of neural retina. In addition, some cells in the external placode-like epithelial tissue (olfactory epithelial-like tissue) were NeuroD1, Ebf2, Tuj 1, NCAM-positive olfactory receptor neurons or immature precursor cells thereof (FIGS. 13B to 13M, FIGS. 14N to 14U). From the above, by subjecting pluripotent stem cells to suspension culturing in the presence of a first Wnt signaling pathway inhibitory substance, culturing the resulting cell aggregate in the presence of a BMP signaling pathway-activating substance for a certain period of time, and then culturing in the presence of an FGF signaling pathway-activating substance, and further culturing in the presence of an FGF signaling pathway-activating substance and a BMP signaling pathway inhibitory substance, a cell cluster similar to that in Experiment 3 could be produced. In addition, neural cells or precursor cells thereof inside of the cell cluster formed a neural epithelial tissue of neural retina. Furthermore, compared to Experiment 3, it was found that culturing in the presence of both an FGF signaling pathway-activating substance and a BMP signaling pathway inhibitory substance from day 13 after the start of suspension culturing results in the formation of a non-neural epithelial tissue part including an olfactory receptor neuron or a precursor cell thereof which is more stable than that when cultured in the presence of only an FGF signaling pathway-activating substance. A schematic diagram of the cell cluster formed by the above production method is shown in FIG. 14V.

### [Experiment 5: Production of cell cluster including olfactory receptor neuron or precursor cell thereof from human iPS cell]

In Experiment 5, as shown in the upper section of FIG. 15, a cell cluster including an olfactory receptor neuron or a precursor cell thereof was produced from human iPS cells by further adding an EGF signaling pathway-activating substance to the conditions of Experiment 4 in step (3c). The same manipulations as in Experiment 3 were performed except for those specifically indicated.

Specifically, step (a) was performed with maintenance-cultured human iPS cells, then the cells were seeded into a non-cell-adhesive 96-well culture plate (PrimeSurface 96 V-bottom plate), and steps (1), (2) and (3a) were performed. Then, on day 13 after the start of suspension culturing, in addition to the substances described above, K02288 at a final concentration of 1 µM and human recombinant EGF (EGF signaling pathway-activating substance, manufactured by PrimeGene) at a final concentration of 20 ng/ml were added (start of step (3c)), and the medium was half exchanged on days 17, 21, and 24 with a medium containing IWP-2, SB-431542, FGF2, Heparin Sodium, K02288 and EGF.

On day 28 after the start of suspension culturing, bright-field observation was performed with an inverted microscope (FIG. 15A). As a result, it was found that spherical cell clusters of around 600 µm in diameter were formed from human iPS cells, as in the case formed from human ES cells in Experiment 1.

The cell clusters on day 28 after the start of suspension culturing were subjected to immunofluorescence staining in accordance with the same method as in Comparative Experiment 1. As primary antibodies, antibodies against Dlx5, NeuroD1, NCAM, p63, Sox2, E-Cadherin, Pax6, Chx10, N-Cadherin, Six1, Sp8, EpCAM, Tuj1, Ebf2, and pan-cytokeratin, described in Table 1, were used. The antibodies described in Comparative Experiment 2 were used as fluorescently labeled secondary antibodies to perform multiple staining. Hoechst 33342 was used for contrast staining of the nucleus. The positive and negative determinations of the staining results were performed in the same manner as in Preliminary Experiment 1.

The staining results are shown in FIGS. 15 and 16. On day 28 from the start of suspension culturing, a cell cluster was formed in which the external was an E-Cadherin, N-Cadherin, pan-cytokeratin, EpCAM, Sox2, Six1, Sp8-positive non-neural epithelial tissue part of placode-like (olfactory epithelial-like tissue), and the internal was an E-Cadherin, pan-cytokeratin, EpCAM-negative and N-Cadherin, NCAM, Tuj1, Chx10-positive neural epithelial tissue of neural retina. In addition, some cells in the external placode-like epithelial tissue (olfactory epithelial-like tissue) were NeuroD1, Ebf2, Tuj 1, NCAM-positive olfactory receptor neurons or immature precursor cells thereof (FIGS. 15B to 15M, FIGS. 16N to 16U). From the above, by subjecting pluripotent stem cells to suspension culturing in the presence of a first Wnt signaling pathway inhibitory substance, culturing the resulting cell aggregate in the presence of a BMP signaling pathway-activating substance, and then further culturing in the presence of an FGF signaling pathway-activating substance for a certain period of time, and then culturing in the presence of an FGF signaling pathway-activating substance, a BMP signaling pathway inhibitory substance and an EGF signaling pathway-activating substance, a cell cluster similar to that in Experiment 3 could be produced. It was also found that neural cells or precursor cells thereof inside of the cell cluster formed a neural epithelial tissue of neural retina. Furthermore, compared to Experiment 3, it was found that culturing in the presence of an FGF signaling pathway-activating substance, a BMP signaling pathway inhibitory substance, and further an EGF signaling pathway-activating substance from day 13 from the start of suspension culturing results in the formation of a non-neural epithelial tissue part including an olfactory receptor neuron or a precursor cell thereof which is more stable than that when cultured in the presence of only an FGF signaling pathway-activating substance.

### [Experiment 6: Production of cell cluster including olfactory receptor neuron or precursor cell thereof from human iPS cell]

In Experiment 6, as shown in the upper section of FIG. 17, a cell cluster including an olfactory receptor neuron or a precursor cell thereof was produced by performing step (3c) as step (3) from the beginning, adding EGF from the middle of step (3c), and then culturing in the presence of XAV939 as a second Wnt signaling pathway inhibitory substance. The same manipulations as in Experiment 3 were performed except for those specifically indicated.

Specifically, steps (a), (1) and (2) were started with maintenance-cultured human iPS cells, and then, on day 3 after the start of suspension culturing, the medium was half exchanged with a serum-free medium being free of Y27632 and BMP4 and containing IWP-2, SB-431542, FGF2, Heparin Sodium and K02288 (start of step (3c)). FGF2, Heparin Sodium, and K02288 were included to be final concentrations in the well of 20 ng/ml, 10 µg/ml, and 1 µM, respectively. Thereafter, on days 6 and 10 after the start of suspension culturing, the medium was half exchanged with a serum-free medium being free of Y27632 and BMP4 and containing IWP-2, SB-431542, FGF2, Heparin Sodium and K02288. In addition, on day 13 after the start of suspension culturing, in addition to the above factors, EGF (manufactured by PrimeGene Co., Ltd.) was added to be a final concentration of 20 ng/ml. Furthermore, on day 17 after the start of suspension culturing, in addition to IWP-2, SB-431542, FGF2, Heparin Sodium, K02288, and EGF, XAV939 (manufactured by Cayman Chemicals) was added to be a final concentration of 300 nM, and the medium was half exchanged on days 21 and 24 after the start of suspension culturing.

On day 28 after the start of suspension culturing, bright-field observation was performed with an inverted microscope (FIG. 17A). As a result, it was found that spherical cell clusters of around 600 µm in diameter were formed from human iPS cells, as in the case formed from human ES cells in Experiment 1.

The cell clusters on day 28 after the start of suspension culturing were subjected to immunofluorescence staining in accordance with the same method as in Comparative Experiment 1. As primary antibodies, in addition to antibodies against Dlx5, NeuroD1, NCAM, p63, Sox2, E-Cadherin, Pax6, Chx10, N-Cadherin, Six1, Sp8, EpCAM, Tuj1, Ebf2, pan-cytokeratin, Lhx2, Calretinin, and Otx2 described in Table 1, an anti-Nestin antibody staining immature neurons and neural crests, an anti-Islet-1 antibody staining olfactory epithelial placode and a nerve nuclei of some of central nervous system, an anti-P-catenin antibody staining epithelial cells, an anti-PKCζ antibody staining top end surfaces, an anti-Laminin antibody staining basal membranes, an anti-CK8 antibody staining non-neural epithelium, and an anti-Eya2 antibody staining placode were used. The antibodies described in Comparative Experiment 2 were used as fluorescently labeled secondary antibodies to perform multiple staining. Hoechst 33342 was used for contrast staining of the nucleus. The positive and negative determinations of staining results were performed in the same manner as in Preliminary Experiment 1.

The staining results are shown in FIGS. 17 to 19. On day 28 from the start of suspension culturing, a cell cluster was formed in which the external was an E-Cadherin, N-Cadherin, pan-cytokeratin, EpCAM, Sox2, Six1, Sp8, Eya2, Islet-1, β-Catenin-positive non-neural epithelial tissue part of placode-like (olfactory epithelial-like tissue), and in part of the internal of which an E-Cadherin, pan-cytokeratin, EpCAM-negative and N-Cadherin, NCAM, Tuj1, Chx10-positive neural epithelial tissue of neural retina was formed. The olfactory epithelial-like tissue on the external of the cell cluster had a PKCζ-positive top end surface on the external and a Laminin-positive basal surface on the internal, with top end-basal surface polarity. In addition, some cells in the external placode-like epithelial tissue (olfactory epithelial-like tissue) were Lhx2, NeuroD1, Ebf2, Tuj 1, Calretinin, and NCAM-positive olfactory receptor neurons or immature precursor cells thereof. (FIGS. 17B to 17M, FIGS. 18N to 18AE, FIGS. 19AF to 19AJ). From the above results, it was found that, by subjecting pluripotent stem cells to suspension culturing in the presence of a first Wnt signaling pathway inhibitory substance, culturing the resulting cell aggregate in the presence of a BMP signaling pathway-activating substance, and then further culturing in the presence of an FGF signaling pathway-activating substance and a BMP signaling pathway inhibitory substance, and by adding an EGF signaling pathway-activating substance and a second Wnt signaling pathway material, a cell cluster similar to that in Experiment 3 could be produced. It was also found that neural cells or precursor cells thereof inside of the cell cluster formed a neural epithelial tissue of neural retina. Furthermore, compared to Experiment 3, it was found that the addition of EGF on day 13 from the start of suspension culturing and the addition of a second Wnt signaling pathway inhibitory substance on day 17 from the start of suspension culturing results in the formation of a non-neural epithelial tissue part including an olfactory receptor neuron or a precursor cell thereof which is more stable than that under the conditions without addition of those. A schematic diagram of the formed cell cluster is shown in FIG. 19AK.

### [Experiment 7: Study for addition-concentration of BMP signaling pathway-activating substance in cell cluster production]

In Experiment 7, a study of cell cluster production efficiency was performed for concentration of the BMP signaling pathway-activating substance in step (2), using human ES cells. The same experimental manipulations as in Experiment 2 (see the upper section of FIG. 8) were performed, and the concentration of BMP4 in the medium in step (2) was set to 7 conditions of 0.025 nM, 0.1 nM, 0.25 nM, 0.5 nM, 1.5 nM, 5 nM, and addition-free control.

On day 28 after the start of suspension culturing, bright-field observation was performed with an inverted microscope. As a result, it was found that spherical cell clusters of around 600 µm in diameter were formed from human ES cells as in Experiment 1 in all of the BMP4 concentrations except for the control in which a BMP signaling pathway-activating substance was not added.

In addition, evaluation was performed for the cell clusters formed by adding each concentration of BMP4 according to the following four stages: cell cluster in a form close to spherical, whose 80% or more of the entire circumference was covered with non-neural epithelial tissue part (Grade 1, example = FIG. 20A); cell cluster whose 40% to 80% of the entire circumference was covered with non-neural epithelial tissue part or cell cluster in a distorted form (Grade 2, example = FIG. 20B); cell cluster whose ratio of non-neural epithelial tissue parts in the surface of the cell cluster was 40% or less (Grade 3, example = FIG. 20C); and cell cluster in which no non-neural epithelial tissue part was formed (Grade 4, example = FIG. 20D). The evaluation was performed by bright-field observation with an inverted microscope. As a result, non-neural epithelial tissues were not observed under the culture condition without adding of BMP4, as shown in FIG. 20E. Under the culture conditions in which 0.5 nM or more BMP4 was added in step (2), the Grade 1 cell clusters in a form close to a sphere, whose 80% or more of the entire circumference was covered with the non-neural epithelial tissue parts, were formed with high efficiency. It was thus found that, in the efficient production of a cell cluster including a non-neural epithelial tissue part including an olfactory receptor neuron or a precursor cell thereof, the concentration of BMP4 added in step (2) is preferably 0.5 nM or more.

### [Experiment 8: Study for pretreatment before start of differentiation induction in cell cluster production]

In Experiment 8, a study of cell cluster production efficiency was performed with or without a pretreatment step (a), using human iPS cells. Experiments were performed in accordance with the procedures shown in the upper section of FIG. 21, and the same experimental manipulations as in Experiment 1 were performed except for those specifically indicated. Maintenance-cultured human iPS cells were pretreated at the same time with the culture medium exchange with StemFit medium after 6 days from seeding into a 6-well plate by any of the following four conditions: (1) addition of DMSO alone (control) at the same amount as the other conditions, (2) addition of 300 nM SAG alone, (3) addition of 5 µM SB-431542 alone, and (4) addition of 5 µM SB431542 and 300 nM SAG were added.

Twenty-four hours after the start of step (a), suspension culturing was started in the presence of Y27632 (final concentration 20 µM), SB-431542 (final concentration 1 µM) and IWP-2 (final concentration 2 µM) in the same manner as in Experiment 1 (start of step (1)), and on day 2 after the start of suspension culturing, the medium was half exchanged with a medium being free of Y27632 and containing IWP-2, SB-431542 and BMP4 (final concentration 1.5 nM) (start of step (2)). On day 3 after the start of suspension culturing, the medium was half exchanged with a serum-free medium being free of Y27632 and BMP4 and containing IWP-2, SB-431542, FGF2, Heparin Sodium, and K02288 (start of step (3c)). FGF2, Heparin Sodium, and K02288 were included to be final concentrations in the well of 20 ng/ml, 10 µg/ml, and 1 µM, respectively. Thereafter, on days 6 and 10 after the start of suspension culturing, the medium was half exchanged with a serum-free medium being free of Y27632 and BMP4 and containing IWP-2, SB-431542, FGF2, Heparin Sodium and K02288.

On day 13 after the start of suspension culturing, bright-field observation was performed with an inverted microscope (FIGS. 21A to 21G). The cell aggregate formed from human iPS cells which had not been pretreated was smaller in diameter than the cell aggregate that had been pretreated by the (2) to (4). Also in the cell aggregate, variations were observed among wells in the formation of non-neural epithelial tissues on the external of the cell aggregate, and not only the cell aggregate in which non-neural epithelial tissues were formed but also the cell aggregate in which non-neural epithelial tissues were not formed was observed (FIGS. 21A to 21D). Meanwhile, 90% or more of the cell aggregates formed from cells that had been pretreated under any of the conditions of (2) SAG alone, (3) SB-431542 alone, and (4) SAG + SB-431542 had a larger diameter than the control, and surface non-neural epithelial tissues were also efficiently formed (FIGS. 21E to 21G).

In addition, the aggregate clusters formed from human iPS cells that had been pretreated under each condition were evaluated in four stages of the Grades 1 to 4, as in Experiment 7. As a result, when a pretreatment was performed under any of the conditions of (2) SAG alone, (3) SB-431542 alone, and (4) SAG + SB-431542, the Grade 1 cell cluster in a form close to a sphere, whose 80% or more of the entire circumference was covered with the non-neural epithelial tissue parts, was stably formed with high efficiency, as shown in FIG. 21H. It was thus found that in the efficient production of a cell cluster including an olfactory receptor neuron or a precursor cell thereof, it is preferable to perform pretreatment under any of the conditions of (2) SAG alone, (3) SB-431542 alone, and (4) SAG + SB-431542.

### [Experiment 9: Study for first Wnt signaling pathway inhibitory substance in cell cluster production]

In Experiment 9, a study of cell cluster production efficiency was performed for the type of the first Wnt signaling pathway inhibitory substance, using human iPS cells. The experiments were performed in accordance with the procedures shown in the upper section of FIG. 22, and the same manipulations as in Experiment 3 were performed except for those specifically indicated. The suspension culturing of the human iPS cells subjected to maintenance culturing and step (a) was started in a medium supplemented with a first Wnt signaling pathway inhibitory substance, Y27632 (final concentration 20 µM) and SB-431542 (final concentration 1 µM).

As the first Wnt signaling pathway inhibitory substance, IWP-2 (manufactured by Tocris Biosciences, final concentration 2 µM), IWP-3 (manufactured by Cayman Chemicals, final concentration 2 µM), IWP-4 (manufactured by Cayman Chemicals, final concentration 2 µM), Wnt C-59 (manufactured by Cayman Chemicals, final concentration 20 nM), or KY02111 (manufactured by Cayman Chemicals, final concentration 1 µM) was used. As a control in which the first Wnt signaling pathway inhibitory substance was not added, DMSO was added.

On day 2 after the start of suspension culturing, a serum-free medium being free of Y27632 and containing each first Wnt signaling pathway inhibitory substance, SB-431542 and BMP4 (final concentration 1.5 nM) was added at 100 µl per well. In addition, on day 3 after the start of suspension culturing, the medium was half exchanged with a serum-free medium being free of Y27632 and BMP4 and containing SB-431542, K02288, FGF2, Heparin Sodium, and EGF. K02288, FGF2, Heparin Sodium, EGF were included to final concentrations in the well were 10 µM, 20 ng/ml, 10 µg/ml and 20 ng/ml, respectively. On days 6, 10, 13, 17, 21, and 24 after the start of suspension culturing, the medium was half exchanged with a serum-free medium being free of Y27632 and BMP4 and containing each first Wnt signaling pathway inhibitory substance, SB-431542, K02288, FGF2, Heparin Sodium, and EGF.

On day 28 after the start of suspension culturing, bright-field observation was performed with an inverted microscope (FIGS. 22A to 22F). It was found that spherical cell clusters in which non-neural epithelial-like tissues were formed on the external were formed, as in Experiment 3, except for the condition that the first Wnt signaling pathway inhibitory substance was not added.

The cell clusters on day 28 after the start of suspension culturing were subjected to immunofluorescence staining in accordance with the same method as in Comparative Experiment 1. As primary antibodies, an anti-Six 1 antibody and an anti-pan-cytokeratin antibody staining placode and non-neural epithelial tissues, and an anti-Sox2 antibody staining placode and neural cells, described in Table 1, were used. As fluorescently labeled secondary antibodies, an Alexa488-labeled donkey anti-rabbit antibody, a CF555-labeled donkey anti-goat antibody, and an Alexa647-labeled donkey anti-mouse antibody, described in Table 2, were used to perform multiple staining. Hoechst 33342 was used for contrast staining of the nucleus. The positive and negative determinations of the staining results were performed in the same manner as in Preliminary Experiment 1.

The staining results are shown in FIG. 23. Under the conditions in which the first Wnt signaling pathway inhibitory substance was not added, no non-neural epithelial tissue was formed on the surface of the cell aggregate even when BMP4 was added on day 2 after the start of suspension culturing. Meanwhile, under each condition in which IWP-2, IWP-3, IWP-4, Wnt C-59, or KY02111 was added from the start of suspension culturing, neural epithelial-like aggregates were present on the internal, and aggregates having non-neural epithelial tissues on the surface were formed. Furthermore, as a result of immunofluorescence staining, it was found that Six1, Sox2, cytokeratin-positive, thickened olfactory epithelial placode-like tissues were formed in the non-neural epithelium on the surface of the cell cluster under conditions in which various first Wnt signaling pathway inhibitory substances were added (FIG. 23G). From the above results, it was found that the addition of a first Wnt signaling pathway inhibitory substance is useful for the production of a cell cluster including an olfactory epithelial placode-like non-neural epithelial tissue by addition of a BMP signaling pathway-activating substance.

### [Experiment 10: Cell cluster production with three-dimensional culture vessel]

In Experiment 10, human iPS cells were cultured using a culture vessel different from Experiment 5, and cell clusters were produced by washing the BMP signaling pathway inhibitory substance on day 4 from the start of suspension culturing. The experiments were performed in accordance with the procedures shown in the upper section of FIG. 24, and the same experimental manipulations as in Experiment 3 were performed except for those specifically indicated.

Specifically, maintenance-cultured human iPS cells were subjected to step (a), and then the human iPS cells dispersed into single cells were suspended in 100 µl of a serum-free medium in a non-cell-adhesive 96-well three-dimensional culture vessel (PrimeSurface 96 slit-well plate, MS-9096S, manufactured by Sumitomo Bakelite Co., Ltd.) to be 1 x 10⁴ cells per well, and the cells were suspension-cultured under conditions of 37°C, 5%CO₂. At the start of suspension culturing (day 0 after the start of suspension culturing, start of step (1)), Y27632 (final concentration 20 µM), IWP-2 (final concentration 2 µM), and SB-431542 (final concentration 1 µM) were added to the serum-free medium.

On day 2 after the start of suspension culturing, 19.2 ml of a serum-free medium being free of Y27632 and containing IWP-2, SB-431542 and BMP4 was added per plate (start of step (2)). BMP4 was added to the medium at 2.25 nM to be a final concentration in the medium of 1.5 nM.

On day 3 after the start of suspension culturing, the medium was half exchanged with a serum-free medium being free of Y27632 and BMP4 and containing IWP-2, SB-431542, K02288 (final concentration 10 µM), FGF2 (final concentration 20 ng/ml), EGF (final concentration 20 ng/ml) and Heparin Sodium (final concentration 10 µg/ml) (start of step (3c)). K02288, FGF2, EGF and Heparin Sodium were added to the medium at twice the concentration to be final concentrations in the well as described. Next, on day 4 after the start of suspension culturing, the medium in the plate was removed by suction with a pipette, then a washing manipulation with 15 ml of a 1 : 1 mixture of IMDM and F-12 was performed three times, and 30 ml of a medium being free of Y27632, BMP4 and K02288 and containing IWP-2, SB-431542, FGF2, EGF and Heparin Sodium was added to remove K02288 in the plate as much as possible (start of step (3d)). On days 10, 13, and 17 after the start of suspension culturing, the medium was half exchanged with a serum-free medium being free of Y27632, BMP4, and K02288 and containing IWP-2, SB-431542, FGF2, EGF, and Heparin Sodium.

On day 28 after the start of suspension culturing, bright-field observation was performed with an inverted microscope (FIGS. 24A and 24B). Cell clusters with thickened olfactory epithelial-like non-neural epithelial tissues on the external were formed even under differentiation induction conditions in which a slit-well plate, a three-dimensional culture vessel, was used and the BMP signaling pathway inhibitory substance was removed from the medium during culturing.

### [Experiment 11: Study for time to add BMP signaling pathway-activating substance in cell cluster production]

In Experiment 11, study of cell cluster production efficiency was performed for time to add the BMP signaling pathway-activating substance, that is, the start time of step (2), by changing the time to add the BMP signaling pathway-activating substance from day 1 to day 6 after the start of suspension culturing, using human iPS cells. The experiments were performed in accordance with the procedures shown in the upper section of FIG. 25, and the same experimental manipulations as in Experiment 3 were performed except for those specifically indicated.

Maintenance-cultured human iPS cells were subjected to step (a), and then the human iPS cells dispersed into single cells were suspended into 100 µl of a serum-free medium in a non-cell-adhesive 96-well three-dimensional culture vessel (PrimeSurface 96 slit-well plate, MS-9096S, manufactured by Sumitomo Bakelite Co., Ltd.) to be 1 x 10⁴ cells per well, and the cells were suspension-cultured under conditions of 37°C, 5%CO₂.At the start of suspension culturing (day 0 after the start of suspension culturing, start of step (1)), Y27632 (final concentration 20 µM), IWP-2 (final concentration 2 µM) and SB-431542 (final concentration 1 µM) were added to the serum-free medium.

On any of days 1, 2, 3, 4, and 6 after the start of suspension culturing, a serum-free medium being free of Y27632 and containing IWP-2, SB-431542 and BMP4 was added at 100 µl per well (start of step (2)). BMP4 was added to the medium at 3 nM to be a final concentration in the well of 1.5 nM.

In addition, on day after the addition of a serum-free medium containing BMP4, the medium was half exchanged with a serum-free medium being free of Y27632 and BMP4 and containing SB-431542, K02288, FGF2, Heparin Sodium, and EGF (start of step (3c)). K02288, FGF2, Heparin Sodium, and EGF were added to the medium at 20 µM, 40 ng/ml, 20 µg/ml, and 40 ng/ml, respectively, to be final concentrations in the well were 10 µM, 20 ng/ml, 10 µg/ml, and 20 ng/ml. Thereafter, on days 6 and 10 after the start of suspension culturing, the medium was half exchanged with a serum-free medium being free of Y27632 and BMP4 and containing IWP-2, SB-431542, FGF2, Heparin Sodium and K02288. In the conditions of adding BMP4 on days 4 and 6 after the start of suspension culturing, 100 µl of the medium containing only SB-431542 and IWP-2 was added on day 3 after the start of suspension culturing, and 100 µl of the medium was removed from the well immediately before adding the medium containing BMP4.

On day 13 after the start of suspension culturing, bright-field observation was performed with an inverted microscope (FIGS. 25A to 25E). Under conditions in which BMP4 was added on any of days 1, 2, and 3 after the start of suspension culturing, a non-neural epithelial tissue was formed on the surface of the cell aggregate. Furthermore, under the condition in which BMP4 was added on day 2 after the start of suspension culturing, cell aggregates having non-neural epithelial tissues on the surface with larger diameter of the cell aggregate were most efficiently and stably formed (FIGS. 25A to 25C). Meanwhile, under conditions in which BMP4 was added on day 4 or 6 after the start of suspension culturing, only thick neural epithelium of neural retina was formed, and no formation of non-neural epithelial tissue was observed on the surface of the cell aggregates (FIGS. 25D, 25E). From the above results, it was found that, in order to produce a cell cluster including a nervous tissue part and a non-neural epithelial tissue part, it is preferable to start step (2) by adding a BMP signaling pathway-activating substance within 96 hours after the start of suspension culturing, and more preferably by adding a BMP signaling pathway-activating substance from 24 hours to 72 hours after the start of suspension culturing.

### [Experiment 12: Study for start time of step (2) in cell cluster production]

In Experiment 12, the surfaces of cell aggregates on days 2 to 6 after the start of suspension culturing were observed, and a study for appropriate time to add the BMP signaling pathway-activating substance was performed. The experiments were performed in accordance with the procedures shown in the upper section of FIG. 26, and the same experimental manipulations as in Comparative Experiment 1 were performed except for those specifically indicated.

Maintenance-cultured human iPS cells were subjected to step (a), and then the human iPS cells dispersed into single cells were suspended into 100 µl of a serum-free medium in a non-cell-adhesive 96-well three-dimensional culture vessel (PrimeSurface 96 slit-well plate, MS-9096S, manufactured by Sumitomo Bakelite Co., Ltd.) to be 1 x 10⁴ cells per well, and the cells were suspension-cultured under conditions of 37°C, 5%CO₂.At the start of suspension culturing (day 0 after the start of suspension culturing, start of step (1)), Y27632 (final concentration 20 µM), IWP-2 (final concentration 2 µM), and SB-431542 (final concentration 1 µM) were added to the serum-free medium.

On any of days 2, 3, 4, and 6 after the start of suspension culturing, bright-field observation was performed with an inverted microscope (FIGS. 26A to 26D). The aggregates on day 2 after the start of suspension culturing showed irregularities on the surface, and had a distorted shape, whereas the aggregates on days 3, 4, and 6 after the start of suspension culturing had reduced irregularities which were observed on day 2, and had a shape close to a sphere (FIGS. 26B to 26D).

The cell clusters on days 2, 3, 4 and 6 after the start of suspension culturing were subjected to immunofluorescence staining in accordance with the same method as in Comparative Experiment 1. As the primary antibody, an anti-ZO-1 antibody staining tight junction, described in Table 1, was used. As the fluorescently labeled secondary antibody, an Alexa488-labeled donkey anti-rabbit antibody was used. Hoechst 33342 was used for contrast staining of the nucleus. The positive and negative determinations of the staining results were performed in the same manner as in Preliminary Experiment 1.

The staining results are shown in FIG. 26. In cell aggregates on day 2 after the start of suspension culturing, some of the cells in the most superficial layer of the aggregates were ZO-1 -positive and formed tight junction (FIG. 26E). In cell aggregates on days 3, 4, and 6 after the start of suspension culturing, the percentage of ZO-1-positive cells in the superficial layer was significantly reduced (FIGS. 26F to 26H). It was found that, as described in Experiment 11, the second day after the start of suspension culturing in the production of a cell cluster including an olfactory receptor neuron or a precursor cell thereof is the preferred time to add a BMP signaling pathway-activating substance. It was also found, from the results of Experiment 12, that detection of tight junction in cells in the most superficial layer of cell aggregates can be used as a method for determining the time to add a BMP signaling pathway-activating substance in the production process of a cell cluster including an olfactory receptor neuron or a precursor cell thereof.

### [Experiment 13: Analysis of genes expressed in olfactory epithelium of living organism]

In Experiment 13, coronal sections of the head containing olfactory epithelium was made from a rat embryo at embryonic day 14.5, immunostained, and compared to the olfactory epithelium produced by the production method described in the present application. An embryo in uterus was collected from a rat at gestation day 14.5, washed with PBS, and then fixed with 4% paraformaldehyde phosphate buffer (manufactured by FUJIFILM Wako Pure Chemical Corporation) for 1 hour at room temperature. The embryo after fixation was washed with PBS, and immersed in 10%, 20%, 30% sucrose/PBS solutions sequentially until the embryos after fixation settled for tissue protection upon freezing. The neck of the embryo after the freezing-protection treatment was cut with anatomical scissors, and the head was transferred to cryomold No.2 (manufactured by Sakura Finetek). The excess 30% sucrose/PBS around the head was removed with a micropipetter. The cells were then embedded in an O. C. T compound (manufactured by Sakura Finetek), and rapidly frozen on an aluminum heat block cooled with dry ice to prepare blocks for making frozen sections. Frozen sections with a thickness of 10 µm were prepared with Lycra CM1950 cryostat (manufactured by Leica) from the blocks embedding the above aggregate, and attached onto PLATINUM PRO coated glass slides (manufactured by Matsunami Glass Co., Ltd.). The surroundings of the frozen sections on the glass slides were enclosed with ultra pap pens (manufactured by Bio Medical Science Inc.) and then permeabilized with 0.2% Triton-X 100/TBS for 10 minutes at room temperature, followed by blocking treatment with a mixture of a blocking reagent N-102 (manufactured by Nippon Oil, Inc.) and SuperBlock (TBS) Blocking Buffer (manufactured by Thermo Fisher Scientific) in a volume ratio of 1 : 4 for 30 minutes at room temperature.

To the frozen sections after the blocking treatment, of a dilution of primary antibody with Antibody Diluent OP Quanto (manufactured by Thermo Fisher Scientific) was added at 300 µl per glass slide, and the mixture was allowed to react overnight at 4°C in a wet box. The frozen sections after the primary antibody treatment were subjected to three washing manipulations of treating with 0.05% Tween-20/TBS for 10 minutes at room temperature. Secondary antibodies were diluted with a buffer of a mixture of Blocking One Histo (manufactured by NACALAI TESQUE, INC.) and 0.2% Triton-X 100/TBS in a volume ratio of 1 : 19. The diluent was then added to the frozen sections at 300 µl per glass slide, and allowed to react at room temperature for 1 hour. The frozen sections after the secondary antibody treatment were subjected to three washing manipulations of treating with 0.05% Tween-20/TBS for 10 minutes, then washed once with pure water. The frozen sections were then encapsulated with NEO cover glass (manufactured by Matsunami Glass Industries, Ltd.) and 30 µl of Prolong Diamond (manufactured by Thermo Fisher Scientific) per glass slide. The encapsulated specimens were allowed to stand overnight in the dark at room temperature to solidify Prolong Diamond. The surroundings of the cover glass were then coated with a clear varnish, then the varnish was air-dried in the dark at room temperature, and then observation by fluorescence microscopy was performed. For observation of the specimens and acquisition of the images, an upright fluorescence microscope Axio Imager M2 (manufactured by Carl Zeiss) and the affiliated software Axio Vision were used.

As primary antibodies for use in the immunofluorescence staining, antibodies against Sox2, E-Cadherin, Otx2, β-Catenin, Dlx5, Tuj1, Ebf2, pan-cytokeratin, PKOPζ, EpCAM, and laminin, and an anti-Ebfl antibody staining olfactory receptor neurons, described in Table 1, were used. As fluorescently labeled secondary antibodies, the antibodies described in Table 2 were used, and 1 µg/ml of Hoechst 33342 was added to the secondary antibody diluent for contrast staining of the nucleus. The positive and negative determinations of the staining results were performed in the same manner as in Preliminary Experiment 1.

The staining results are shown in FIG. 27. It was found that the olfactory epithelium of the living organism is a cytokeratin, EpCAM, β-Catenin, E-Cadherin-positive non-neural epithelium, while Ebf1, Ebf2, Tuj1-positive olfactory receptor neurons are present. Furthermore, placode marker genes of the anterior region of embryo, such as Otx2, Dlx5, and Sox2 were expressed. In addition, it was found that the surface of the olfactory epithelium was a PKCζ-positive top end surface, and the internal was a Laminin-positive basal surface, making top end-basal axis polarity. Compared to the above results, it was found that tissues in a cell cluster including an olfactory receptor neuron or a precursor cell thereof produced by the method described in the present application have succeeded in reproducing structures very similar to the olfactory epithelium of living organisms.

### [Experiment 14: Study for combination use of Wnt signaling pathway-activating substance and inhibitory substance in cell cluster production]

Cell clusters were produced in accordance with the procedures shown in the upper section of FIG. 28 by using Wnt signaling pathway-activating substance and inhibitory substance in combination. Human iPS cells (HC-6#10 cell line, obtained from the Institute of Physical and Chemical Research) were subjected to maintenance culturing and step (a) in the same manner as in Experiment 1. The suspension culturing was started on a 96-well culture plate in the same manner as in Experiment 1, except that the number of cells per well was 9 x 10³ cells.

At the start of suspension culturing (day 0 after the start of suspension culturing, start of step (1)), Y27632 (final concentration 20 µM), IWP-2 (final concentration 2 µM), and SB-431542 (final concentration 1 µM) were added to the serum-free medium. In addition, CHIR99021 (manufactured by Cayman Chemical, Inc.) was added as a Wnt signaling pathway-activating substance such that the final concentration was 300 nM. When the Wnt signaling pathway inhibitory substance IWP-2 and the Wnt signaling pathway-activating substance CHIR99021 are used in combination, β-catenin-dependent Wnt Canonical Pathway is activated and β-catenin-independent Wnt-non-Canonical Pathway is inhibited.

On day 2 after the start of suspension culturing, a serum-free medium being free of Y27632 and containing SB-431542, BMP4, IWP-2 and CHIR99021 was added at 100 µl per well (start of step (2)). BMP4 was added to the medium at 3 nM to be a final concentration in the well of 1.5 nM.

On day 3 after the start of suspension culturing, the medium was half exchanged with a serum-free medium being free of Y27632 and BMP4 and containing IWP-2, SB-431542, FGF2 and CHIR99021 (start of step (3a)). Thereafter, on days 6 and 10 after the start of suspension culturing, the medium was half exchanged with a serum-free medium being free of Y27632 and BMP4 and containing IWP-2, SB-431542, FGF2, CHIR99021, and K02288 (start of step (3c)). The concentration and the time to add K02288 were adjusted for the human iPS cell line HC-6#10, and on day 6 after the start of suspension culturing, K02288 was added to the medium at 2 µM to be a final concentration in the well of 1 µM. On day 13 after the start of suspension culturing, the medium was half exchanged with a medium containing IWP-2, SB-431542, FGF2, CHIR99021 and K02288 supplemented with EGF at twice as much to be a final concentration of 20 ng/ml. On day 17 after the start of suspension culturing, the medium was half exchanged with a medium containing IWP-2, SB-431542, FGF, CHIR99021, K02288 and EGF, and the culturing was performed until day 21 after the start of suspension culturing. Bright-field observation of the resulting cell cluster was performed with an inverted microscope on day 21 after the start of suspension culturing.

It was found that the cell cluster on day 21 after the start of suspension culturing induced by the differentiation induction method described above from the human iPS cell line HC-6#10 formed an olfactory epithelial-like tissue on the external of the cell cluster even when the Wnt signaling pathway activator CHIR99021 at downstream of the signaling was added under the condition in which IWP-2, a Wnt signaling pathway inhibitory substance, was added. Furthermore, the number of dead cells in neural tissues inside of the cell clusters derived from the human iPS cell line was reduced, and higher-quality cell clusters including olfactory epithelial-like tissues were formed (FIGS. 28A, 28B). From this result, it was shown that 1) the non-neural epithelial tissue formation promoting action of the PORCN inhibitor is not due to inhibition of the β-catenin-dependent Wnt-Canonical Pathway, and 2) a certain extent (about 3 µM CHIR99021 or less) of the activity of the β-catenin-dependent Wnt-Canonical Pathway acts to promote the survival of nervous tissues inside of the cell cluster and the epithelial formation, and the combination use of a Wnt signaling pathway inhibitory substance and a Wnt signaling pathway activator is effective in the production of high quality cell clusters depending on the nature of ES cell or iPS cell line.

### [Experiment 15: Study of effect of TAK1 inhibitor in cell cluster production]

Cell clusters were produced by adding a TAK1 inhibitor in step (3) in accordance with the procedures shown in the upper section of FIG. 29. Human iPS cells (HC-6#10 cell line, obtained from the Institute of Physical and Chemical Research) were subjected to maintenance culturing and step (a) in the same manner as in Experiment 1. The suspension culturing was started on a 96-well culture plate in the same manner as in Experiment 1, except that the number of cells per well was 9 x 10³ cells.

At the start of suspension culturing (day 0 after the start of suspension culturing, start of step (1)), Y27632 (final concentration 20 µM), IWP-2 (final concentration 2 µM), SB-431542 (final concentration 1 µM) and CHIR99021 (final concentration 300 nM) were added to the serum-free medium.

On day 2 after the start of suspension culturing, a serum-free medium being free of Y27632 and containing SB-431542, BMP4, IWP-2 and CHIR99021 was added at 100 µl per well (start of step (2)). BMP4 was added to the medium at 3 nM to be a final concentration in the well of 1.5 nM.

On day 3 after the start of suspension culturing, the medium was half exchanged with a serum-free medium being free of Y27632 and BMP4 and containing IWP-2, SB-431542, FGF2, Heparin Sodium, and CHIR99021. In addition, a TAK1 inhibitor 5z-7-oxozeaenol (manufactured by Cayman Chemical) was added to the medium at 4 µM to be a final concentration in the well of 2 µM (start of step (3a)). Thereafter, on days 6 and 10 after the start of suspension culturing, the medium was half exchanged with a serum-free medium being free of Y27632 and BMP4 and containing IWP-2, SB-431542, FGF2, Heparin Sodium, CHIR99021, K02288, and 5z-7-oxozeaenol (start of step (3c)). On day 13 after the start of suspension culturing, the medium was half exchanged with a medium containing IWP-2, SB-431542, FGF2, Heparin Sodium, CHIR99021, K02288 and 5z-7-oxozeaenol supplemented with EGF at twice as much to be a final concentration of 20 ng/ml. On day 17 after the start of suspension culturing, the medium was half exchanged with a medium containing IWP-2, SB-431542, FGF2, Heparin Sodium, CHIR99021, K02288, 5z-7-oxozeaenol and EGF, and the culture was performed until day 21 after the start of suspension culturing. On day 21 after the start of suspension culturing, bright-field observation of the resulting cell clusters was performed with an inverted microscope.

The cell clusters on day 21 after the start of suspension culturing induced by the differentiation induction method described above from human iPS cell line HC-6#10 resulted in more efficient formation of olfactory epithelium-like tissues on the surface of the cell cluster than in the cell cluster produced without the addition of the TAK1 inhibitor (FIGS. 29A, 29B). From this result, it was found that, in addition to a TGF-β receptor inhibitor and a BMP receptor inhibitor, an inhibition of TAK1 that is an intracellular signaling factor at downstream of the TGF-β receptor and the BMP receptor, in step (3), results in more efficient differentiation induction into placode and the olfactory epithelial-like tissues.

### [Experiment 16: Cell cluster production in viscous medium and maturation culture of cell clusters in the same medium]

Cell clusters were maturation-cultured in a viscous medium in accordance with the method shown in the upper section of FIG. 30. The viscous medium was prepared with reference to Japanese Patent No. 6176770. Specifically, 3 g of methylcellulose (manufactured by Sigma Aldrich, Inc., viscosity: 4000 cP, M0512) was weighed, and sterilized by autoclaving in a 500 ml wide-open medium bottle containing an agitator, and then 100 ml of medium was added thereto. As the medium, a 1 : 1 in volume mixture of F-12 + Glutamax medium and IMDM + Glutamax medium supplemented with 5% Knockout Serum Replacement, 10% fetal bovine serum (manufactured by Biosera), 450 µM 1-monothioglycerol, 1x Chemically defined lipid concentrate, 50 unit/ml penicillin-50 µg/ml streptomycin, 20 ng/ml FGF-TS, 20 ng/ml EGF, 20 ng/ml IGF-1 (manufactured by R&D Systems), 10 µg/ml Heparin Sodium, 1 µM SB431542, 1 µM K02288, 300 nM CHIR99021, and 100 nM EC23 (retinoic acid signaling pathway-activating substance) was used. The medium bottle containing methylcellulose supplemented with the medium was stirred in a cryogenic chamber overnight using a stirrer corresponding to a high viscosity liquid (manufactured by Asahi Rika Factory. Ltd, AMG-H). The next day, the resulting medium was checked for any remaining undissolved methylcellulose, then it was left to stand in the refrigerator for 3 days, and the air bubbles in the culture medium were removed. The medium containing 3% methylcellulose prepared by this method was a liquid with a high viscosity like glutinous syrup.

To a gas-permeable film-bottom 6 cm dish (lumox dish, 94.6077.333, manufactured by Zarstat, Inc.) was added 8 ml of the viscous medium prepared by the above method using a positive displacement pipette (Micoroman M1000, manufactured by Gilson, Inc.), and the medium was equilibrated in a CO₂ incubator. The cell cluster on day 21 of culturing prepared by the method described in Experiment 15 was then recovered from a 96-well plate into a 15 ml tube, washed once with 5% KSR gfCDM medium, and then transferred to a 6 cm dish for suspension culturing containing 5% KSR gfCDM medium. In addition, the cell clusters in the dish were collected using a wide-open pipette tip, and discharged one by one into a dish containing a viscous medium together with 8 µl of the medium. The number of cell clusters per 6 cm dish was set to 24. The dishes after transfer of the cell clusters were returned into the CO₂ incubator, and the culturing was performed (start of step (3e)). Once every 3 or 4 days after the start of culturing, 500 µl of 5% KSR gfCDM containing 400 ng/ml FGF-TS and 400 ng/ml EGF was added dropwise uniformly. Bright-field observation was performed with an inverted microscope on day 24 after the start of high viscosity culturing and day 45 after the start of differentiation induction.

In the cell clusters on day 45 after the start of suspension culturing induced by the differentiation induction method described above from human iPS cell line HC-6#10, more thickened olfactory epithelial-like tissues were formed on the surface of the cell clusters (FIG. 30A). From this result, it was found that culturing cell clusters in a viscous medium suppresses physical damages of tissues and is effective for long-term culturing. It was also found that adding IGF, serum, and a retinoic acid signaling pathway-activating substance to the culture medium in addition to the factors used in differentiation induction could further efficiently maintain olfactory epithelial-like tissues.

### [Experiment 17: Study of effect of basement membrane preparation in cell cluster production]

Cell clusters were produced in the presence of a basement membrane preparation in step (3) in accordance with the procedures shown in the upper section of FIG. 31. Human iPS cells (HC-6#10 cell line, obtained from the Institute of Physical and Chemical Research) were subjected to maintenance culturing and step (a) by the same method as in Experiment 1. The suspension culturing was started on a 96-well culture plate in the same manner as in Experiment 1, except that the number of cells per well was 9 x 10³ cells.

At the start of suspension culturing (day 0 after the start of suspension culturing, start of step (1)), Y27632 (final concentration 20 µM), IWP-2 (final concentration 2 µM), SB-431542 (final concentration 1 µM) and CHIR99021 (final concentration 300 nM) were added to the serum-free medium.

On day 2 after the start of suspension culturing, a serum-free medium being free of Y27632 and containing SB-431542, BMP4, IWP-2 and CHIR99021 was added at 100 µl per well (start of step (2)). BMP4 was added to the medium at 3 nM to be a final concentration in the well of 1.5 nM.

On day 3 after the start of suspension culturing, the medium was half exchanged with a serum-free medium being free of Y27632 and BMP4 and containing IWP-2, SB-431542, FGF2, CHIR99021, Heparin Sodium, and 5z-7-oxozeaenol (start of step (3a)). Thereafter, on days 6 and 10 after the start of suspension culturing, the medium was half exchanged with a serum-free medium being free of Y27632 and BMP4 and containing IWP-2, SB-431542, FGF2, CHIR99021, Heparin Sodium, K02288, and 5z-7-oxozeaenol (start of step (3c)). On day 13 after the start of suspension culturing, the medium was half exchanged with a medium containing IWP-2, SB-431542, FGF2, CHIR99021, Heparin Sodium, K02288, and 5z-7-oxozeaenol supplemented with EGF at twice as much to be a final concentration of 20 ng/ml. On day 17 after the start of suspension culturing, the medium was half exchanged with a medium containing IWP-2, SB-431542, FGF2, CHIR99021, Heparin Sodium, K02288, 5z-7-oxozeaenol and EGF, and the culture was performed until day 21 after the start of suspension culturing. The above culture conditions were used as the control, and comparison was performed with the conditions in which, on days 6, 10, or 13 from the start of suspension culturing, the medium was half exchanged with a medium supplemented with Corning Matrigel basement membrane matrix Growth Factor Reduced (GFR) (manufactured by Corning) at a final concentration of 1%. On day 21 after the start of suspension culturing, bright-field observation of the resulting cell clusters was performed with an inverted microscope.

In the cell cluster on day 21 after the start of suspension culturing induced by the differentiation induction method described above from human iPS cell line HC-6#10, under conditions in which Matrigel was added on day 6 from the start of suspension culturing, the cell cluster was grown large while the percentage of olfactory epithelium-like epithelium on the surface was reduced relative to the control to which no Matrigel was added (FIGS. 31A, 31B). Under conditions in which Matrigel was added on day 10 or 13 from the start of suspension culturing, the epithelium of the olfactory epithelial-like on the surface significantly thickened and proliferated (FIGS. 31C, 31D). From the above results, it was found that the growth of olfactory epithelial-like tissue can be promoted by adding a basement membrane preparation in the cell cluster production process. The concentration of the basement membrane preparation was preferably 0.5% or more and 4% or less when Matrigel was used, and most preferably 0.5% or more and 1.5% or less from the standpoint of experimental manipulation.

### [Experiment 18: Study of effect of embedded culturing in basement membrane preparation in cell cluster production]

Matrigel-embedded culturing was performed in accordance with the procedures in the upper section of FIG. 32. The cell clusters prepared on day 10 or day 13 of culturing prepared by the method described in Experiment 15 was recovered from a 96-well plate into a 15 ml tube, washed once with 5% KSR gfCDM medium, and then transferred to a 6 cm dish for suspension culturing containing 5% KSR gfCDM medium and placed at 37°C. Matrigel thawed on ice was added dropwise by 30 µl into a 6 cm dish for suspension culturing placed on a refrigerant stored at 4°C, and about 20 droplets were made. The collected cell clusters were discharged one by one with a wide bore tip into Matrigel in a dish, then placed at 37°C for 30 minutes, and Matrigel was gelled. After Matrigel was solidified, 5 ml of medium of 5% KSR gfCDM supplemented with 20 ng/ml FGF-TS, 20 ng/ml EGF, 10 µg/ml Heparin Sodium, 2 µM IWP-2, 1 µM SB431542, 1 µM K02288, 300 nM CHIR99021, and 2 µM 5z-7-Oxozeaenol were added, and the culturing was performed (start of step (3e)). Bright-field observation was performed with an inverted microscope on day 11 after the start of Matrigel-embedded culturing and day 21 after the start of differentiation induction.

As a result, in samples in which Matrigel-embedded culturing of cell clusters was performed on day 10 of culturing, a neural crest cell or head mesenchymal cell-like tissue was formed on the external of the olfactory epithelium-like epithelial tissue (FIG. 32A). The same was occurred with the sample in which the embedded culturing was performed on day 13 of culturing. From the above results, it was found that, by Matrigel-embedded culturing, a cell cluster including a neural crest cell or head mesenchymal tissue which becomes olfactory ensheating glia, in addition to olfactory epithelium and central nervous system, can be formed.

### INDUSTRIAL APPLICABILITY

According to the present invention, a cell cluster including an olfactory receptor neuron or a precursor cell thereof can be efficiently produced from a pluripotent stem cell at a low cost.

**[Table 1]**

| **Antigen** | **Host** | **Vendor** | **Catalog#** | **Dilution** |
|---|---|---|---|---|
| AP2 alpha | Mouse | Santa Cruz | SC-53163 | 1/200 |
| Bf1/FoxG1 | Rabbit | Takara Bio | M227 | 1/500 |
| Calretinin | Goat | R&D Systems | AF5065-SP | 1µg/ml |
| Chx10 | Goat | Santa Cruz | SC-21690 | 1/100 |
| Chx10 | Sheep | Exalpha | X1180P | 1/500 |
| CK, pan | Mouse | Sigma Aldrich | C2562-.2ML | 1/1000 |
| CK8 | Mouse | R&D Systems | MAB3165-SP | 1µg/ml |
| c-Maf | Mouse | R&D Systems | MAB8227-SP | 1/200 |
| Crystallin αA | Goat | R&D Systems | AF4848-SP | 1µg/ml |
| Dlx5 | Rabbit | Atlas Antibodies | HPA005670 | 1/500 |
| Ebf1 | Goat | R&D Systems | AF5165-SP | 1µg/ml |
| Ebf2 | Sheep | R&D Systems | AF7006-SP | 0.5µg/ml |
| E-Cadherin | Goat | R&D Systems | AF648-SP | 1/1000 |
| Emx2 | Sheep | R&D Systems | AF6470-SP | 1/500 |
| EpCAM | Mouse | Acris Antibodies | AM33251SU-S | 1/500 |
| EpCAM | Goat | R&D Systems | AF960-SP | 0.5µg/ml |
| EYA2 | Rabbit | Atlas Antibodies | HPA027024 | 1/500 |
| Islet-1 | Goat | R&D Systems | AF1837-SP | 1µg/ml |
| Laminin | Mouse | Dai-ichi Fine chemical | F-54 | 1/1000 |
| Laminin | Rabbit | LSL | LB-1013 | 1/1000 |
| Lhx2 | Rabbit | Millipore | AB10557 | 1/500 |
| N-Cadherin | Mouse | BD Biossience | 610920 | 1/500 |
| NCAM | Mouse | Biolegend | 304601 | 1/100 |
| Nestin | Rabbit | Spring Bioscience | E18610 | 1/500 |
| NeuroD1 | Goat | R&D Systems | AF2746-SP | 1µg/ml |
| Otx2 | Rabbit | Takara | M199 | 1/1000 |
| p63 | Rabbit | Santa Cruz | SC-8343 | 1/100 |
| Pax6 | Rabbit | Covance | PRB-278P | 1/1000 |
| Pbx1/2/3/4 | Mouse | Santa Cruz | SC-28313 | 1/100 |
| PKCζ | Rabbit | Santa Cruz | SC-216 | 1/100 |
| Prox1 | Goat | R&D Systems | AF2727-SP | 1µg/ml |
| Six1 | Rabbit | Atlas Antibodies | HPA001893 | 1/500 |
| Sox1 | Goat | R&D Systems | AF3369-SP | 1µg/ml |
| Sox2 | Mouse | R&D Systems | MAB2018-SP | 0.5µg/ml |
| Sox3 | Goat | R&D Systems | AF2569-SP | 1µg/ml |
| Sp8 | Goat | Santa Cruz | SC-104661 | 1/400 |
| Tuj1 | Mouse | Sigma Aldrich | T8660 | 1/500 |
| Tuj1 | Rabbit | GeneTex | GTX130245 | 1/500 |
| ZO-1 | Rabbit | Thermo Fisher Scientific | 61-7300 | 1/500 |
| β-Catenin | Mouse | BIOCARE Medical | ACR406A | 1/500 |

**[Table 2]**

| **Target** | **label** | **Host** | **Vendor** | **Catalog#** | **Dilution** |
|---|---|---|---|---|---|
| Rabbit IgG | Alexa 488 | Donkey | Thermo Fisher Scientific | A21206 | 1/1000 |
| Sheep IgG | CF 543 | Donkey | Biotium | 20322 | 1/1000 |
| Goat IgG | CF 555 | Donkey | Biotium | 20039 | 1/1000 |
| Mouse IgG | CF 555 | Donkey | Biotium | 20037 | 1/1000 |
| Goat IgG | Alexa 647 | Donkey | Thermo Fisher Scientific | A21447 | 1/1000 |
| Mouse IgG | Alexa 647 | Donkey | Thermo Fisher Scientific | A31571 | 1/1000 |

## Claims

1. A method for producing a cell cluster including an olfactory receptor neuron or a precursor cell thereof, comprising the following steps (1) to (3):
step (1) of suspension culturing a pluripotent stem cell in the presence of a first Wnt signaling pathway inhibitory substance to form a cell aggregate;
step (2) of suspension culturing the cell aggregate obtained in the step (1) in the presence of a BMP signaling pathway-activating substance; and
step (3) of suspension culturing the cell aggregate obtained in the step (2) to obtain the cell cluster, wherein step (3) comprises at least one step selected from the group consisting of:
step (3a) of suspension culturing in the presence of an FGF signaling pathway-activating substance;
step (3b) of suspension culturing in the presence of a BMP signaling pathway inhibitory substance; and
step (3c) of suspension culturing in the presence of an FGF signaling pathway-activating substance and a BMP signaling pathway inhibitory substance.

2. The production method according to claim 1, wherein the step (3) comprises the step (3a) and further comprises the step (3c) after the step (3a).

3. The production method according to claim 1 or 2, wherein the step (3) comprises the step (3b) or the step (3c) and further comprises step (3d) for suspension culturing in the absence of a BMP signaling pathway inhibitory substance after the step (3b) or the step (3c).

4. The production method according to any one of claims 1 to 3, wherein, in the step (3), an EGF signaling pathway-activating substance is further present.

5. The production method according to any one of claims 1 to 4, further comprising step (a), before the step (1), of culturing the pluripotent stem cell in the absence of a feeder cell in medium containing 1) at least one selected from the group consisting of TGFβ family signaling pathway inhibitory substances and sonic hedgehog signaling pathway-activating substances and 2) an undifferentiated state maintenance factor.

6. The production method according to any one of claims 1 to 5, wherein the BMP signaling pathway-activating substance includes at least one protein selected from the group consisting of BMP2, BMP4, BMP7, BMP13, and GDF7.

7. The production method according to any one of claims 1 to 6, wherein the step (3) comprises at least one step selected from the group consisting of the step (3a) and the step (3c), and the FGF signaling pathway-activating substance includes at least one selected from the group consisting of FGF2 and FGF8, and variants thereof.

8. The production method according to any one of claims 1 to 7, wherein a start time of the step (3) is 12 hours or more and 72 hours or less after addition of the BMP signaling pathway-activating substance in the step (2).

9. The production method according to any one of claims 1 to 8, wherein, in at least one step selected from the group consisting of the step (2) and the step (3), the first Wnt signaling pathway inhibitory substance is present.

10. The production method according to any one of claims 1 to 9, wherein the first Wnt signaling pathway inhibitory substance includes a substance having an inhibitory activity on a non-canonical Wnt pathway.

11. The production method according to any one of claims 1 to 10, wherein the first Wnt signaling pathway inhibitory substance includes a PORCN inhibitor.

12. The production method according to any one of claims 1 to 11, wherein the first Wnt signaling pathway inhibitory substance includes at least one selected from the group consisting of KY02111 and KY03-I.

13. The production method according to any one of claims 1 to 12, wherein the step (3) comprises at least one step selected from the group consisting of the step (3b) and the step (3c), and the BMP signaling pathway inhibitory substance includes a type 1 BMP receptor inhibitor.

14. The production method according to any one of claims 1 to 13, wherein, in at least one step selected from the group consisting of the step (1), the step (2), and the step (3), a TGFβ signaling pathway inhibitory substance is further present.

15. The production method according to claim 14, wherein the TGFβ signaling pathway inhibitory substance includes an Alk5/TGFβR1 inhibitor.

16. The production method according to any one of claims 1 to 15, wherein, in at least one step selected from the group consisting of the step (1), the step (2), and the step (3), a Wnt signaling pathway-activating substance is further present.

17. The production method according to claim 16, wherein the Wnt signaling pathway-activating substance acts on a signaling factor downstream of an active site of the first Wnt signaling pathway inhibitory substance.

18. The production method according to claim 16 or 17, wherein the Wnt signaling pathway-activating substance includes a substance that activates a Wnt-Canonical pathway.

19. The production method according to claim 18, wherein the substance that activates a Wnt-Canonical pathway inhibits decomposition of β-catenin or accelerates stabilization of β-catenin.

20. The production method according to any one of claims 16 to 19, wherein the Wnt signaling pathway-activating substance includes a GSK3 inhibitor.

21. The production method according to any one of claims 16 to 20, wherein the first Wnt signaling pathway inhibitory substance includes a PORCN inhibitor, and the Wnt signaling pathway-activating substance includes a GSK3 inhibitor.

22. The production method according to any one of claims 1 to 21, wherein, in the step (3), a TAK1 inhibitory substance is further present.

23. The production method according to any one of claims 1 to 22, wherein the step (3) further comprises, after the step (3a), the step (3b), or the step (3c), step (3e) for culturing.

24. The production method according to claim 23, wherein, in the step (3e), at least one selected from the group consisting of BMP signaling pathway inhibitory substances, TGFβ signaling pathway inhibitory substances, Wnt signaling pathway-activating substances, FGF signaling pathway-activating substances, and EGF signaling pathway-activating substances is present.

25. The production method according to claim 23 or 24, wherein, in the step (3e), at least one selected from the group consisting of retinoic acid signaling pathway-activating substances, serum, insulin-like growth factor receptor-activating substances, and neurotrophic factor receptor-activating substances is further present.

26. The production method according to any one of claims 23 to 25, wherein the step (3e) is carried out in medium containing a thickener.

27. The production method according to claim 26, wherein the medium containing a thickener has a viscosity of 100 mPa·s or more.

28. The production method according to any one of claims 23 to 27, wherein, in the step (3e), adherent culture is carried out.

29. The production method according to claim 28, wherein the adherent culture is carried out on a culture vessel coated with at least one selected from the group consisting of extracellular matrices, basement membrane preparations, and synthesized cell adhesion molecules.

30. The production method according to any one of claims 23 to 29, wherein, in the step (3e), culture is carried out by air liquid interface culture.

31. The production method according to any one of claims 1 to 30, wherein the step (3) comprises a step of culturing a cell aggregate that is embedded in a gel.

32. The production method according to claim 31, wherein the gel is Matrigel.

33. The production method according to any one of claims 1 to 32, wherein the step (3) comprises a step of suspension culturing in medium containing a basement membrane preparation.

34. The production method according to claim 33, wherein the basement membrane preparation is Matrigel, and a concentration of the Matrigel in medium is 0.5% to 4%.

35. The production method according to any one of claims 1 to 34, wherein, in the step (3), a second Wnt signaling pathway inhibitory substance different from the first Wnt signaling pathway inhibitory substance is further present.

36. The production method according to claim 35, wherein the second Wnt signaling pathway inhibitory substance includes a substance having an inhibitory activity on a canonical Wnt pathway.

37. The production method according to claim 35 or 36, wherein the second Wnt signaling pathway inhibitory substance includes a Tankyrase inhibitor.

38. A cell cluster including an olfactory receptor neuron or a precursor cell thereof obtained by the production method according to any one of claims 1 to 37.

39. A cell cluster including
1) a non-neural epithelial tissue part including an olfactory receptor neuron or a precursor cell thereof, and
2) a nervous tissue part including a neural cell or a precursor cell thereof,
wherein the neural cell or a precursor cell thereof includes a neural cell or a precursor cell thereof constituting a central nervous system, and at least a part of the surface of the nervous tissue part is covered with the non-neural epithelial tissue part.

40. The cell cluster according to claim 39, wherein the olfactory receptor neuron or a precursor cell thereof is expressing Tuj 1, EpCAM, and Lhx2.

41. The cell cluster according to claim 39 or 40, wherein the non-neural epithelial tissue part further includes a basement membrane-like structure, and the basement membrane-like structure is formed between the non-neural epithelial tissue part and the nervous tissue part.

42. The cell cluster according to claim 39, wherein the non-neural epithelial tissue part forms pseudostratified epithelium or stratified epithelium.

43. The cell cluster according to any one of claims 39 to 42, wherein the non-neural epithelial tissue part includes an olfactory epithelial-like tissue, and the olfactory receptor neuron or a precursor cell thereof is included in the olfactory epithelial-like tissue.

44. The cell cluster according to claim 43, wherein the olfactory epithelial-like tissue includes a basal cell or a precursor cell thereof.

45. The cell cluster according to claim 43 or 44,
wherein the olfactory epithelial-like tissue has a basal surface opposing to the nervous tissue part and a top end surface positioned on the opposite side of the basal surface,
wherein the basal surface faces a basement membrane, and
the top end surface is PKCζ or Ezrin-positive.

46. The cell cluster according to any one of claims 43 to 45,
wherein the olfactory epithelial-like tissue includes a medial olfactory epithelium and a lateral olfactory epithelium provided around the medial olfactory epithelium,
wherein the medial olfactory epithelium includes Sox2-, Tuj1-, and Ascl1-positive cells, and
the lateral olfactory epithelium includes Pax6- and Pbx-positive cells.

47. The cell cluster according to any one of claims 43 to 46, wherein the olfactory epithelial-like tissue further includes an olfactory ensheathing glia or a precursor cell thereof.

48. The cell cluster according to any one of claims 43 to 47, wherein the non-neural epithelial tissue part further includes a non-neural epithelial tissue other than the olfactory epithelial-like tissue.

49. The cell cluster according to any one of claims 39 to 48, wherein the neural cell or a precursor cell thereof further includes a cell or a precursor cell thereof constituting the retina.

50. The cell cluster according to any one of claims 39 to 49, wherein the neural cell or a precursor cell thereof includes the cerebrum.

51. A therapeutic drug for diseases due to an olfactory system disorder, comprising a cell or a tissue included in the cell cluster according to any one of claims 38 to 50.

52. A therapeutic drug for diseases due to a nervous tissue disorder, comprising a cell or a tissue included in the cell cluster according to any one of claims 38 to 50.
